# EUROPEAN PATENT SPECIFICATION

(11) **EP 3 391 903 B1**
(45) Date of publication and mention of the grant of the patent: **23.02.2022**
(21) Application number: 17167638.0
(22) Date of filing: 21.04.2017
(51) Int. Cl.: A61K 39/17, A61K 39/255

(54) **RECOMBINANT GALLID HERPESVIRUS 3 VACCINES ENCODING HETEROLOGOUS AVIAN PATHOGEN ANTIGENS**
ANTIGENE VON GEFLÜGELPATHOGENEN EXPRIMIERENDE REKOMBINANTE GALLID-HERPESVIRUS 3-IMPFSTOFFE
VACCINS RECOMBINANTS DE GALLID HERPÈSVIRUS 3 CODANT DES ANTIGÈNES DE PATHOGÈNES AVIAIRES HÉTÉROLOGUES

(43) Date of publication of application: 24.10.2018
(73) Proprietor: The Pirbright Institute, Pirbright, Surrey GU24 0NF (GB)
(72) Inventor: SADIGH, Yashar, Pirbright, Surrey GU24 0NF (GB); NAIR, Venugopal, Pirbright, Surrey GU24 0NF (GB)
(74) Representative: Oetke, Cornelia

(56) References cited:
- WO-A1-2017/027324
- WO-A2-2013/082317
- US-A1- 2014 147 465
- PETHERBRIDGE L ET AL: "Cloning of Gallid herpesvirus 3 (Marek's disease virus serotype-2) genome as infectious bacterial artificial chromosomes for analysis of viral gene functions", JOURNAL OF VIROLOGICAL METHODS, ELSEVIER BV, NL, vol. 158, no. 1-2, 1 June 2009 (2009-06-01), pages 11-17, XP026022528, ISSN: 0166-0934, DOI: 10.1016/J.JVIROMET.2009.01.009 [retrieved on 2009-01-31]
- ROBYN N HALL ET AL: "Identification of non-essential loci within the Meleagrid herpesvirus 1 genome", VIROLOGY JOURNAL, BIOMED CENTRAL, LONDON, GB, vol. 12, no. 1, 27 August 2015 (2015-08-27), page 130, XP021228646, ISSN: 1743-422X, DOI: 10.1186/S12985-015-0362-9

## Description

### TECHNICAL FIELD

The invention relates to a recombinant Gallid herpesvirus 3 vector encoding heterologous avian pathogen antigens comprising one or more heterologous polynucleotide(s) inserted into the intergenic loci UL3/UL4 or UL3/UL4 and UL21/UL22. The invention further relates to vaccines comprising said recombinant Gallid herpesvirus 3 vector and optionally a further Marek's disease virus vector and to a use of the vaccine for protecting an avian species against one or more avian pathogens. Further an *in vitro* method for producing the recombinant Gallid herpesvirus 3 vector encoding heterologous avian pathogen antigens is provided.

### BACKGROUND

Marek's disease (MD) is a highly contagious disease of poultry characterised by rapid-onset of T-cell lymphomas. The disease has a worldwide distribution and causes huge economic losses to the poultry industry. The causative agent of the disease, Marek's disease virus-1 (MDV-1, *Gallid herpesvirus* 2, GaHV-2), is an alpha herpesvirus of the genus *Mardivirus* (ICTV, 2006), which also includes the antigenically related herpesvirus of turkeys (HVT, *Meleagrid herpesvirus* 1), a strain used widely as a vaccine against MD since the late 1960s (Kawamura et al., 1969; Witter et al., 1970). The third member of the genus *Mardivirus* is the MDV-2 (*Gallid herpesvirus* 3, GaHV-3), which includes apathogenic strains some of which are used as live vaccines against MD (von Bülow et al., 1975).

Marek's disease virus-1 (MDV-1) or Gallid herpesvirus 2 (GaHV2), the causative agent of Marek's disease (MD), is one of the major pathogens in poultry. MDV-1 is a member of the Mardivirus genus in the family Herpesviridae. In the infected birds, MDV-1 replicates in the feather follicle epithelial cells and spreads through the respiratory route by the inhalation of the poultry house dust contaminated with infectious virus shed with the dead skin and dander. MD is characterized by widespread T cell lymphomas and paralytic symptoms due to neuronal infiltration of lymphocytes. The mortality rate due to MD usually varies between 10-30%, but can go up to 60-80%. Live vaccines comprising of different strains are used in different combinations for the control of MD in the last 40 years (Witter RL. Curr Top Microbiol Immunol. 2001; 255: 57-90; Calnek BW et al., Avian diseases. 1983;27:844-9). These include the naturally attenuated MDV-1 strain Rispens (CVI-988), MDV-2 (GaHV3) strain SB-1 and herpesvirus of turkeys (HVT) strain Fc126. For instance, WO 2017/027324 describes recombinant turkey herpesvirus vaccines and uses thereof.

In addition to their use as successful vaccines inducing long-term protection against MD, avian herpesvirus vaccine strains have also been recognised as recombinant viral vectors for inducing protection against other major avian infectious diseases. The most successful and widely used recombinant vaccine vector is HVT, which has been shown to be very effective in protecting against a number of avian viral pathogens (Morgan RW, et al., Avian diseases 1993; 37: 1032-40; Li Y, et al., Vaccine. 2011; 29: 8257-66; Darteil R et al., Virology 1995;211:481-90; Kapczynski DR et al., Vaccine. 2015; 33: 1197-205). Although individual recombinant HVT vaccines have proven to be extremely effective, when using more than one HVT-vector vaccine it has been problematic to get the desired immune responses against each component of the combined vaccine. There are clear recommendations not to use other HVT with the recombinant vectors, as there will be interference resulting in poor efficacy against the foreign insert (American Association of Avian Pathologists A. Frequently asked questions on viral tumor diseases http://www.aaap.info/frequently-asked-questions-on-viral-tumor-diseases, 2012). With this constraint on the HVT vector for its use as multivalent vaccines, there is a need for other vector platforms that will complement rather than interfere with protection against multiple components in the vaccine.

The first GaHV3 (MDV-2) that was licensed for use as a vaccine was the SB-1 strain (Schat KA et al., J Natl Cancer Inst. 1978; 60: 1075-82). It was originally introduced as a vaccine against MD in the mid-1980s and used in combination with HVT vaccine (Calnek BW et al., Avian diseases. 1983; 27: 844-9). Bivalent vaccines containing SB-1 and HVT Fc126 strains have been successfully used in many countries including USA, South America and Asia (Witter RL. Curr Top Microbiol Immunol. 2001; 255: 57-90; Bublot M, Sharma J. Vaccination against Marek's disease. In: Davison F, Nair V, editors. Marek's Disease-An Evolving Problem. London: Elsevier Academic Press; 2004. p. 168-85). SB-1/HVT bivalent vaccines are thought to provide superior protection through a synergistic effect although the molecular mechanism has not been identified (Calnek BW et al., Avian diseases. 1983;27:844-9; Witter RL et al., Avian pathology: journal of the WVPA. 1984;13:75-92). SB-1 has also been reported to induce very good protection even in maternal antibody-positive chicks (Witter RL et al., Avian pathology: journal of the WVPA. 1984; 13: 75-92). The 166-Kb genome of the SB-1 strain of GaHV3 has a similar genome organization as MDV-1 sharing a number of homologous genes (Spatz SJ et al., Virus Genes. 2011;42:331-8) as well as unique set of genes and microRNAs (Yao Y et al., J Virol. 2007;81:7164-70).

Unlike MDV-1, where extensive studies have been carried out to examine the molecular determinants of various biological characteristics, only limited studies have been carried out on the GaHV3 (MDV-2) genome to unravel some of the unique properties associated with this virus. Determination of the complete genome sequence of the MDV-2 prototype strain HPRS-24 has enabled the comparisons of the genome structure and sequence with other MDV species (Izumiya et al., 2001). Recent advances in cloning of herpesvirus genomes as bacterial artificial chromosomes (BAC) have helped to identify molecular characteristics of several herpesviruses (Adler et al., 2003; Zelnik, 2003) including MDV-1 (Petherbridge et al., 2004, 2003; Schumacher et al., 2000), HVT (Baigent et al., 2006) and MDV-2 vaccine strain SB-1 (Petherbridge et al., 2009). WO 2013/082317 describes recombinant Gallid herpesvirus 3 (mdv serotype 2) vectors expressing antigens of avian pathogens and uses thereof.

Vaccines against MDV based on a recombinant herpesvirus MDV strain offers the advantage of simultaneously achieve immunity against MDV and at least a further viral pathogen. Different methods have been employed to produce such bivalent vaccines. One of the widely used and successful approaches has been the delivery of the IBDV VP2 antigen in various MDV vaccine vector platforms, including the HVT (Tsukamoto K et al., J Virol. 2002; 76: 5637-45; Perozo F et al., Avian diseases. 2009; 53: 624-8), MDV-1 (Tsukamoto K et al., Virology. 1999; 257: 352-62; Zhou X et al., Vaccine. 2010;28:3990-6) and fowl pox viral vectors (Tsukamoto K et al., Virology. 2000; 269: 257-67). Considering the need for protecting poultry against multiple pathogens, there is a need for additional vector platforms that will not interfere with each other to deliver protective immune responses to all the components in the vaccine.

MDV and IBDV are highly infectious viruses whose high mortality rates have made them a constant threat to the worldwide poultry industry for decades. Presence of maternal antibodies, emergence of new variants, cost of production and in some cases lack of compliance with DIVA strategy (reviewed in Muller H, et al., Avian pathology: journal of the WVPA. 2012; 41: 133-9) are challenges that limit the efficient control of IBDV. IBDV is one of the most difficult viruses to protect against using live recombinant avian vaccines and hence suitable protecting efficacy needs to be shown. Bivalent MDV/IBDV vaccines allow for vaccination against both diseases simultaneously, lowering the costs of production and inoculation. They are also safer and can be given *in ovo*, unlike attenuated IBDV vaccines that cause subclinical IBD in chicks and are fatal to embryos. VAXXITEK_{HVT+IBD} is an existing MDV/IBDV bivalent vaccine based on HVT. However there is still a need for further bivalent MDV vaccines.

### SUMMARY OF THE INVENTION

Provided herein is a recombinant Gallid herpesvirus 3 (GaHV3; MDV-2) vector comprising one or more heterologous polynucleotide(s) coding for and expressing at least one antigen of an avian pathogen, inserted into the intergenic loci UL3/UL4 or UL3/UL4 and UL21/UL22 of the Gallid herpesvirus 3 vector. Preferably the recombinant Gallid herpesvirus 3 vector is a recombinant Gallid herpesvirus 3 strain SB-1 vector. In one embodiment the recombimant Gallid herpesvirus 3 vector comprises the one or more heterologous polynucleotide(s) inserted into the intergenic locus UL3/UL4 of the Gallid herpesvirus 3 vector. Preferably the at least one antigen is protective against infectious bursal disease virus (IBDV), infectious laryngotracheitis virus (ILTV), Newcastle disease virus (NDV), avian influenza virus (AIV) or avian infectious bronchitis virus (IBV). The at least one antigen may be selected from the group consisting of (a) VP2, VP3, VP4 and VPX of infectious bursal disease virus (IBDV); (b) glycoprotein B, glycoprotein I, glycoprotein D, glycoprotein E and glycoprotein C of ILTV; (c) Newcastle disease virus fusion protein (NDV-F) and viral hemagglutinin neuraminidase (NDV-NH) of NDV (d) Avian influenza hemagglutinin (HA) and neuraminidase (NA); and (e) S1 and S2 protein of IBV. Preferably the at least one antigen is protective against IBDV. More preferably the at least one antigen is VP2 of IBDV. In one embodiment the VP2 protein amino acid sequence has at least 80% sequence identity to the sequence set forth in SEQ ID NOs: 12. In another embodiment the VP2 protein has the sequence set forth in SEQ ID NOs: 12.

The Gallid herpesvirus 3 vector preferably contains an expression cassette(s) containing the one or more heterologous polynucleotide(s), wherein optionally the expression cassette further comprises a promoter. In one embodiment the promoter is selected from the group consisting of immediate early cytomegalovirus (CMV) promoter, guinea pig CMV promoter, murine CMV promoter, SV40 promoter, pseudorabies virus promoters of glycoprotein X promoter, herpes simplex virus-1 alpha 4 promoter, chicken beta-actin promoter, rabbit beta-globin promoter, herpes simplex virus thymidine kinase promoter, Marek's Disease Virus promoters of glycoproteins gC, gB, gE, or gI genes and infectious laryngotracheitis virus promoters of glycoprotein gB, gE, gI, gD genes.

In another aspect a vaccine is provided comprising the recombinant Gallid herpesvirus 3 vector of the invention. The vaccine may further comprise a pharmaceutically acceptable excipient, carrier or adjuvant. In one embodiment the vaccine also comprises a further Marek's disease virus (MDV) vector selected from the group consisting of Gallid herpesvirus 3 vector, naturally attenuated MDV-1 strain Rispens (CVI-988) vector and herpesvirus of turkeys (HTV) strain Fc126 vector, wherein the further MDV vector may be a recombinant MDV vector. In a specific embodiment the further Marek's disease virus (MDV) vector is a recombinant Marek's disease virus (MDV) vector comprising one or more heterologous polynucleotide(s) coding for and expressing at least one antigen of an avian pathogen. According to the invention the further recombinant Marek's disease virus vector may be a second recombinant Gallid herpesvirus 3 vector in addition to the first recombinant Gallid herpesvirus 3 vector according to the invention, preferably the second recombinant Gallid herpesvirus 3 vector is a recombinant Gallid herpesvirus 3 strain SB-1 vector.

In one embodiment the second recombinant Gallid herpesvirus 3 vector also comprises the one or more heterologous polynucleotide(s) inserted into the intergenic loci UL3/UL4 or UL3/UL4 and UL21/UL22 of said second Gallid herpesvirus 3 vector, preferably into the intergenic locus UL3/UL4 of the second Gallid herpesvirus 3, wherein the second recombinant Gallid herpesvirus 3 vector comprises at least one heterologous polynucleotide coding for and expressing a different antigen of an avian pathogen as the one or more heterologous polynucleotides of the first recombinant Gallid herpesvirus 3 vector. The further Marek's disease virus vector and the (first) recombinant Gallid herpesvirus 3 vector according to the invention may be administered together or separate from each other.

Further described herein is an isolated DNA encoding the recombinant Gallid herpesvirus 3 vector according to the invention.

In yet another aspect the present invention provides a bacterial artificial chromosome (BAC) comprising a polynucleotide coding for the recombinant Gallid herpesvirus 3 vector according to the invention.

In yet another aspect the vaccine of the invention is provided for use in vaccinating an avian species against one or more diseases caused by one or more avian pathogens, preferably against Marek's disease and one or more diseases caused by one or more avian pathogens. In one embodiment the one or more diseases are caused by one or more of infectious bursal disease virus (IBDV), infectious laryngotracheitis virus (ILTV), Newcastle disease virus (NDV), avian influenza virus (AIV) or avian infectious bronchitis virus (IBV). The vaccine is further provided for use in protecting an avian species against clinical symptoms caused by one or more avian pathogens, preferably against clinical symptoms caused by Marek's disease virus and clinical symptoms caused by one or more avian pathogens. In one embodiment the one or more avian pathogen causing the diseases or clinical symptoms is selected from the group consisting of Newcastle disease virus, infectious bursal disease virus and avian infectious laryngotracheitis virus, avian influenza virus and avian infectious bronchitis virus .

The avian species may be poultry, preferably the avian species is chicken, duck, goose, turkey, quail, guinea or pigeon, more preferably the avian species is turkey or chicken, even more preferably chicken. The vaccine according to the invention may be administered by spray administration, in ovo, subcutaneously, intramuscularly, orally or nasally. In a particular embodiment the vaccine is administered in ovo preferably in ovo in 18 day old embryonated eggs. In an alternative embodiment the vaccine is administered in subcutaneously or intramuscularly in chicks, preferably in 1 day old chicks.

Further described is a method of treating an avian species for protection against Marek's Disease and one or more diseases caused by one or more avian pathogens comprising the step of administering an effective amount of the vaccine according to the invention, wherein preferably the one or more diseases is caused by one or more of infectious bursal disease virus (IBDV), infectious laryngotracheitis virus (ILTV), Newcastle disease virus (NDV), avian influenza virus (AIV) or avian infectious bronchitis virus (IBV).

In yet another aspect a recombinant Gallid herpesvirus 3 (GaHV3; MDV-2) vector is provided comprising one or more marker(s) inserted into the intergenic loci UL3/UL4 or UL3/UL4 and UL21/UL22 of the Gallid herpesvirus 3 vector, preferably into the intergenic locus UL3/UL4 of the Gallid herpesvirus 3 vector, wherein the marker is preferably a selection marker gene, a reporter gene or a DNA bar code. Preferably, the recombinant Gallid herpesvirus 3 vector is a recombinant Gallid herpesvirus 3 strain SB-1 vector. Also provided is a bacterial artificial chromosome (BAC) comprising a polynucleotide coding for the recombinant Gallid herpesvirus 3 vector comprising one or more marker(s) inserted into the intergenic loci UL3/UL4 or UL3/UL4 and UL21/UL22 of the Gallid herpesvirus 3 vector, wherein the recombinant Gallid herpesvirus 3 vector is preferably a recombinant Gallid herpesvirus 3 strain SB-1 vector.

Also provided is an *in vitro* method of producing a recombinant Gallid herpesvirus 3 vector comprising (a) providing a Gallid herpesvirus 3 vector, (b) inserting one or more heterologous polynucleotide(s) coding for and expressing at least one antigen of an avian pathogen into the intergenic loci UL3/UL4 or UL3/UL4 and UL21/UL22 of the Gallid herpesvirus 3 vector, and optionally (c) amplifying the Gallid herpesvirus 3 vector comprising one or more heterologous polynucleotide(s) coding for at least one antigen of an avian pathogen of step (b). In one embodiment the *in vitro* method comprises (a) providing a Gallid herpesvirus 3 vector comprising one or more marker(s) in the intergenic loci UL3/UL4 or UL3/UL4 and UL21/UL22 of the Gallid herpesvirus 3 vector, (b) replacing the one or more marker(s) with an expression cassette comprising one or more heterologous polynucleotides coding for at least one antigen of an avian pathogen, and (c) amplifying the Gallid herpesvirus 3 vector comprising one or more heterologous polynucleotide(s) coding for at least one antigen of an avian pathogen of step (b). Preferably, the recombinant Gallid herpesvirus 3 vector is a recombinant Gallid herpesvirus 3 strain SB-1 vector.

### FIGURE LEGENDS

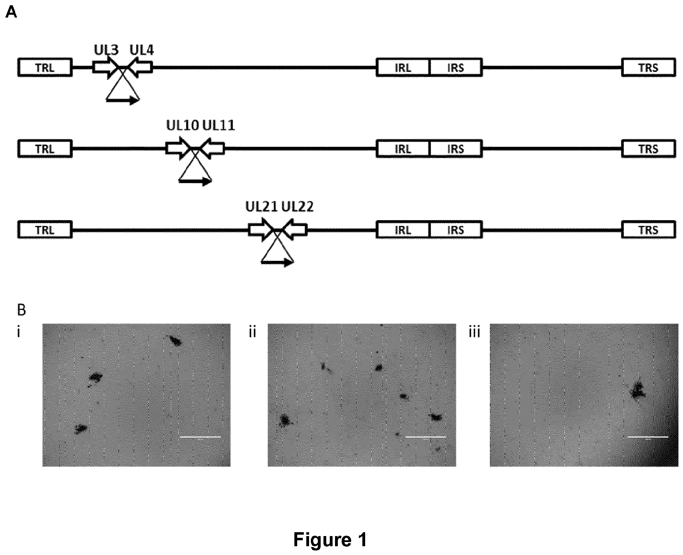

Figure 1. A) Recombineering process to generate (top, A-i) SB-1-UL3/4VP2, (middle, A-ii) SB-1-UL10/11VP2 and (bottom, A-iii) SB-1-UL21/22VP2. The VP2 expression cassette was inserted in the intergenic loci UL3/UL4, UL10/UL11 and UL21/UL22. The VP2 expression cassette is shown as a solid arrow. The location and the orientation of the genes from SB-1 is shown. B) VP2 immunostaining (dark blue) in plaques of infected cells. CEF cells were infected with (B-i) SB-1-UL3/4VP2, (B-ii) SB-1-UL21/22VP2 and (B-iii) SB-1-UL10/11VP2. Cells were fixed five days post infection and stained as described in the materials and methods section (scale bar = 2000 µm).

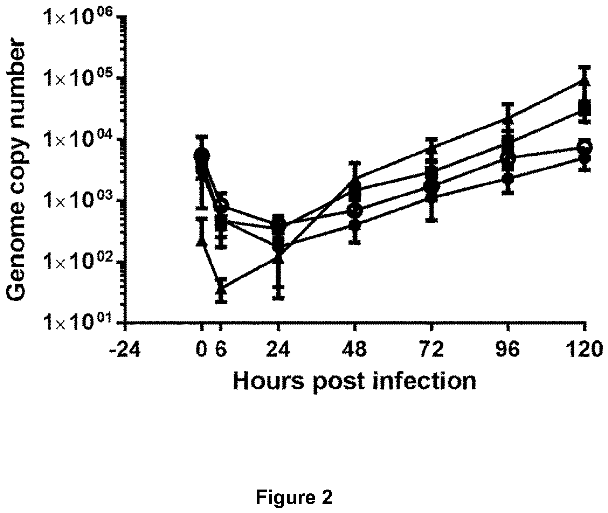

Figure 2. Growth curve for SB-1 recombinant viruses *in vitro.* The genome copy number per one million CEF cells is plotted against hours post infection; SB-1-UL3/4VP2 (■), SB-1-UL21/22VP2 (●), SB-1-UL10/11VP2 (○) and SB-1 (▲) vaccine viruses. No significant difference between SB-1 and SB-1 UL3/4VP2 after 24 hours post infection was observed. Replication level for SB-1-UL10/11VP2 and SB-1-UL21/22VP2 were lower than that of SB-1 and SB-1-UL3/4VP2 viruses. Each time point was calculated based on three replicates. Each replicate was measured in triplicate. Error bars represent the standard deviation.

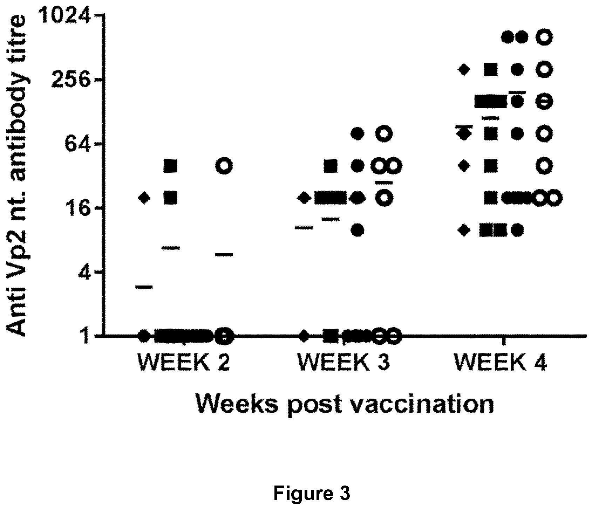

Figure 3. Titres of neutralizing antibodies against VP2 in the sera of chickens vaccinated with VAXXITEK_{HVT+IBD} ( 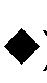 ), SB-1-UL3/4VP2 ( 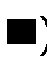 ), SB-1-UL21/22VP2 ( 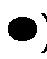 ), SB-1-UL10/11VP2 (○) and SB-1 ( 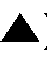 ) vaccine viruses. Serum samples were collected at weeks 2, 3 and 4 post-vaccination. The mean titre in each group is shown as a horizontal bar.

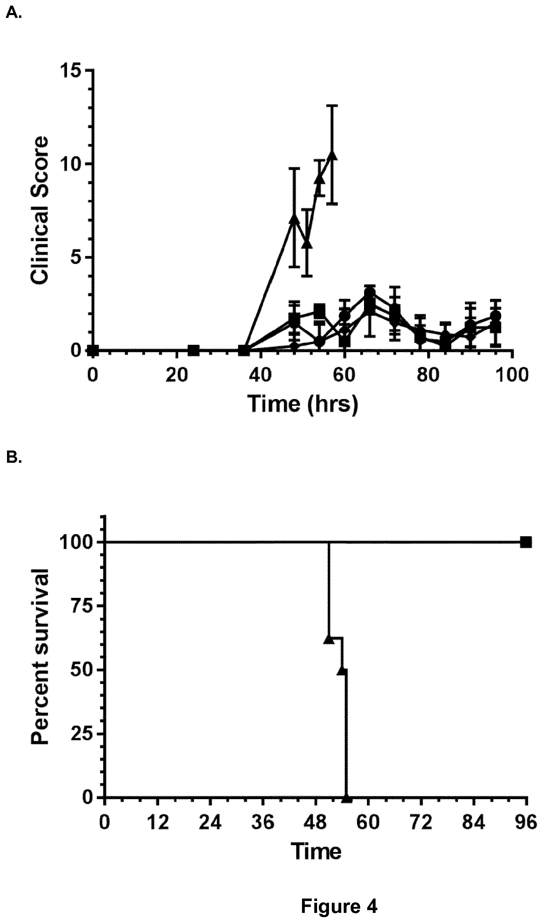

Figure 4. Chickens were vaccinate with VAXXITEK_{HVT+IBD} ( 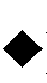 ), SB-1-UL3/4VP2 ( 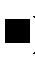 ), SB-1-UL21/22VP2 ( 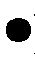 ) and SB-1 ( 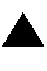 ) followed by infection with IBDV UK661 at time point 0. A) Mean clinical score in vaccinated birds challenged with IBDV UK661. Bars show standard deviation, n=8. B) Percentage survival for the vaccinated birds challenged with IBDV UK661. Birds inoculated with pSB-1 were euthanized for humane reasons or died 55 hours post IBDV infection.

### DETAILED DESCRIPTION

The general embodiments "comprising" or "comprised" encompass the more specific embodiment "consisting of". Furthermore, singular and plural forms are not used in a limiting way. As used herein, the singular forms "a", "an" and "the" designate both the singular and the plural, unless expressly stated to designate the singular only.

The term "animal" is used herein to include all mammals, birds and fish. In this particular context animal refers to an avian species, such as chicken, duck, goose, turkey, quail, guinea, pigeon, swan, pheasant, parrot, finche, hawk, crow, ostrich, emu and cassowary. The term "animal" and "avian" also includes an individual animal in all stages of development, including embryonic and fetal stages. Preferably, the animal is poultry. As used herein the term "poultry" refers to a domestic or commercial bird kept for the eggs they produce, their meat or feathers. Poultry may be birds from the order Galliformes, such as chicken, duck, goose, turkey, quail, guinea and pigeon.

The term "avian species" as used herein relates to birds, preferably poultry, such as chicken, duck, goose, turkey, quail, guinea or pigeon. Particularly preferred in the context of the present invention is turkey or chicken, even more preferred chicken.

The terms "nucleic acid", "nucleotide", and "polynucleotide" as used herein are used interchangeably and refer to a single or double- stranded polymer of deoxyribonucleotide bases or ribonucleotide bases read from the 5' to the 3' end and include RNA, DNA, cDNA, or cRNA and derivatives thereof, such as those containing modified backbones. In the context of the Gallid herpesvirus 3 vector the skilled person would understand that it refers to deoxyribonucleic acid, i.e., DNA or cDNA. Polynucleotides according to the invention can be prepared in different ways (e.g. by chemical synthesis, by gene cloning etc.) and can take various forms (e.g. linear or branched, single or double stranded, or a hybrid thereof, primers, probes etc.). The term "nucleotide sequence" or "nucleic acid sequence" refers to both the sense and antisense strands of a nucleic acid as either individual single strands or in the duplex. The term "ribonucleic acid" (RNA) is inclusive of RNAi (inhibitory RNA), dsRNA (double stranded RNA), siRNA (small interfering RNA), mRNA (messenger RNA), miRNA (micro-RNA), tRNA (transfer RNA, whether charged or discharged with a corresponding acylated amino acid), and cRNA (complementary RNA).

The term "genomic DNA", or "genome" is used interchangeably and refers to the heritable genetic information of a host organism. The genomic DNA comprises the DNA of the nucleus (also referred to as chromosomal DNA) but also of other cellular organelles (e.g., mitochondria).

The term "genomic RNA" or "genome" refers to the heritable genetic information of an RNA virus. The genomic RNA may be a positive strand or a negative strand RNA.

The term "gene" as used herein refers to a DNA or RNA locus of heritable genomic sequence which affects an organism's traits by being expressed as a functional product or by regulation of gene expression. Genes and polynucleotides may include introns and exons as in genomic sequence, or just the coding sequences as in cDNAs, such as an open reading frame (ORF), comprising a start codon (methionine codon) and a translation stop codon. Genes and polynucleotides can also include regions that regulate their expression, such as transcription initiation, translation and transcription termination. Thus, also included are regulatory elements such as promoters.

The term "heterologous polynucleotide" as used herein refers to a polynucleotide derived from a different organism or a different species from the recipient coding for a heterologous protein. In the context of the Gallid herpesvirus 3 vector the skilled person would understand that it refers to a DNA or cDNA. A heterologous polynucleotide may also be referred to as transgene. Thus, it may be a gene or open reading frame (ORF) coding for a heterologous protein. In the context of the Gallid herpesvirus 3 "heterologous polynucleotide" refers to a polynucleotide derived from a different avian pathogen or virus (different species and/or strain), particularly a different avian virus, including a different virus of the family *Herpesviridae* that causes avian infection and a different strain of Gallid herpesvirus 3. The term "heterologous" when used with reference to portions of a nucleic acid indicates that the nucleic acid comprises two or more sequences that are not found in the same relationship to each other in nature. Heterologous may also refer to a viral polynucleotide sequence, such as a gene or transgene, or a portion thereof, being inserted into a viral genome in which it is not typically found, or a gene introduced into an organism in which it is not typically found.

A "recombinant viral vector" or "viral vector" as used herein refers to a recombinant virus comprising a virus genome and a heterologous polynucleotide for transduction of a host cell. Such a recombinant viral vector according to the invention may be derived from a Gallid herpesvirus 3 strain SB-1 or HPRS24. As appropriate, viral gene- or protein- coding sequences may be incorporated into such a recombinant viral vector as described herein for vaccinating a chicken or other poultry against one or more viral diseases. A recombinant viral vector may also comprise a heterologous polynucleotide coding for and expressing a marker. Typically a recombinant viral vector contains a heterologous polynucleotide or transgene that is operatively linked to a promoter in order to effect transcription of the transgene. The term "recombinant" further includes any modification, alteration or engineering of a polynucleotide or protein in its native form or structure, or any modification, alteration or engineering of a polynucleotide or protein in its native environment or surrounding. The modification, alteration or engineering of a polynucleotide or protein may include, but is not limited to, deletion of one or more nucleotides or amino acids, deletion of an entire gene, codon-optimization of a gene, conservative substitution of amino acids and insertion of one or more heterologous polynucleotides.

The term "recombinant Gallid herpesvirus 3 (GaHV3, MDV-2) vector" as used herein refers to a recombinant virus comprising all essential genes of GaHV3, such as of strain SB-1 or HPRS24. The Gallid herpesvirus 3 (MDV-2) strains used for the recombinant viral vector may be any SB-1 strains, including, but not limited to, the commercial Marek's Disease Vaccine (SB-1 vaccine) (Merial Select Inc., Gainesville, GA 30503, USA), having a genome sequence as defined by GenBank Accession Number HQ840738.1 The recombinant Gallid herpesvirus 3 (GaHV3, MDV-2) vector may further contain deleted or mutated non-essential genes. The Gallid herpesvirus 3 (MDV-2) may be any Gallid herpesvirus 3 (MDV-2) comprising a genome sequence having at least 95%, 96%, 97%, 98%, or 99% sequence identity to the sequence of Gallid herpesvirus 3 (MDV-2) strain SB-1 as defined in GenBank Accession Number HQ840738.1 (without considering inserted heterologous polynucleotide sequences). Also deletion of any non-essential genes is not considered for sequence alignment.

A Gallid herpesvirus 3 (MDV-2) strain other than SB-1 is the HPRS24 strain having the genome sequence as defined by GenBank Accession Number NC_002577.1 (INSDC: AB049735.1). The genomes of HPRS24 and SB-1 share 98.4% sequence identity (Spatz and Schat, 201 1; Virus Gene 42, 331-338). The Gallid herpesvirus 3 (MDV-2) strains used for the recombinant viral vector may be the HN-1, 287C/1, 401/1, 437A/1, 301B/1, 471B/1, 281MI/1 and 298B/1 isolates described in the publications (Witter (1987) Avian Dis 31, 752-765; Witter et al. (1987) Avian Dis 31, 829-840; Witter (1995) Avian Pathology (1995) 24, 665-678). Although not used as a vaccine strain to date, strain HPRS24, or any of the above mentioned MDV-2 isolates can also considered a suitable vaccine strain, due to their relatedness and antigenic similarity.

The term "protein" is used interchangeably with "amino acid residue sequences" or "polypeptide" and refers to polymers of amino acids of any length. These terms also include proteins that are post-translationally modified through reactions that include, but are not limited to, glycosylation, acetylation, phosphorylation or protein processing. Modifications and changes, for example fusions to other proteins, amino acid sequence substitutions, deletions or insertions, can be made in the structure of a polypeptide while the molecule maintains its biological functional activity. For example certain amino acid sequence substitutions can be made in a polypeptide or its underlying nucleic acid coding sequence and a protein can be obtained with the same properties.

The term "antigen" as uses herein is a substance which provokes an adaptive immune response in a host animal and may also be referred to as immunogen. Antigens are typically of high molecular weight and proteins or polysaccharides. Peptides, lipids, nucleic acids and many other materials can also function as antigens. An antigen can be a whole organism, killed, attenuated or live or a subunit or portion of an organism; a piece or fragment of DNA capable of inducing an immune response upon presentation to a host animal; a polypeptide or a fragment thereof, an epitope, a hapten, or any combination thereof. An immunogen or antigen may be also a toxin or antitoxin. The antigen coded for and expressed by the heterologous polynucleotides according to the invention is typically an immunogenic or antigenic protein. As used herein an antigen may also refer to the immunogenic part or fragment of an antigen comprising the epitope, e.g., peptide(s).

As used herein the term "antigen of an avian pathogen" refers to a protein encoded by a pathogen, preferably a virus described herein, including structural and non-structural proteins. Such antigens may include naturally occurring or non- naturally occurring viral proteins from IBDV, NDV, AIV, ILTV and/or IBV including VP2, F, and/or HN proteins. As used herein, an "antigen" refers to a viral protein or polypeptide, such as a viral polypeptide, as well as viral particles. In some embodiments, an antigen in accordance with the invention may also be a viral nucleic acid.

The term "immunogenic" protein or peptide as used herein includes polypeptides that are immunologically active in the sense that once administered to the host, it is able to evoke an immune response of the humoral and/or cellular type directed against the protein. Preferably the protein fragment is such that it has substantially the same immunological activity as the full length protein. Thus, a protein fragment according to the invention comprises or consists essentially of or consists of at least one epitope or antigenic determinant. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above.

The term "immunogenic protein or peptide" further contemplates deletions, additions and substitutions to the sequence, as long as the polypeptide functions to produce an immunological response as defined herein. The term "conservative variation" denotes the replacement of an amino acid residue by another biologically similar residue, or the replacement of a nucleotide in a nucleic acid sequence such that the encoded amino acid residue does not change or is another biologically similar residue. In this regard, particularly preferred substitutions will generally be conservative in nature, i.e., those substitutions that take place within a family of amino acids. For example, amino acids are generally divided into four families: (1) acidic-aspartate and glutamate; (2) basic- lysine, arginine, histidine; (3) non-polar-alanine, valine, leucine, isoleucine, proline, phenylalanine, methionine, tryptophan; and (4) uncharged polar- glycine, asparagine, glutamine, cysteine, serine, threonine, tyrosine. Phenylalanine, tryptophan, and tyrosine are sometimes classified as aromatic amino acids. Examples of conservative variations include the substitution of one hydrophobic residue such as isoleucine, valine, leucine or methionine for another hydrophobic residue, or the substitution of one polar residue for another polar residue, such as the substitution of arginine for lysine, glutamic acid for aspartic acid, or glutamine for asparagine, and the like; or a similar conservative replacement of an amino acid with a structurally related amino acid that will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide. All of the polypeptides produced by these modifications are included herein. The term "conservative variation" also includes the use of a substituted amino acid in place of an unsubstituted parent amino acid provided that antibodies raised to the substituted polypeptide also immunoreact with the unsubstituted polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen. An epitope may be linear or conformational. A conformational epitope is composed of discontinuous sections of the antigen's amino acid sequence.

An "immunological response" to a composition or vaccine is the cellular and/or antibody-mediated immune response elicited by and to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The term "avian pathogen" as used herein refers to an infectious agent that is pathogenic in birds, including bacteria, viruses, yeast, nematodes, fungi etc. Particularly preferred in the present context are avian viruses and avian bacteria and more preferred avian viruses. Avian viruses of particular interest are infectious bursal disease virus (IBDV), New castle disease virus (NDV), infectious laryngotracheitis virus (ILTV), avian influenza virus (AIV) and avian infectious bronchitis virus (IBV).

As used herein, an "insertion site" refers to a region in a viral genome into which a heterologous polynucleotide is inserted and which is a nonessential region. The insertion sites of the present invention may be an intergenic region or intergenic locus, particularly the intergenic locus between UL3 and UL4 or the intergenic locus between UL3 and UL4 and between UL21 and UL22 in the unique long (UL) region of the genome, preferably the intergenic locus between UL3 and UL4. Insertion of one or more heterologous polynucleotides into one of these regions enables the production of a recombinant viral vector that can then be introduced into a chicken or other poultry for protection against one or more diseases. In some embodiments, a heterologous polynucleotide coding for and expressing an antigen of an avian pathogen as described herein may be inserted at an insertion site as disclosed herein in addition to one or more insertion sites known in the art. A suitable insertion site should allow insertion of a polynucleotide without affecting replication and growth of the virus. The skilled person will understand that in the context of a vaccine a suitable insertion site should also allow that the inserted polynucleotide elicits an appropriate immune response and protection against the respective avian pathogen.

The term "nonessential region" refers to a region of a virus genome which is not essential for replication and propagation of the virus in tissue culture of the host. Theoretically, any nonessential region or portion thereof can be deleted from the Gallid herpesvirus 3 strain SB-1 or HPRS24 genome or a foreign sequence can be inserted in it, and the viability and stability of the recombinant Gallid herpesvirus 3 vector resulting from the deletion or insertion can be used to ascertain whether a deleted region or portion thereof is indeed nonessential.

The term "intergenic locus" as used herein refers to the region between two genes and in the context of the present invention to the locus between two genes of the unique long (UL) region of a Gallid herpesvirus 3 (GaHV3, MDV-2) vector. Insertion into the intergenic locus at a specific location means that the transgene or heterologous polynucleotide is inserted between two genes, more specifically between two genes of the UL region, without replacing or deleting a gene. For example, the intergenic locus of UL3/UL4 and UL21/UL22 has a sequence as provided in SEQ ID NO: 1 and 2, respectively.

As used herein, the term "expression cassette" refers to the part of a vector comprising all elements required for the expression of a polynucleotide in a host cell. It contains one or more gene(s) coding for a protein and sequences controlling the expression, i.e. regulatory elements. Thus it comprises a promoter operably linked to an open reading frame (ORF) and a 3' untranslated region containing a transcription termination region. Additional elements are, e.g., an enhancer. The expression cassette may be part of a vector, typically an expression vector, including a plasmid or a viral vector. It may also be integrated in a chromosome by random or targeted integration, such as by homologous recombination or by viral integration. An expression cassette is prepared using cloning techniques and does therefore not refer to a natural occurring gene structure.

As used herein, the term "promoter" refers to a region of DNA that initiates transcription of a particular gene or open reading frame. Promoters are located in the 5' region near the transcription start site of genes on the same strand. Typically, a promoter is about 100 to 1000 base pairs long. Suitable promoters for use in vectors are well known in the art, such as a bacterial promoter, a viral promoter or the like. For example promoters useful in accordance without the present invention may include, but are not limited to, an immediate early cytomegalovirus (CMV) promoter, guinea pig CMV promoter, murine CMV promoter, SV40 promoter, pseudorabies virus promoters of glycoprotein X promoter, herpes simplex virus-1 alpha 4 promoter, chicken beta-actin promoter, rabbit beta-globin promoter, herpes simplex virus thymidine kinase promoter, Marek's Disease Virus promoters of glycoproteins, including any isolate or strain of MDV, such as MDV-1, MDV-2 and HTV, for example a promoter controlling expression of glycoproteins such as gC, gB, gE, or gI, infectious laryngotracheitis virus promoters such as those of glycoprotein gB, gE, gI, gD genes or any other suitable promoters.

The transcription termination region provides for efficient termination of transcription. The termination region may be from the same gene as the promoter sequence, or it may be from a different gene. It may further be from the same gene as the ORF, or it may be from a different gene.

The terms "identical" or "percent identity," in the context of two or more nucleic acids or polypeptide sequences, refer to two or more sequences or subsequences that are the same or have a specified percentage of amino acid residues or nucleotides that are the same (i.e. , about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (see, e.g. , the NCBI web site found at https://blast.ncbi.nlm.nih.gov/Blast.cgi or the like). Such sequences are then referred to as "substantially identical." This definition also refers to, or applies to, the compliment of a particular sequence. The definition may also include sequences that have deletions, additions, and/or substitutions.

For sequence comparison, one sequence typically serves as a reference sequence, to which other sequences are compared. When using a sequence comparison algorithm, reference and comparison sequences may be entered into a computer, and sequence algorithm program parameters are selected as desired. Percent sequence identities are then generated for the comparison sequences relative to the reference sequence, based on the parameters selected. An example of an algorithm that may be suitable for determining percent sequence identity and sequence similarity are the BLAST and BLAST 2.0 algorithms, which are described in Altschul et ciL , (Nuc Acids Res 25:3389-3402, 1977) and Altschul et ciL , (J Mol Biol 215:403-410, 1990), respectively. BLAST and BLAST 2.0 are well known in the art and may be used to determine percent sequence identity for any nucleic acids or proteins, such as those described herein. When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

As used herein, a "vaccine" or an "immunogenic composition" is meant to encompass a composition suitable for administration to a subject, such as an avian subject. In general a "vaccine" is sterile, and preferably free of contaminants that are capable of eliciting an undesirable response within the subject (e.g., the compound(s) in the vaccine is pharmaceutical grade) and contains an antigen. In the present context the vaccine contains at least the recombinant Gallid herpesvirus 3 vector of the invention as antigen and possibly a further Marek's disease virus vector as described herein. Vaccines may be designed for administration to subjects in need thereof via a number of different routes of administration including *in ovo*, oral, intravenous, buccal, rectal, parenteral, intraperitoneal, intradermal, intracheal, intramuscular, subcutaneous, inhalational, and the like. Preferably the vaccine is administered *in ovo,* intramuscularly or subcutaneously. The term "vaccinating" as used herein means conferring a protective immune response and hence protecting against a disease caused by an avian pathogen.

The terms "polyvalent vaccine", "combination or combo vaccine" and "multivalent vaccine" are used interchangeably to refer to a vaccine containing more than one antigen. The polyvalent vaccine may contain two, three, four or more antigens. The polyvalent vaccine may comprise recombinant viral vectors, active or attenuated or killed wild-type viruses, or a mixture of recombinant viral vectors and wild-type viruses in active or attenuated or killed forms.

The term "Marek's disease virus" or MDV as used herein refers to any alphaherpesvirus of the genus Mardivirus, including the herpesvirus of Turkeys (HVT), Marek's disease virus serotype 1 (MDV-1) and Gallid herpesvirus 3. Typically a MDV used as vaccine virus is Gallid herpesvirus 3 strain SB-1, naturally attenuated MDV-1 strain Rispens (CVI-988) and herpesvirus of turkeys (HTV) strain Fc126. As used herein, such a virus may include the genetic components of the virus, i.e., the genome and transcripts thereof, proteins encoded by the genome (including structural and nonstructural proteins), and functional or nonfunctional viral particles. The polynucleotide and polypeptide sequences encoding such viruses are well known in the art and would be easily found by one of skill in the art. MDV includes a wild type virus, a naturally attenuated wild type virus and a recombinant MDV. A recombinant MDV may comprise one or more heterologous polynucleotide(s) coding for and expressing at least one antigen of an avian pathogen.

The term "isolated DNA" as used herein means a DNA that has been substantially separated from, or enriched relative to, other substances with which it occurs in nature. Isolated DNA is usually at least about 80%, at least 90% pure, at least 98% pure, or at least about 99% pure, by weight and does only contain minimum amounts of contaminating DNA or protein.

The term "bacterial artificial chromosome" abbreviated as BAC as used herein refers to a DNA construct based on F-plasmid comprising an insert of about 150 to 350 kbp used for transforming and cloning in bacteria such as *Escherichia coli.* BAC vectors can harbor large DNA sequences, such as DNA virus genomes or DNA sequences coding for RNA virus genomes. This allows for efficient modification of viral genomes using well-established mutagenesis techniques in *E.coli,* such as CRISPR/Cas9, transcription activator-like effector nucleases (TALENs) or zinc-finger nucleases (ZFNs). Further encompassed is a cloning vector based on the bacterial P1-plasmid and often referred to as P1-derived artificial chromosome (PAC).

As used herein, a "pharmaceutically acceptable carrier," "pharmaceutically acceptable adjuvant," or "adjuvant" refers to an agent that modifies the effect of other agents and is useful in preparing a vaccine that is generally safe, non-toxic, and neither biologically nor otherwise undesirable. Such an agent may be added to a vaccine to modify the immune response of a subject by boosting the response such as to give a higher amount of antibodies and longer-lasting protection. Such an agent may include an excipient, diluent, carrier, or adjuvant that is acceptable for veterinary or pharmaceutical use. Such an agent may be non-naturally occurring, or may be naturally occurring, but not naturally found in combination with other agents in the immunogenic composition.

As used herein, an "antibody" refers to a polypeptide comprising a framework region from an immunoglobulin gene or fragments thereof that specifically binds and recognizes an antigen. The recognized immunoglobulin genes may include the kappa, lambda, alpha, gamma, delta, epsilon, and mu constant region genes, as well as the myriad immunoglobulin variable region genes. Light chains may be classified as either kappa or lambda. Heavy chains may be classified as gamma, mu, alpha, delta, or epsilon, which in turn define the immunoglobulin classes, IgY, IgG, IgM, IgA, IgD, and IgE, respectively.

### Recombinant Gallid herpesvirus 3 (GaHV3; MDV-2) vector

The present invention provides for a recombinant Gallid herpesvirus 3 (GaHV3; MDV-2) vector according to the claims, preferably a recombinant Gallid herpesvirus 3 (GaHV3; MDV-2) strain SB-1 vector, comprising one or more heterologous polynucleotide(s) coding for and expressing at least one antigen of an avian pathogen, inserted into the intergenic loci UL3/UL4 or UL3/UL4 and UL21/UL22 of the Gallid herpesvirus 3 vector. Preferably the vector comprises the one or more heterologous polynucleotide(s) inserted into the intergenic locus UL3/UL4 of the Gallid herpesvirus 3 vector. The heterologous polynucleotide may be incorporated anywhere within the intergenic loci UL3/UL4 or UL3/UL4 and UL21/UL22. The intergenic locus UL3/UL4 of strain SB-1 (HQ840738.1) has the nucleotide sequence of SEQ ID NO: 1 and the intergenic locus of UL21/UL22 of strain SB-1 has the nucleotide sequence of SEQ ID NO: 2. The nucleotide sequences of the intergenic loci UL3/UL4 and UL21/UL22 are the same for strain HPRS24 (NC_002577.1). The heterologous polynucleotide may be incorporated anywhere within the sequences of SEQ ID NO: 1 or SEQ ID NO: 2, preferably within nucleotides 10 to 90 of SEQ ID NO:1 or SEQ ID NO:2, more preferably within nucleotides 20 to 80 of SEQ ID NO:1 or SEQ ID NO:2, more preferably within nucleotides 30 to 70 of SEQ ID NO:1 or SEQ ID NO:2.

The avian pathogens may be Newcastle Disease Virus (NDV), Infectious Bursal Disease Virus (i.e., IBDV or Gumboro Disease virus), Marek's Disease Virus (MDV), Infectious Laryngotracheitis Virus (ILTV), avian infectious bronchitis virus (IBV), avian encephalomyelitis virus and other picornavirus, avian reovirus, avian paramyxovirus, avian metapneumovirus, avian influenza virus, avian adenovirus, fowl pox virus, avian coronavirus, avian rotavirus, avian parvovirus, avian astrovirus and chick anemia virus, coccidiosis (Eimeria sp.), Campylobacter sp., Salmonella sp., Mycoplasma gallisepticum, Mycoplasma synoviae, Pasteurella sp., Avibacterium sp., E. coli or Clostridium sp. Preferably the antigen of an avian pathogen is an antigen of an avian virus. More preferably, the recombinant vector comprises one or more heterologous polynucleotides coding for and expressing genes from infectious bursal disease virus (IBDV), infectious laryngotracheitis virus (ILTV) Newcastle disease virus (NDV), avian influenza virus (AIV), or the like.

A particularly suitable antigen of an avian pathogen according to the invention is an antigen of an avian virus such as infectious bursal disease virus (IBDV), infectious laryngotracheitis virus (ILTV), Newcastle disease virus (NDV), avian influenza virus (AIV) or avian infectious bronchitis virus (IBV). Exemplary antigens are VP2, VP3, VP4 and VPX of IBDV; glycoprotein B, glycoprotein I, glycoprotein D, glycoprotein E and glycoprotein C of IBDV, preferably glycoprotein B, glycoprotein I or glycoprotein E; Newcastle disease virus fusion protein (NDV-F) and viral hemagglutinin neuraminidase (NDV-NH) of NDV; avian influenza hemagglutinin (HA) or neuraminidase (NA) protein of avian influenza virus, preferably of avian influenza type A virus, more preferably avian influenza A serotype H5 hemagglutinin (H5 HA), avian Influenza A serotype H7 hemagglutinin (H7 HA), avian Influenza A serotype H9 hemagglutinin (H9 HA) or avian Influenza A serotype H5N1 neuraminidase (H5N1 NA); and S1 or S2 protein of IBV. The S1 and S2 protein are glycoproteins that are cleavage products of spike glycoprotein (S glycoprotein). Particularly preferred are antigens of IBDV, more preferred is the antigen VP2 of IBDV. The at least one antigen of an avian pathogen may also be combinations of two or more of the above listed antigens.

In certain embodiments, a recombinant viral vector of the invention may have a polynucleotide encoding an IBDV viral protein or gene product, such as an IBDV VP2 protein or gene product. In another embodiment, such a recombinant viral vector may have a polynucleotide encoding an infectious laryngotracheitis virus (ILTV) viral protein or gene product, such as a ILTV gB or gC or gD or gE or gI, UL-32 protein or gene product. In another embodiment, such a recombinant viral vector may have a polynucleotide encoding a NDV viral protein or gene product, such as a NDV F or HN protein or gene product. In another embodiment, such a recombinant viral vector may have a polynucleotide encoding an Avian Influenza Virus (AIV) viral protein or gene product, such as an AIV HA or NA protein or gene product. In another embodiment, such a recombinant viral vector may have a polynucleotide encoding an infectious bronchitis virus (IBV) viral protein or gene product, such as IBV S1 or S2 protein or gene product. A polynucleotide may have more than one gene, including a gene-fusion protein or gene product, such as a NDV F-HN fusion protein, chimera, or gene product. In some embodiments, the complete coding sequence of such a gene may be used such that a full-length or fully functional protein or polypeptide is produced. Alternatively, a portion or fragment of a viral protein or polypeptide may be sufficient to provide protection against a particular virus or viruses.

In a preferred embodiment, the recombinant viral vector of the invention has a polynucleotide encoding an IBDV viral protein or gene product, such as an IBDV VP2 protein or gene product. In one embodiment the heterologous polynucleotide coding for and expressing VP2 of IBDV has at least 70%, 75%, 80%, 85%, 90%, 95%, 96%, 97%, 98% or 99% sequence similarity to a polynucleotide having the sequence as set forth in SEQ ID NO: 10, preferably has at least 70%, 75%, 80%, 85%, 90%, 95%, 96%, 97%, 98% or 99% sequence identity to a polynucleotide having the sequence as set forth in SEQ ID NO: 6 and more preferably has a sequence of SEQ ID NO: 10. In another embodiment the antigen of an avian pathogen is VP2 and has at least 70%, 75%, 80%, 85%, 90%, 95%, 96%, 97%, 98% or 99% sequence similarity to a protein having the amino acid sequence as set forth in SEQ ID NO: 12, preferably has at least 70%, 75%, 80%, 85%, 90%, 95%, 96%, 97%, 98% or 99% sequence identity to a protein having the amino acid sequence as set forth in SEQ ID NO: 12, more preferably has a protein sequence of SEQ ID NO: 12. The antigen may also be an immunogenic fragment comprising at least eight or at least ten consecutive amino acids of SEQ ID NO: 12. Moreover the immunogenic fragment or the full-length protein of IBDV VP2 may be fused to an immunogenic fragment or full-length protein of another antigen of an avian pathogen.

The antigen of an avian pathogen may be infectious laryngotracheitis virus (Gallid herpesvirus 1) glycoprotein E (gE), glycoprotein I (gl) or glycoprotein B (gB) and has at least 70%, 75%, 80%, 85%, 90%, 95%, 96%, 97%, 98% or 99% sequence similarity to a protein having the amino acid sequence as set forth in SEQ ID NO: 31 (gE), SEQ ID NO: 32 (gl) or SEQ ID NO: 33 (gB), respectively, preferably has at least 70%, 75%, 80%, 85%, 90%, 95%, 96%, 97%, 98% or 99% sequence identity to a protein having the amino acid sequence as set forth in SEQ ID NO: 31 (gE), SEQ ID NO: 32 (gl) or SEQ ID NO: 33 (gB), respectively, more preferably has a protein sequence of SEQ ID NO: 31 (gE), SEQ ID NO: 32 (gl) or SEQ ID NO: 33 (gB), respectively. The antigen may also be an immunogenic fragment comprising at least eight or at least ten consecutive amino acids of SEQ ID NO: 31 (gE), SEQ ID NO: 32 (gl) or SEQ ID NO: 33 (gB), respectively. Moreover the immunogenic fragment or the full-length protein of ILTV gE, gI or gB may be fused to an immunogenic fragment or full-length protein of another antigen of an avian pathogen.

The antigen of an avian pathogen may be Newcastle disease virus fusion protein (NDV-F) or Newcastle disease virus hemagglutinin neuraminidase (NDV-NH) and has at least 70%, 75%, 80%, 85%, 90%, 95%, 96%, 97%, 98% or 99% sequence similarity to a protein having the amino acid sequence as set forth in SEQ ID NO: 34 (NDV-F) or SEQ ID NO: 35 (NDV-NH), respectively, preferably has at least 70%, 75%, 80%, 85%, 90%, 95%, 96%, 97%, 98% or 99% sequence identity to a protein having the amino acid sequence as set forth in SEQ ID NO: 34 (NDV-F) or SEQ ID NO: 35 (NDV-NH), respectively, more preferably has a protein sequence of SEQ ID NO: 34 (NDV-F) or SEQ ID NO: 35 (NDV-NH), respectively. The antigen may also be an immunogenic fragment comprising at least eight or at least ten consecutive amino acids of SEQ ID NO: 34 (NDV-F) or SEQ ID NO: 35 (NDV-NH), respectively. Moreover the immunogenic fragment or the full-length protein of NDV-F or NDV-NH may be fused to an immunogenic fragment or full-length protein of another antigen of an avian pathogen.

The antigen of an avian pathogen may be avian influenza A serotype H5 hemagglutinin (H5 HA), avian Influenza A serotype H7 hemagglutinin (H7 HA), avian Influenza A serotype H9 hemagglutinin (H9 HA) or avian Influenza A serotype H5N1 neuraminidase (H5N1 NA) and has at least 70%, 75%, 80%, 85%, 90%, 95%, 96%, 97%, 98% or 99% sequence similarity to a protein having the amino acid sequence as set forth in SEQ ID NO: 36 (H5 HA), SEQ ID NO: 37 (H7 HA), SEQ ID NO: 38 (H9 HA) or SEQ ID NO: 39 (H5N1 NA), respectively, preferably has at least 70%, 75%, 80%, 85%, 90%, 95%, 96%, 97%, 98% or 99% sequence identity to a protein having the amino acid sequence as set forth in SEQ ID NO: 36 (H5 HA), SEQ ID NO: 37 (H7 HA), SEQ ID NO: 38 (H9 HA) or SEQ ID NO: 39 (H5N1 NA), respectively, more preferably has a protein sequence of SEQ ID NO: 36 (H5 HA), SEQ ID NO: 37 (H7 HA), SEQ ID NO: 38 (H9 HA) or SEQ ID NO: 39 (H5N1 NA), respectively. The antigen may also be an immunogenic fragment comprising at least eight or at least ten consecutive amino acids of SEQ ID NO: 36 (H5 HA), SEQ ID NO: 37 (H7 HA), SEQ ID NO: 38 (H9 HA) or SEQ ID NO: 39 (H5N1 NA), respectively. Moreover the immunogenic fragment or the full-length protein of H5 HA, H7 HA, H9 HA or H5N1 NA may be fused to an immunogenic fragment or full-length protein of another antigen of an avian pathogen.

The antigen of an avian pathogen may be S1 or S2 glycoprotein of IBV and has at least 70%, 75%, 80%, 85%, 90%, 95%, 96%, 97%, 98% or 99% sequence similarity to a protein having the amino acid sequence as set forth in amino acids 25 to 530 of SEQ ID NO: 40 (S1) or in amino acids 553 to 1146 of SEQ ID NO: 40 (S2), respectively, preferably has at least 70%, 75%, 80%, 85%, 90%, 95%, 96%, 97%, 98% or 99% sequence identity to a protein having the amino acid sequence as set forth in amino acids 25 to 530 of SEQ ID NO: 40 (S1) or in amino acids 553 to 1146 of SEQ ID NO: 40 (S2), respectively, more preferably has a protein sequence of amino acids 25 to 530 of SEQ ID NO: 40 (S1) or amino acids 553 to 1146 of SEQ ID NO: 40 (S2), respectively. The antigen may also be an immunogenic fragment comprising at least eight or at least ten consecutive amino acids of amino acids 25 to 530 of SEQ ID NO: 40 (S1) or amino acids 553 to 1146 of SEQ ID NO: 40 (S2), respectively. Moreover the immunogenic fragment or the full-length protein of S1 or S2 protein may be fused to an immunogenic fragment or full-length protein of another antigen of an avian pathogen.

The antigens may also be an allelic variant of any of the antigens of an avian pathogen disclosed herein. The term "allelic variant" refers to a polynucleotide or a polypeptide containing polymorphisms that lead to changes in the amino acid sequences of a protein and that exist within a natural population (e.g., a virus species or variety). Such natural allelic variations can typically result in 1- 5% variance in a polynucleotide or a polypeptide. Allelic variants can be identified by sequencing the nucleic acid sequence of interest in a number of different species, which can be readily carried out by using hybridization probes to identify the same gene genetic locus in those species. Any and all such nucleic acid variations and resulting amino acid polymorphisms or variations that are the result of natural allelic variation and that do not alter the functional activity of gene of interest, are intended to be within the scope of the invention.

The polynucleotides used in the present invention may be codon optimized for a specific host. "Codon optimized" as used herein refers to a polynucleotide that is genetically engineered to increase its expression in a given species. To provide optimized polynucleotides coding for IBDV VP2 polypeptides, the DNA sequence of the VP2 protein gene can be modified to 1) comprise codons preferred by highly expressed genes in a particular species; 2) comprise an A+T or G+C content in nucleotide base composition to that substantially found in said species; 3) form an initiation sequence of said species; or 4) eliminate sequences that cause destabilization, inappropriate polyadenylation, degradation and termination of RNA, or that form secondary structure hairpins or RNA splice sites. Increased expression of VP2 protein in said species can be achieved by utilizing the distribution frequency of codon usage in eukaryotes and prokaryotes, or in a particular species.

The viral antigens coded for and expressed by the recombinant viral vector of the present invention may be encoded by a viral gene. However, one of skill in the art will appreciate in this regard that it may not be required to incorporate the entirety of a particular viral gene in order to obtain a desired immune response. Rather, a portion of such a gene may be sufficient. Optimization of a desired viral protein or polynucleotide encoding such a protein regardless of the length of the protein may be readily carried out using methods known in the art. One of skill in the art will further appreciate that modifications may be made to a viral gene or genes, or the proteins encoded thereby, to increase the immunogenicity of the viral protein when introduced into the subject.

Moreover, the recombinant Gallid herpesvirus 3 vector may comprise more than one heterologous polynucleotide coding for and expressing at least one antigen of an avian pathogen. In one embodiment the vector of the invention comprises two or three heterologous polynucleotides, preferably two. Preferably at least one of the heterologous polynucleotides codes and expresses IBDV VP2 as described above.

Thus, in some embodiments, the recombinant viral vector may express one heterologous protein from a single virus species or may express more than one heterologous protein from a single virus species different to Gallid herpesvirus 3 in order to obtain protection against MDV and a further disease caused by an avian virus. For instance, in one embodiment, the invention provides a recombinant viral vector according to the claims comprising the GalHV3 genome and a polynucleotide coding for and expressing an antigen of a different pathogen, thus providing protection in an avian species such as poultry against Marek's disease, and at least one other disease caused by a different avian pathogen. Specifically the invention provides a recombinant viral vector comprising the GalHV3 genome and a polynucleotide coding for and expressing an antigen of a different virus, thus providing protection in an avian species such as poultry against Marek's disease, and at least one disease caused by a different avian viral pathogen. For example, the recombinant viral vector in accordance with the invention may provide protection in avian species such as poultry against MDV and IBDV, or may provide protection against MDV and ILTV, or may provide protection against MDV and NDV, or may provide protection against MDV and AIV, or may provide protection against MDV and IBV. Preferably, the recombinant viral vector in accordance with the invention may provide protection in avian species such as poultry against MDV and IBDV

In other embodiments, the recombinant viral vector according to the claims may express heterologous proteins from more than one virus species in order to obtain protection to multiple viruses in addition to MDV. For instance, in one embodiment, the invention provides a recombinant viral vector ccording to the claims comprising the GalHV3 genome and at least two polynucleotides coding for and expressing an antigen of two different pathogens, thus providing protection in an avian species such as poultry against Marek's disease, and at least two other diseases caused by avian pathogens. Specifically the invention provides a recombinant viral vector comprising the GaHV3 genome and at least two heterologous polynucleotides coding for and expressing antigens of two different viruses, thus providing protection in avian species such as poultry against Marek's disease, and at least two other disease caused by avian viral pathogen. For example, the recombinant viral vector in accordance with the invention may provide protection in avian species such as poultry against MDV, IBDV and one of the diseases selected from NDV, AIV and IBV; or against MDV, NDV and one of the diseases selected from IBDV, AIV and IBV; or against MDV, AIV and one of the diseases selected from IBDV, NDV and IBV; or against MDV, IBV and one of the diseases selected from IBDV, NDV and AIV. Preferably, the recombinant viral vector in accordance with the invention may provide protection in avian species such as poultry against MDV, IBDV and one of the diseases selected from NDV, AIV and IBV.

In case the recombinant viral vector comprises more than one heterologous polynucleotide, the polynucleotides may be inserted at the same insertion site or at different insertion sites. At least one of the heterologous polynucleotides is inserted into the intergenic locus of UL3/UL4. Further the more than one heterologous polynucleotide may be coded for and expressed by the same expression cassette using one promoter or by separate expression cassettes.

Thus, in accordance with the invention, the recombinant viral vector may comprise one or more transgene(s) operatively linked to one or more promoters for expression of one or more viral proteins or peptides or fragments or portions thereof. In some embodiments, a single transgene may be operably linked to a single promoter, or more than one transgene may be operatively linked to a single promoter. In other embodiments, more than one transgene may be present in a recombinant vector wherein a first transgene is operatively linked to a first promoter and a second transgene is operatively linked to a second promoter.

Successful expression of the inserted cDNA genetic sequence by the modified infectious virus requires two conditions. First, the insertion must be into a non-essential region of the genome of the virus in order for the modified virus to remain viable (intact replication and amplification), e.g., by insertion into the intergenic loci UL3/UL4 or UL3/UL4 and UL21/UL22. The second condition for expression of inserted cDNA is the presence of a regulatory sequences allowing expression of the gene in the viral background (for instance: promoter, enhancer, donor and acceptor splicing sites and intron, Kozak translation initiation consensus sequence, polyadenylation signals, untranslated sequence elements).

In general, it is advantageous to employ a strong promoter functional in eukaryotic cells. The promoters include, but are not limited to, an immediate early cytomegalovirus (CMV) promoter, guinea pig CMV promoter, murine CMV promoter an SV40 promoter, pseudorabies virus promoters such as that of glycoprotein X promoter, herpes simplex virus- 1 such as the alpha 4 promoter, chicken beta-actin promoter, rabbit beta-globin promoter, herpes simplex virus thymidine kinase promoter, Marek's Disease Viruses (including MDV-1, MDV-2 and HVT) promoters such as those driving glycoproteins gC, gB, gE, or gI expression, infectious laryngotracheitis virus promoters such as those of glycoprotein gB, gE, gI, gD genes, or other herpesvirus promoters. When the insertion locus consists of a SB-1 gene (for instance, gC, gD, US2 or US 10 genes), the foreign gene can be inserted into the vector with no additional promoter sequence since the promoter of the deleted gene of the vector will drive the transcription of the inserted foreign gene.

An intermediate recombinant Gallid herpesvirus 3 (GaHV3; MDV-2) vector comprising one or more maker(s)

In a further aspect the invention relates to an intermediate product used for the production of the recombinant Gallid herpesvirus 3 of the invention. This intermediate recombinant Gallid herpes virus 3 vector comprises one or more marker(s) inserted into the intergenic loci UL3/UL4 or UL3/UL4 and UL21/UL22 of the Gallid herpesvirus 3 vector, wherein the recombinant Gallid herpesvirus 3 vector is preferably a Gallid herpesvirus 3 strain SB-1 vector. Preferably the one or more marker(s) is/are inserted into the intergenic locus of UL3/UL4.

The marker may be a selection marker gene, a reporter gene or a DNA bar code. Selection markers may encode for example, biocide resistance, or antibiotic resistance (e.g., kanamycin, G418, bleomycin, hygromycin, etc.). A preferred selectable marker is *E.coli* galactokinase gene K (galK), which confers sensitivity to 2-desoxy-galactose (2-DOG), but allows survival and growth on galactose as the only carbon source. Another preferred selectable marker is kan/sacB, an expression cassette containing the neomycin (kanamycin) gene from Tn5 and the *sacB* gene from *Bacillus subtilis.* Selectable markers are well known to one of skill in the art and may include any markers suitable for use in accordance with the invention. Reporter genes may be used to monitor expression, but usually do not result in death of a cell. Suitable reporter genes include for example, a β-glucuronidase or uidA gene (GUS), one or more of the various fluorescent protein genes, such as green fluorescent protein (GFP), red fluorescent protein (RFP), or any one of a large family of proteins which fluorescence at characteristic wavelengths, a β-lactamase gene, a gene that encodes an enzyme for which various chromogenic substrates are known, a luciferase gene, a xylE gene, which encodes a catechol dioxygenase that converts chromogenic catechols, an oc-amylase gene, a tyrosinase gene, which encodes an enzyme capable of oxidizing tyrosine to DOPA and dopaquinone, which in turn condense to melanin, or an a-galactosidase, which catalyzes a chromogenic a-galactose substrate. A DNA bar code does not have a specific function other than having a detectable and unique sequence. Preferably the marker is a selection marker gene or a reporter gene. More preferably the intermediate recombinant Gallid herpesvirus 3 vector comprises one or more expression cassette(s) comprising a selection marker gene and/or a reporter gene. In a preferred embodiment the selection marker gene or reporter gene is galK or a kan/sacB combination. The marker may serve as a placeholder at the intergenic loci of UL3/UL4 or UL3/UL4 and UL21/UL22 for the at least one antigen of an avian pathogen, as it can be easily replaced by the at least one antigen of an avian pathogen as described below.

### In vitro methods for producing a recombinant Gallid herpesvirus 3 (GaHV3; MDV-2) vector

The present invention further provides an *in vitro* method for producing a recombinant Gallid herpesvirus 3 vector comprising the introduction of one, two or more polynucleotides into the intergenic loci UL3/UL4 or UL3/UL4 and UL21/UL22 of the Gallid herpesvirus 3 vector, wherein the Gallid herpesvirus 3 vector is preferably a Gallid herpesvirus 3 strain SB-1 vector.

In one embodiment the *in vitro* method of producing a recombinant Gallid herpesvirus 3 vector comprises (a) providing a Gallid herpesvirus 3 vector, (b) inserting one or more heterologous polynucleotide(s) coding for and expressing at least one antigen of an avian pathogen into the intergenic loci UL3/UL4 or UL3/UL4 and UL21/UL22 of the Gallid herpesvirus 3 vector, and optionally (c) amplifying the Gallid herpesvirus 3 vector comprising one or more heterologous polynucleotide(s) coding for at least one antigen of an avian pathogen of step (b).

In another embodiment the *in vitro* method of producing a recombinant Gallid herpesvirus 3 vector comprises (a) providing a Gallid herpesvirus 3 vector comprising one or more marker(s) in the intergenic loci UL3/UL4 or UL3/UL4 and UL21/UL22 of the Gallid herpesvirus 3 vector, (b) replacing the one or more marker(s) with an expression cassette comprising one or more heterologous polynucleotides coding for at least one antigen of an avian pathogen, and (c) amplifying the Gallid herpesvirus 3 vector comprising one or more heterologous polynucleotide(s) coding for at least one antigen of an avian pathogen of step (b). The recombinant Gallid herpes virus 3 may be coded for and expressed by a bacterial artificial chromosome (BAC) or a P1-derived artificial chromosome (PAC), preferably a BAC. Thus, step (a) may comprise providing a bacterial artificial chromosome or a P1-derived artificial chromosome comprising a polynucleotide coding for the (recombinant) Gallid herpesvirus 3 vector. The skilled person would understand that the advantage of using BAC or PAC as a vector for the Gallid herpesvirus 3 is that the insertion of the one or more heterologous polynucleotide(s) and the amplification can be performed in *E.coli.* This also includes a screening step for the desired viral vector. The method may therefore further comprise the steps of (d) isolating the BAC or PAC comprising a polynucleotide coding for the recombinant Gallid herpesvirus 3 vector comprising one or more heterologous polynucleotide(s) coding for at least one antigen of an avian pathogen of step (b) and (e) transfecting chicken embryonic fibroblasts with the BAC or PAC of step (d).

As outlined above the term "amplifying the Gallid herpes virus 3 vector" relates to amplification in *E.coli* or in avian host cells such as chicken embryonic fibroblasts, depending on the Gallid herpes virus 3 vector used. In *E.coli* this involves culturing *E.coli* transfected with the plasmid (BAC or PAC) coding for the desired viral vector under suitable condition and isolating the plasmid DNA. In avian cells, the cells containing the desired viral vector are cultured and either the supernatant or whole cells containing the desired viral vector are harvested. For propagation of the virus, the harvested desired viral vector may be used directly for transducing avian host cells or may be frozen and stored before further use.

The term "replacing the one or more marker(s)" as used herein means inserting an expression cassette at the site of the one or more marker(s) by any methods for targeted integration known to the person skilled in the art. E.g., the expression cassette comprising one or more heterologous polynucleotide coding for at least one antigen of an avian pathogen may be inserted by homologous recombination. Alternatively the one or more marker(s), preferably an expression cassette coding for the one or more marker(s), may be flanked by recognition sites (e.g., IoxP or FRT sites) for a site specific recombinase (e.g., Cre or Flp recombinase) and one or more marker(s) are then replaced by an expression cassette comprising one or more heterologous polynucleotide coding for at least one antigen of an avian pathogen, wherein the expression cassette is also flanked by recognition sites for the same site specific recombinase using Cre-loxP recombination or Flp-FRT recombination technology.

A recombinant Gallid herpesvirus-3 (MDV-2) viral vector may be constructed as described in WO 2013/057235 and WO 2017/027324 in two steps. First, the Gallid herpesvirus-3 (MDV-2) genomic regions flanking the locus of insertion are cloned into an *E.coli* plasmid construct; unique(s) restriction site(s) is (are) placed between the two flanking regions (insertion plasmid) in order to allow the insertion of the donor expression cassette DNA. Separately, the cDNA or DNA gene sequence to be inserted is preceded by a promoter region (gene start region) and a terminator (or poly-adenylation,poly-A) sequence which is specific for the Gallid herpesvirus-3 (MDV-2) vector and/or eukaryotic cells, such as mammalian cells. The whole expression cassette (promoter-transgene-poly-A) is then cloned into the unique(s) restriction site(s) of the insertion plasmid to construct the "donor plasmid" which contains the expression cassette flanked by Gallid herpesvirus-3 (MDV-2) "arms" flanking the insertion locus. The resulting donor plasmid construct is then amplified in *E.coli* and plasmid DNA is extracted. The plasmid is then linearized using a restriction enzyme that cuts the plasmid backbone (outside the Gallid herpesvirus-3 (MDV-2) arms and expression cassette). Chicken embryo fibroblasts are then co-transfected with parental Gallid herpesvirus-3 (MDV-2) DNA and linearized donor plasmid DNA. The resulting virus population is then cloned by multiple limiting dilution steps where recombinant viral vector expressing the transgene is isolated from the non-expressing viral population. Similarly, another foreign cassette can be inserted in another locus of insertion to create a double Gallid herpesvirus-3 (MDV-2) vector expressing two recombinant genes (polynucleotides). The second cassette can also be inserted into the same locus. The recombinant Gallid herpesvirus-3 (MDV-2) is produced in primary chicken embryo fibroblasts similarly to the parental Gallid herpesvirus-3 (MDV- 2) strain SB-1 MD vaccine. After incubation, infected cells are harvested, mixed with a freezing medium allowing survival of infected cells, and frozen usually in cryovial or glass ampoules and stored in liquid nitrogen.

Alternatively, a recombinant Gallid herpesvirus-3 (MDV-2) vector may be constructed as bacterial artificial chromosome (BAC) or as P1-derived artificial chromosome (PAC). Specifically, a pSB-1 BAC clone (Petherbridge L et al., J Virol Methods. 2009; 158: 11-7) comprising the entire Gallid herpesvirus 3 strain SB-1 genome (Genebank Accession Number HQ840738.1 may be used to generate a recombinant virus with a galK expression cassette (SEQ ID NO: 8) inserted into the indicated locus. The primers (such as the primers listed in Table 2) have homologous sequences for the exact nucleotides of the region where the fragment is going to be inserted. Homologous recombination between the homologous sequences from the primers and the nucleotide sequence of the locus in the vector results in the insertion of the fragment. Positive colonies are selected based on their ability to utilise galactose as the sole carbon source in a minimal media. The galK expression cassette (SEQ ID NO: 8) may than be replaced by the VP2 expression cassette (SEQ ID NO: 9) or any other heterologous polynucleotide expression cassette. Positive colonies are selected based on their ability to grow in the presence of 2-deoxy-galactose and the integration of the VP2 expression cassette is confirmed by specific PCR and sequencing. Recombinant Gallid herpesvirus vector according to the invention is isolated from *E.coli* and CEF are transfected with the BAC DNA encoding the recombinant Gallid herpesvirus-3 vector of the invention and reconstituted viruses are passaged to generate working virus stocks.

### Vaccines

In some aspects the recombinant Gallid herpesvirus 3 vector comprising one or more heterologous polynucleotide according to the invention may be used as a vaccine, i.e., an immunogenic composition, for administering to a subject, such as a chicken or other poultry in order to provide protection from one or more avian viruses. Thus, the vaccine comprises a recombinant Gallid herpesvirus 3 (GaHV3; MDV-2) vector comprising one or more heterologous polynucleotide(s) coding for and expressing at least one antigen of an avian pathogen, inserted into the intergenic loci UL3/UL4 or UL3/UL4 and UL21/UL22 of the Gallid herpesvirus 3 vector, wherein the recombinant Gallid herpesvirus 3 vector is preferably a recombinant Gallid herpesvirus 3 strain SB-1 vector.

The vaccine of the present invention may comprise a recombinant Gallid herpesvirus-3 (MDV-2) vector of the present invention and a pharmaceutically or veterinarily acceptable carrier, excipient or adjuvant.

In another embodiment, the vaccine comprises: i) a recombinant Gallid Herpesvirus-3 (MDV-2) vector comprising one or more heterologous polynucleotides coding for and expressing at least one antigen of an avian pathogen inserted into the intergenic loci UL3/UL4 or UL3/UL4 and UL21/UL22 of the Gallid herpesvirus 3 vector, wherein the recombinant Gallid herpesvirus 3 vector is preferably a recombinant Gallid herpesvirus 3 strain SB-1 vector; and ii) at least a further Marek's disease virus (MDV) vector. The further MDV vector is preferably selected from the group consisting of Gallid herpesvirus 3 vector, naturally attenuated MDV-1 strain Rispens (CVI-988) vector and herpesvirus of turkeys (HTV or MDV-3) strain Fc126 vector, more preferably from Gallid herpesvirus 3 strain SB-1 vector, naturally attenuated MDV-1 strain Rispens (CVI-988) vector and herpesvirus of turkeys (HTV or MDV-3) strain Fc126 vector. The further MDV vector may be a wild type vector, preferably selected from the group consisting of Gallid herpesvirus 3 vector, naturally attenuated MDV-1 strain Rispens (CVI-988) vector and herpesvirus of turkeys (HTV or MDV-3) strain Fc126 vector or a recombinant MDV vector preferably derived from a MDV vector selected from the group consisting of Gallid herpesvirus 3 vector, naturally attenuated MDV-1 strain Rispens (CVI-988) vector and herpesvirus of turkeys (HTV or MDV-3) strain Fc126 vector.

The recombinant Marek's disease virus (MDV) vector may comprise one or more heterologous polynucleotide(s) coding for and expressing at least one antigen of an avian pathogen. The recombinant MDV vector can be derived from the naturally attenuated MDV-1 strain Rispens (CVI-988) vector or the herpesvirus of turkeys (HTV or MDV-3) strain Fc126 vector. The recombinant MDV vector may also be a second recombinant Gallid herpesvirus 3 vector, preferably a recombinant Gallid herpesvirus 3 strain SB-1 vector, in addition to the first recombinant Gallid herpesvirus 3 vector of the invention. The person skilled in the art would understand that the second recombinant Gallid herpesvirus 3 vector is different to the first recombinant Gallid herpesvirus 3 vector in that they express different heterologous polynucleotides. Thus, the second recombinant Gallid herpesvirus 3 vector comprises at least one heterologous polynucleotide coding for and expressing a different antigen of an avian pathogen as the one or more heterologous polynucleotides of the first recombinant Gallid herpesvirus 3 vector. The person skilled in the art would further understand that the two recombinant Gallid herpesvirus 3 vectors can express different heterologous polynucleotides and hence may confer protection to different diseases caused by an avian pathogen in addition to Marek's disease. This second recombinant Gallid herpesvirus 3 vector may likewise comprise the one or more heterologous polynucleotide(s) inserted into the intergenic loci UL3/UL4 or UL3/UL4 and UL21/UL22 of said second Gallid herpesvirus 3 vector, preferably into the intergenic locus UL3/UL4. While the first and the second recombinant Gallid herpesvirus 3 vector are preferably recombinant Gallid herpesvirus 3 strain SB-1 vectors, they may also be derived of different strains such as the first vector being derived from a SB-1 strain and the second vector from a HPRS24 strain or the first vector being derived from a HPRS24 strain and the second from a HPRS24 strain. Preferably the (first) recombinant Gallid herpesvirus 3 vector and the further Marek's disease virus as described above are administered together in one dosage form. Alternatively the (first) recombinant Gallid herpesvirus 3 vector and the further Marek's disease virus as described above may be administered in separate dosage form at the same time or at different time points.

The vaccine according to the invention is designed to generate antibody immunity and/or cellular immunity in a subject. The vaccine may further comprise a pharmaceutically acceptable excipient, carrier or adjuvant. A pharmaceutically acceptable excipient, carrier or adjuvant may be a substance that enhances an immune response in a subject to an exogenous antigen, including but not limited to, adjuvants, liposomes, biodegradable microspheres. A pharmaceutically acceptable carrier or adjuvant may contain a substance designed to protect the antigen from rapid catabolism, such as aluminum hydroxide or mineral oil, or a stimulator of immune responses, such as proteins derived from *Bordetella pertussis* or *Mycobacterium tuberculosis.* The vaccines may comprise or consist essentially of one or more adjuvants. Suitable adjuvants for use in the practice of the present invention are (1) polymers of acrylic or methacrylic acid, maleic anhydride and alkenyl derivative polymers, (2) immunostimulating sequences (ISS), such as oligodeoxyribonucleotide sequences having one or more non-methylated CpG units (Klinman et al, 1996; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, e.g., DDA (5) cytokines, (6) aluminum hydroxide or aluminum phosphate, (7) saponin or (8) other adjuvants discussed in any document cited in the instant application, or (9) any combinations or mixtures thereof. Commercially available adjuvants may include for example, Freund's Incomplete Adjuvant and Complete Adjuvant, Merck Adjuvant 65, aluminum salts such as aluminum hydroxide gel (alum) or aluminum phosphate; CpG oligonucleotides, salts of calcium, iron or zinc; an insoluble suspension of acylated tyrosine; acylated sugars; cationically or anionically derivatized polysaccharides; polyphosphazenes; biodegradable microspheres; and monophosphoryl lipid A.

One of skill in the art will be able to identify appropriate pharmaceutically acceptable carriers for use with the present invention. A suitable pharmaceutically acceptable carrier may be determined in part by the particular composition being administered, as well as by the particular method used to administer the composition. Accordingly, a wide variety of suitable formulations of vaccines are available that may be of use in the present invention. A suitable pharmaceutically acceptable carrier includes aqueous and nonaqueous, isotonic sterile injection solutions, which can contain antioxidants, buffers, bacteriostats, and solutes that render the formulation isotonic with the blood of the intended subject, and aqueous and nonaqueous sterile suspensions that can include suspending agents, solubilizers, thickening agents, stabilizers, and preservatives. The pharmaceutically acceptable carrier or excipients may be any compound or combination of compounds facilitating the administration of the vector (or protein expressed from an inventive vector in vitro), or facilitating transfection or infection and/or improve preservation of the vector (or protein). Pharmaceutically acceptable carrier or excipients that can be used for methods of this invention include, but are not limited to, 0.9% NaCl (e.g., saline) solution or a phosphate buffer, poly-(L-glutamate) or polyvinylpyrrolidone. Such vaccines may also comprise buffers (e.g. , neutral buffered saline or phosphate buffered saline), carbohydrates (e.g. , glucose, mannose, sucrose or dextrans), mannitol, proteins, polypeptides or amino acids such as glycine, antioxidants, bacteriostats, chelating agents such as EDTA or glutathione, adjuvants (e.g., aluminum hydroxide), solutes that render the formulation isotonic, hypotonic, or weakly hypertonic with the blood of a subject, suspending agents, thickening agents, and/or preservatives. Alternatively, compositions of the present invention may be formulated as a lyophilizate. Injection solutions and suspensions may be prepared from sterile powders, granules, and tablets.

Administration may be in any convenient manner, e.g., by injection, oral administration, inhalation, transdermal application, or rectal administration. Administration may also be by injection *in ovo.* Injection of a recombinant viral vector or a vaccine as described herein may be provided to a subject such as poultry in a single administration or dose, or may be administered more than once, such as in repeated doses.

A vaccine may generally be used for prophylactic and/or therapeutic purposes. For example, the vaccine may be provided to a subject, such as an avian species, to vaccinate against one or more diseases caused by one or more avian pathogens. Typically the vaccine is administered prior to infection with or exposure to an avian pathogen in order to provide protection against infection with one or more avian pathogen or development of clinical symptoms caused by one or more avian pathogen. Preferably the avian pathogen is an avian virus, more preferably the avian pathogen is Marek's disease virus and one or more further avian virus. The one or more further avian viruses are preferably infectious bursal disease virus (IBDV), infectious laryngotracheitis virus (ILTV), Newcastle disease virus (NDV), avian influenza virus or avian infectious bronchitis virus (IBV). In order to provide protection or vaccinate against an avian pathogen the vaccine is administered at an early age, preferably *in ovo*, such as in 18 d old embryonated eggs, such as in chicken eggs, or in chicks, preferably in 1 day old chicks. In another embodiment, the vaccine may be also provided to a subject, such as a bird, after infection with or exposure to one or more avian pathogen in order to provide treatment of the pathogen in the subject, such as by reducing or eliminating infection in the subject.

Vaccines according to the invention may be provided in single-dose or multi-dose containers, such as sealed ampoules or vials. Such containers may be sealed to preserve sterility of the composition until use. In general, compositions as described herein may be stored as suspensions, solutions, or emulsions in oily or aqueous vehicles. Alternatively, such a composition may be stored in a freeze-dried condition requiring only the addition of a sterile liquid carrier immediately prior to use.

The dose administered to a subject should be sufficient to affect a beneficial prophylactic response in the subject over time, such as protecting a subject, such as a chicken or other poultry, against clinical symptoms caused by one or more avian pathogens or vaccinating against one or more diseases caused by one or more avian pathogens. A protective dose typically has about 3000 to 5000 pfu of the recombinant Gallid herpesvirus 3 of the invention.

### Use of the vaccine

Described herein is a method for inducing an immunological response in an animal against one or more antigens or a protective response in an animal against one or more avian pathogens, which method comprises inoculating the animal at least once with the vaccine of the present invention. Further described herein is a method for inducing an immunological response in an animal to one or more antigens or a protective response in an animal against one or more avian pathogens in a prime-boost administration regimen, which is comprised of at least one primary administration and at least one booster administration using at least one common polypeptide, antigen, epitope or immunogen. The vaccine used in primary administration may be same or may be different from those used as a booster.

Specifically the vaccine of the present invention is used for vaccinating an avian species against one or more diseases caused by one or more avian pathogens preferably against Marek's disease and one or more diseases caused by one or more avian pathogens. In some embodiments the vaccine of the present invention is for use in protecting an avian species against clinical symptoms caused by one or more avian pathogens, preferably against clinical symptoms caused by Marek's disease virus and clinical symptoms caused by one or more avian pathogens.

Described herein is a method of treating an avian species for protection against Marek's Disease and one or more diseases caused by one or more avian pathogens comprising the step of administering an effective amount of the vaccine according to the invention.

The animal to be vaccinated is an avian species and typically poultry. Suitable animals are, e.g., turkey, chicken, quail, ducks geese or pigeons. Preferably the animal to be vaccinated is turkey or chicken, more preferably chicken.

The one or more avian pathogens causing the disease or clinical symptoms is preferably an avian pathogen selected from the group consisting of Newcastle disease virus (NDV), infectious bursal disease virus (IBDV), avian infectious laryngotracheitis virus (ILTV), avian influenza virus (AIV) or avian infectious bronchitis virus (IBV).

Other suitable avian pathogens are avian encephalomyelitis virus and other picornavirus, avian reovirus, avian paramyxovirus, avian metapneumovirus, avian adenovirus, fowl pox virus, avian coronavirus, avian rotavirus, avian parvovirus, avian astrovirus and chick anemia virus, coccidiosis (Eimeria sp.), Campylobacter sp., Salmonella sp., Mycoplasma gallisepticum, Mycoplasma synoviae, Pasteurella sp., Avibacterium sp., E. coli or Clostridium sp. Preferably the avian pathogen is an avian virus.

Preferred diseases vaccinated or protected against that are caused by an avian pathogen are Newcastle disease (ND) caused by Newcastle disease virus (NDV), infectious bursal disease (IBD) caused by infectious bursal disease virus (IBDV), avian infectious laryngotracheitis (ILT) caused by avian infectious laryngotracheitis virus (ILTV), avian influenza caused by avian influenza virus (AIV) or avian infectious bronchitis (IB) caused by avian infectious bronchitis virus (IBV).

Usually, one administration of the vaccine is performed either *in ovo* or in chicks. Chicks are preferably administered at one day-of-age by the subcutaneous or intramuscular route. In ovo administration is typically performed in 17-19 day-old embryonated eggs, preferably in 18 day-old embryonated eggs, e.g., from chicken. The animals are preferably at least 17-day-embryo or one day old at the time of the first administration. A second administration can be done within the first 10 days of age.

The recombinant Gallid herpesvirus 3 (GaHV3; MDV-2) vector or the vaccine of the invention is used for protecting an avian species against clinical symptoms caused by infectious bursal disease virus. The skilled person would understand that the at least one antigen of an avian pathogen coded for and expressed by one or more heterologous polynucleotides would be an antigen of infectious bursal disease virus, preferably VP2 of infectious bursal disease virus.

A variety of administration routes in day-old chicks may be used such as subcutaneously or intramuscularly, intradermally, transdermally. The *in ovo* vaccination can be performed in the amniotic sac and/or the embryo. Commercially available *in ovo* and s.c. administration devices can be used for vaccination.

### Examples

### Cloning of qalK expression cassette into SB-1 virus genome

The pSB-1 BAC clone (Petherbridge L et al., J Virol Methods. 2009; 158: 11-7) comprising the entire Gallid herpesvirus 3 strain SB-1 genome (GenBank Accession Number: HQ840738.1) was used to generate different recombinant viruses with a galK expression cassette (SEQ ID NO: 8) inserted into the indicated locus by employing a homologous recombination based technique using recombination proteins provided from lambda phage. Briefly, galK expression cassette was amplified by specific primers (Table 2) tagged for the specific locus where the insertion is going to occur. E.coli strain SW102 harboring pSB-1 BAC was transformed with the amplified fragment. With the aid of homologous recombination, the fragment was inserted into the exact location directed by the homologous sequences (tags). For example primer pair UL3/4F-GalKF and UL3/4R-GalKR tag the galK cassette with UL3/4 sequence, so that the fragment will be inserted into the UL3/UL4 region by homologous recombination. Positive colonies were selected based on their ability to utilise galactose as the sole carbon source in a minimal media. Protocols for the galK selection-based recombineering approach have been described (Zhao Y, Nair V. Mutagenesis of the Repeat Regions of Herpesviruses Cloned as Bacterial Artificial Chromosomes. In: Braman J, editor. In Vitro Mutagenesis Protocols. 3rd ed: Humana Press; 2010. p. 53-74; Warming S et al., Nucleic Acids Res. 2005; 33:e36). The integration of the galK expression cassette was confirmed by specific PCR and sequencing.

Exemplary insertion sites of UL3/4, UL10/11 and UL21/22 are shown below. The position of the exact insertion site is shown in bold, highlighting the two nucleotides 5' and 3' of the insertion site. The numbers at the 5' and 3' end of the sequences shown in Table 1 as well as the numbers indicated for the insertion site refer to the Gallid herpesvirus 3 strain SB-1 genome (GenBank accession number HQ840738.1).

**Table 1: Insertion loci**

| **Insertion locus** | **SEQ ID NO** | **Sequence** | **Insertion site** |
|---|---|---|---|
| UL3/4 | 1 | | 19516-insertion-19517 (between nucleotides 61 and 62 of SEQ ID NO:1) |
| UL21/22 | 2 | | 49949-insertion-49950 (between nucleotides 49 |
| | | | and 50 of SEQ ID NO:2) |
| UL10/11 | 3 | | 32274-insertion-32275 |
| UL40/41 | 4 | | 93960-insertion- 93961 |
| UL50/51 | 5 | | 109750-insertion- 109751 |
| UL26/27 | 6 | | 58495- insertion- 58496 |
| UL45/46 | 7 | | 101292- insertion-101344 |

**Table 2: Primers used for generating the galK expression cassette**

| Primer | Sequence | SEQ ID NO: |
|---|---|---|
| UL21/22F-GalKF | | SEQ ID NO: 13 |
| UL21/22R-GalKR | | SEQ ID NO: 14 |
| UL26/27F-GalKF | | SEQ ID NO: 15 |
| UL26/27R-GalKR | | SEQ ID NO: 16 |
| UL3/4F-GalKF | | SEQ ID NO: 17 |
| UL3/4R-GalKR | | SEQ ID NO: 18 |
| UL10/11F-GalKF | | SEQ ID NO: 19 |
| UL10/11R-GalKR | | SEQ ID NO: 20 |
| UL40/41F-GalKF | | SEQ ID NO: 21 |
| UL40/41R-GalKR | | SEQ ID NO: 22 |
| UL50/51F-GalKF | | SEQ ID NO: 23 |
| UL50/51R-GalKR | | SEQ ID NO: 24 |

### Cloning of IBDV VP2 expressing cassette into SB-1 virus genome

The pSB-1 BAC clones comprising a galK expression cassette (SEQ ID NO: 8) in the UL3/4 (SEQ ID NO: 1), UL21/22 (SEQ ID NO: 2) or UL10/11 (SEQ ID NO:3) intragenic locus of the SB-1 virus genome were used to generate three different recombinant viruses pSB-1-UL3/4VP2, pSB-1-UL21/22VP2 and pSB-1-UL10/11VP2 that contain the IBDV VP2 expression cassette (SEQ ID NO:9). The galK expression cassette (SEQ ID NO: 8) was replaced by the VP2 expression cassette (SEQ ID NO: 9) amplified from the recombinant HVT expressing IBDV VP2 (Darteil R et al., Virology 1995; 211: 481-90) comprising a nucleotide sequence coding for VP2 (SEQ ID NO: 10) and a murine cytomegalovirus (IE) promoter and enhancer sequence (SEQ ID NO: 11). Positive colonies were selected based on their ability to grow in the presence of 2-deoxy-galactose (Warming S et al., Nucleic Acids Res. 2005; 33:e36) and the integration of the VP2 expression cassette was confirmed by specific PCR and sequencing.

### Cell culture and virus propagation

Chicken embryonic fibroblasts (CEF) were prepared from 9-11 day old embryonated eggs of specific-pathogen-free (SPF) Rhode Island Red (RIR) birds in E199 media (Sigma) with 5% serum. DF-1 cells were propagated in Dulbecco's modified Eagles medium (DMEM, Sigma) with 10% serum. DT40 cells were propagated in RPMI-1640 medium with 10% serum. All of the cell culture media were supplemented with 100 U/ml penicillin, 100 µg/ml streptomycin and 0.25 µg/ml fungizone.

For the preparation of recombinant virus stocks, CEF were transfected with the BAC DNA from the recombinant constructs using Lipofectamine^{®} transfection reagent (ThermoFisher) and reconstituted viruses were passaged to generate working virus stocks. Titration of SB-1 vaccine viruses was performed in CEF and the titres calculated by counting the plaque numbers four days post-infection. Recombinant virus plaques were confirmed using immunohistochemistry with IBDV VP2-specific mouse monoclonal antibody HH7 and SB-1-specific mouse monoclonal antibody Y5 (Lee LF, Liu X, Witter RL. Monoclonal antibodies with specificity for three different serotypes of Marek's disease viruses in chickens. J Immunol. 1983; 130: 1003-6) and goat anti-mouse HRP conjugated antibody (DAKO) and TrueBlue^{™} (KPL) peroxidase substrate.

Virulent IBDV UK661 strain for challenge was used as bursal tissue lysates from infected birds harvested at 3 days post infection (Eterradossi N et al., Zentralbl Veterinarmed B. 1992; 39: 683-91). In brief, IBDV-infected bursae were sampled from birds showing acute signs of the disease, and were homogenized in chlorotrifluoromethane (FREON 13). The supernatant collected after centrifugation at 1500g for 30 min at 4°C was filtered through a 0.45 um filter, and treated with penicillin and streptomycin. The lysates were titrated, aliquoted and kept at -80°C until use. It was then inoculated intranasally to 20 four week old spf white leghorn chicken (0.05 ml per bird). D78 strain was propagated in DF-1 cells and stored at -80°C until use. Titrations of UK661 and D78 virus strains were performed in DT40 and DF-1 cells respectively by calculating the median tissue culture infectious dose (TCID50) by the Spearman-Karber method (Brownie C et al., Biologicals. 2011;39:224-30).

### Virus growth curve studies

Confluent CEF in 10 cm² dishes were infected in triplicate with 10³ pfu of SB-1 viruses. Following the infection, infected CEF cells were harvested at time points 0, 6, 24, 48, 96 and 120 hours post infection. The harvested cells were washed with PBS and kept at -20°C until DNA extraction was performed. Genomic DNA was extracted from the infected cells using QIAamp 96 DNA kit (Qiagen). Quantitation of the copy numbers for the SB-1 genome was carried out using a real-time PCR (Singh SM et al., Res Vet Sci. 2010; 89: 140-5; Islam A et al., J Virol Methods. 2004; 119: 103-13; Renz KGet al., J Virol Methods. 2006; 135: 186-91) using the primers and probes as indicated in Table 3.

**Table 3: Real-time PCR primers**

| **Primers and probes for MDV-2 and Chicken ovotransferrin detection** | **Sequence** | **SEQ IS NO:** |
|---|---|---|
| MDV-2 forward primer | AGCATGCGGGAAGAAAAGAG | 25 |
| MDV-2 reverse primer | GAAAGGTTTTCCGCTCCCATA | 26 |
| MDV-2 probe | CGCCCGTAATGCACCCGTGACT | 27 |
| Ovo forward primer | CACTGCCACTGGGCTCTGT | 28 |
| Ovo reverse primer | GCAATGGCAATAAACCTCCAA | 29 |
| Ovo probe | AGTCTGGAGAAGTCTGTGCAGCAGCCTCCA | 30 |

### Serum neutralization test

Serum samples collected by centrifugation were heat treated at 56°C for 30 minutes to inactivate complement factors, prior to the neutralization test. Briefly, serial dilutions of sera samples were incubated with 100 TCID50 of D78 strain of IBDV for one hour at 37°C, and serum-virus mixtures were incubated on DF-1 cell monolayers in 96 well plates for one hour, before replacing with DMEM media containing 2% FCS. The cells were checked after four days for evidence of cytopathic effects (CPE) to determine the titres.

### Statistical analysis

For comparing the replication level of SB-1 recombinant viruses a linear model was employed. Log10 pfu was considered as the response variable and recombinant viruses in addition to hours post infection were considered as explanatory variables. Significant differences between time points and virus were identified using post-hoc Tukey tests. Differences in levels of neutralizing antibody during the course of study and between the groups were analysed using two-way ANOVA test. The level of antibodies within each group was analysed using one-way ANOVA test. The survival rate between groups of the birds after the challenge was compared using the Mantel-Cox test.

### Example 1

### Analysis of different loci to insert an expression cassette

The construction of a bacterial artificial chromosome (BAC) comprising the SB-1 genome has been reported by Petherbridge L et al. (J Virol Methods. 2009; 158: 11-7; Singh SM et al., Res Vet Sci. 2010; 89: 140-5). Using this pSB-1 BAC clone, we examine the potential of SB-1 as a novel recombinant vector for expressing protective antigens from other avian pathogens using the well-established recombineering techniques as described above. In order to identify locations in the SB-1 genome to insert expression cassettes coding for an antigen of an avian pathogen UL10/UL11, UL3/UL4, UL21/UL22, UL40/41, UL26/27 and UL50/UL51 intergenic regions were tested to insert galk bacterial expression cassette to produce the intermediate BAC constructs. Among the listed locations, UL26/27 and UL50/51 intergenic regions produced very little amount of virus (Table 4). Further, rSB-1 UL40/41 galK produced infectious virus (Table 4), but rSB-1 UL40/41 IBDV VP2 failed to produce infectious virus (data not shown), probably due to the larger insert. As a result, these locations were excluded from the study.

**Table 4. Comparison between different recombinants of rSB-1 virus. The recombinant viruses were made by inserting the indicated expression cassette in the specified locations.**

| **Name of the recombinant** | **Titre** |
|---|---|
| | **(PFU/ml)** |
| rSB-1 UL40/41 galK | 4×10⁴ |
| **rSB-1 UL21/22 galK** | **9.5×10⁴** |
| **rSB-1 UL3/4 galK** | **7×10⁴** |
| **rSB-1 UL10/11 galK** | **1.25×10⁴** |
| rSB-1 UL26/27 galK | 50 |
| rSB-1 UL50/51 galK | 1.95×10² |

Since integration sites UL40/41, UL26/27 and UK50/51 compromised replication we continued to further investigate insertion sites UL3/4, UL10/11 and UL21/22.

### Example 2

### Replication of rSB-1-UL3/4VP2, rSB-1-UL10/11VP2 and rSB-1-UL21/22VP2 viruses

In this study, we used the MDV-2 (GaHV3) strain SB-1 as a novel viral vector to generate three independent constructs that expressed IBDV VP2 in the intergenic loci of UL3/4, UL10/11 or UL21/22 loci of the viral genome.

Details of the construction of pSB-1-UL3/4VP2, pSB-1-UL10/11VP2 and pSB-1-UL21/22VP2 are summarised in Figure 1A. Recombinant SB-1 viruses produced after transfection of BAC DNA were passaged three times in CEF to produce low-passaged virus stocks. Following the rescue of the viruses, growth curve experiment was carried out to compare the replication of the recombinant and parental SB-1 viruses in CEF cells as shown in Figure 2. No significant differences were observed in the growth rate of pSB-1 and rSB-1-UL3/4VP2 viruses between 48 and 120 hours post infection. In contrast expression of the VP2 expression cassette from the UL10/11 locus and the UL21/22 locus appeared to slow the growth of SB-1 *in vitro.* Such a negative effect on replication (based on titer of the virus) was also observed when inserting the galK expression cassette for rSB-1-UL10/11galK virus, but not for rSB-1-UL21/22galK virus (see Table 4). No significant difference on replication between insertion into the intergenic locus UL3/4 and pSB-1 BAC clone was observed (Figure 2).

Therefore, it was concluded that the insertion of the VP2 expression cassette in the intragenic junction between UL3 and UL4 has the least effect on the growth rate of the virus *in vitro.* Expression of VP2 antigen in cells infected with rSB-1-UL3/4VP2, rSB-1-UL10/11VP2 and rSB-1-UL21/22VP2 viruses was further assessed by staining the infected cells with HH7 monoclonal and anti-mouse-HRP antibodies as shown in Figure 1B.

### Example 3

Having narrowed down the integration sites to UL3/4, UL10/11 and UL21/22 we further examined the immunogenic potential of the VP2 protein (Fahey KJ et al., J Gen Virol. 1989;70 ( Pt 6):1473-81) of infectious bursal disease virus (IBDV), the causative agent of infectious bursal disease (IBD, Gumboro disease) delivered by the recombinant SB-1 vector.

### Vaccine development against IBDV: immunization study

One-day-old SPF RIR chicks reared at the Experimental Animal House at Pirbright were used for the validation experiments. All procedures were performed in accordance with the UK Animal (Scientific Procedures) Act 1986 under Home Office Personal and Project licences, after the approval of the internal ethical review committee.

Forty 1-day old chicks were divided into 4 groups of 10 birds each. Each of the three groups received subcutaneous injections of rSB-1-UL10/11VP2, rSB-1-UL21/22VP2 or rSB-1-UL3/4VP2 vaccine viruses respectively, each comprising 3 × 10³ pfu in 100 µl inoculum. Each of the 10 birds in the control group were vaccinated with 3 × 10³ pfu of the VAXXITEK_{HVT+IBD}^{®} vaccine (Merial) as recommended by the manufacturer. Blood samples were collected weekly from the 2^{nd} to the 5^{th} week post-vaccination for serological studies.

Immunogenicity of the recombinant SB-1 vaccines was assessed by measuring the production of neutralizing antibodies in vaccinated chickens. The results of vaccinating subcutaneously with VAXXITEK_{HVT+IBD}, rSB-1-UL10/11VP2, rSB-1-UL21/22VP2 or rSB-1-UL3/4VP2 vaccine virus are described in Figure 3. Neutralizing antibodies started to appear from week two and rose to a maximum titre of 1:640 in week four post-vaccination. Neutralizing antibodies were detectable from week two onwards in all of the groups except for the group of birds vaccinated with rSB-1-UL21/22VP2, in which the level of neutralizing antibodies remained undetectable during week two. On week three post-vaccination all of the four groups showed neutralizing antibodies in the sera of about 50% of the birds. At week four post-vaccination, all of the birds showed neutralizing antibodies (Fig. 3). Although no significant differences between the mean levels of antibody between the groups were observed at each time point, the mean values of neutralizing antibodies in the groups inoculated with the experimental vaccines were higher than those of the group that received commercial vaccine.

### Example 4

For the challenge study vaccination was performed using rSB-1-UL3/4VP2 and rSB-1-UL21/22VP2. rSB-1-UL10/11VP2 was excluded from the study because of its lower growth level compared to SB-1-UL3/4VP2 and SB-1-UL21/22VP2.

### Vaccine development against IBDV: challenge study

One-day-old SPF RIR chicks reared at the Experimental Animal House at Pirbright were used for the validation experiments. All procedures were performed in accordance with the UK Animal (Scientific Procedures) Act 1986 under Home Office Personal and Project licences, after the approval of the internal ethical review committee.

Two groups (eight birds per group) of one-day old birds were inoculated subcutaneously with 1000 pfu of rSB-1-UL3/4VP2 or rSB-1-UL21/22VP2 virus stocks. Two control groups were inoculated with 1000 pfu of the pSB-1-derived virus or with 3000 pfu of the commercial VAXXITEK_{HVT+IBD}^{®} vaccine, respectively. After collecting the blood samples at four week post-vaccination, birds were challenged intranasally with 104.3 TCID50 of the virulent UK661 strain of IBDV (in a total volume of 100 µl divided between the two nostrils). In addition to the recording of the body weight, birds were monitored regularly and clinical signs scored at 6-hour intervals. Birds showing advanced clinical signs (exceeding a score of 9) were euthanized by cervical dislocation.

At week three post vaccination, the level of antibody in vaccinated birds was tested. One bird from the groups vaccinated with VAXXITEK_{HVT+IBD} and rSB-1-UL3/4VP2 and three birds from group vaccinated with rSB-1-UL21/22VP2 showed neutralizing antibodies in their sera. Compared to Example 3, the amount of SB-1 vaccines administered was reduced from 3000 pfu to 1000 pfu (compared to the 3000 pfu dose of VAXXITEK_{HVT+IBD} given in both experiments). Only a very limited number of birds showed neutralizing antibodies at week three post vaccination, presumably due to the smaller dose given. However, after experimental challenge with virulent virus, all of the groups (except for the SB-1 control group) showed 100% protection against IBDV. Thus our studies showed that the novel recombinant Gallid herpesvirus 2 vector vaccine induced similar levels of protection achieved from birds vaccinated with VAXXITEK_{HVT+IBD}. The mean clinical score and survival of the birds during challenge studies are shown in Figure 4. Clinical signs were only seen in the SB-1 vaccinated control group, in which they started appearing from 36 hours post challenge and increased sharply until 56 hours post challenge when the last remaining bird was euthanized. All of the birds in the negative control group were euthanized or died 56 hours post IBDV infection, whereas all of the birds vaccinated with VAXXITEK_{HVT+IBD}, rSB-1-UL3/4VP2 and rSB-1-UL21/22VP2 survived (p<0.0001).

The results show that GaHV3 can be used as a novel vector for immunization with IBDV VP2 in 1 day old chicks, resulting in complete protection against a challenge with a 100% lethal dose of IBDV. Thus, two locations in the GaHV3 genome have been identified that permit effective delivery of the VP2 cassette with no significant cost to replication of the vector: the intergenic UL3/4 locus and the intergenic UL21/22 locus. It is predicted that these loci will also support the delivery of genes from other pathogens. The results indicate that GaHV3, particularly strain SB-1, is suitable for use as a vector for IBDV vaccination of chickens, and that it can offer 100% protection at a commercially viable dose, thereby adding a new vector platform for developing recombinant vaccines against avian diseases.

### SEQUENCE TABLE:

SEQ ID NO: 1_intergenic locus UL3/4
SEQ ID NO: 2_integenic locus UL21/22
SEQ ID NO: 3_intergenic locus UL10/11
SEQ ID NO: 4_intergenic locus UL40/41
SEQ ID NO: 5_intergenic locus UL50/51
SEQ ID NO: 6_intergenic locus UL26/27
SEQ ID NO: 7_intergenic locus UL45/46
SEQ ID NO: 8_galK expression cassette
SEQ ID NO: 9_IBDV VP2 expression cassette nucleotide sequence
SEQ ID NO: 10_IBDV VP2 nucleotide sequence
SEQ ID NO: 11_murine cytomegalovirus (IE) promoter and enhancer sequence
SEQ ID NO: 12_IBDV VP2 protein sequence
SEQ ID NO: 13_UL21/22F-GalKF primer
SEQ ID NO: 14_UL21/22R-GalKR primer
SEQ ID NO: 15_UL26/27F-GalKF primer
SEQ ID NO: 16_UL26/27R-GalKR primer
SEQ ID NO: 17_UL3.5/4F-GalKF primer
SEQ ID NO: 18_UL3.5/4R-GalKR primer
SEQ ID NO: 19_UL10/11F-GalKF primer
SEQ ID NO: 20_UL10/11R-GalKR primer
SEQ ID NO: 21_UL40/41F-GalKF primer
SEQ ID NO: 22_UL40/41R-GalKR primer
SEQ ID NO: 23_UL50/51F-GalKF primer
SEQ ID NO: 24_UL50/51R-GalKR primer
SEQ ID NO: 25_MDV-2 forward primer
SEQ ID NO: 26_MDV-2 reverse primer
SEQ ID NO: 27_MDV-2 probe
SEQ ID NO: 28_ovo forward primer
SEQ ID NO: 29_ovo reverse primer
SEQ ID NO: 30_ovo probe
SEQ ID NO: 31_Gallid herpesvirus 1 glycoprotein E (gE)
SEQ ID NO: 32_Gallid herpesvirus 1 glycoprotein I (gl)
SEQ ID NO: 33_Gallid herpesvirus 1 glycoprotein B (gB)
SEQ ID NO: 34_Newcastle disease virus fusion protein
SEQ ID NO: 35_Newcastle disease virus hemagglutinin-neuraminidase (HN)
SEQ ID NO: 36_lnfluenza virus A serotype H5 hemagglutinin (H5 HA)
SEQ ID NO: 37_lnfluenza virus A serotype H7 hemagglutinin (H7 HA)
SEQ ID NO: 38_lnfluenza virus A serotype H9 hemagglutinin (H9 HA)
SEQ ID NO: 39_lnfluenza virus A serotype H5N1 neuraminidase (NA)
SEQ ID NO: 40_lnfectious bronchitis virus, spike glycoprotein

## Claims

1. A recombinant Gallid herpesvirus 3 (GaHV3; MDV-2) vector comprising one or more heterologous polynucleotide(s) coding for and expressing at least one antigen of an avian pathogen, inserted into the intergenic loci UL3/UL4 or UL3/UL4 and UL21/UL22 of the Gallid herpesvirus 3 vector.

2. The recombinant Gallid herpesvirus 3 vector of claim 1, wherein the recombinant Gallid herpesvirus 3 (GaHV3; MDV-2) vector is a Gallid herpesvirus 3 (GaHV3; MDV-2) strain SB-1 vector.

3. The recombinant Gallid herpesvirus 3 vector of any one of claims 1 or 2, wherein the at least one antigen is protective against infectious bursal disease virus (IBDV), infectious laryngotracheitis virus (ILTV), Newcastle disease virus (NDV), avian influenza virus (AIV) or avian infectious bronchitis virus (IBV).

4. The recombinant Gallid herpesvirus 3 vector of any one of claims 1 to 3, wherein the at least one antigen is preferably selected from the group consisting of
(a) VP2, VP3, VP4 and VPX of IBDV;
(b) glycoprotein B, glycoprotein I, glycoprotein D, glycoprotein E and glycoprotein C of ILTV;
(c) Newcastle disease virus fusion protein (NDV-F) and viral hemagglutinin neuraminidase (NDV-NH) of NDV
(d) Avian influenza hemagglutinin (HA) and neuraminidase (NA), and
(e) S1 and S2 protein of IBV.

5. A vaccine comprising the recombinant Gallid herpesvirus 3 vector of any one of the preceding claims, optionally comprising a pharmaceutically acceptable excipient, carrier or adjuvant.

6. The vaccine of claim 5 comprising a further Marek's disease virus (MDV) vector selected from the group consisting of Gallid herpesvirus 3 vector, naturally attenuated MDV-1 strain Rispens (CVI-988) vector and herpesvirus of turkeys (HTV) strain Fc126 vector.

7. The vaccine of claim 6 wherein the further MDV vector is a recombinant MDV vector and the recombinant Marek's disease virus (MDV) vector comprises one or more heterologous polynucleotide(s) coding for and expressing at least one antigen of an avian pathogen.

8. The vaccine of claim 7, wherein the recombinant Marek's disease virus vector is a second recombinant Gallid herpesvirus 3 vector, preferably a recombinant Gallid herpesvirus 3 strain SB-1 vector, in addition to the first recombinant Gallid herpesvirus 3 vector of any one of claims 1 to 4, and wherein
(a) the second recombinant Gallid herpesvirus 3 vector comprises the one or more heterologous polynucleotide(s) inserted into the intergenic loci UL3/UL4 or UL3/UL4 and UL21/UL22 of said second Gallid herpesvirus 3 vector; and
(b) the second recombinant Gallid herpesvirus 3 vector comprises at least one heterologous polynucleotide coding for and expressing a different antigen of an avian pathogen as the one or more heterologous polynucleotides of the first recombinant Gallid herpesvirus 3 vector.

9. A bacterial artificial chromosome (BAC) comprising a polynucleotide coding for the recombinant Gallid herpesvirus 3 vector of any one of claims 1 to 4.

10. The vaccine of any one of claims 5 to 8 for use in vaccinating an avian species against Marek's disease and one or more diseases caused by one or more avian pathogens.

11. The vaccine of any one of claims 5 to 8 for use in protecting an avian species against clinical symptoms caused by Marek's disease virus and clinical symptoms caused by one or more avian pathogens.

12. The vaccine for use as in claim 10 or 11 wherein
(a) the one or more diseases or clinical symptoms are caused by one or more of infectious bursal disease virus (IBDV), infectious laryngotracheitis virus (ILTV), Newcastle disease virus (NDV), avian influenza virus (AIV) or avian infectious bronchitis virus (IBV), and/or
(b) the avian species is poultry, preferably chicken, duck, goose, turkey, quail, guinea or pigeon, more preferably turkey or chicken.

13. A recombinant Gallid herpesvirus 3 (GaHV3; MDV-2) vector comprising one or more marker(s) inserted into the intergenic loci UL3/UL4 or UL3/UL4 and UL21/UL22 of the Gallid herpesvirus 3 vector, wherein the marker is preferably a selection marker gene, a reporter gene or a DNA bar code.

14. An *in vitro* method of producing a recombinant Gallid herpesvirus 3 vector comprising
(a) providing a Gallid herpesvirus 3 vector,
(b) inserting one or more heterologous polynucleotide(s) coding for and expressing at least one antigen of an avian pathogen into the intergenic loci UL3/UL4 or UL3/UL4 and UL21/UL22 of the Gallid herpesvirus 3 vector, and optionally
(c) amplifying the Gallid herpesvirus 3 vector comprising one or more heterologous polynucleotide(s) coding for at least one antigen of an avian pathogen of step (b).

15. An *in vitro* method of producing a recombinant Gallid herpesvirus 3 vector comprising
(a) providing a Gallid herpesvirus 3 vector comprising one or more marker(s) in the intergenic loci UL3/UL4 or UL3/UL4 and UL21/UL22 of the Gallid herpesvirus 3 vector,
(b) replacing the one or more marker(s) with an expression cassette comprising one or more heterologous polynucleotides coding for at least one antigen of an avian pathogen, and
(c) amplifying the Gallid herpesvirus 3 vector comprising one or more heterologous polynucleotide(s) coding for at least one antigen of an avian pathogen of step (b).

16. The *in vitro* method of claim 14 or 15, wherein step (a) comprises providing a bacterial artificial chromosome comprising a polynucleotide coding for the recombinant Gallid herpesvirus 3 vector and wherein the method optionally further comprises the steps of
(d) isolating the bacterial artificial chromosome comprising a polynucleotide coding for the recombinant Gallid herpesvirus 3 vector comprising one or more heterologous polynucleotide(s) coding for at least one antigen of an avian pathogen of step (b)
(e) transfecting chicken embryonic fibroblasts with the bacterial artificial chromosome of step (d).

## Patentansprüche

1. Rekombinanter Gallid-Herpesvirus-3 (GaHV3; MDV-2) Vektor, umfassend ein oder mehrere heterologe(s) Polynukleotid(e), das/die für mindestens ein Antigen eines Vogelpathogens kodieren und dieses exprimieren, eingefügt in die intergenen Loci UL3/UL4 oder UL3/UL4 und UL21/UL22 des Gallid-Herpesvirus-3 Vektors.

2. Der rekombinante Gallid-Herpesvirus-3 Vektor nach Anspruch 1, wobei der rekombinante Gallid-Herpesvirus-3 (GaHV3; MDV-2) Vektor ein Gallid-Herpesvirus-3 (GaHV3; MDV-2) Stamm SB-1 Vektor ist.

3. Der rekombinante Gallid-Herpesvirus-3 Vektor nach einem der Ansprüche 1 oder 2, wobei das mindestens eine Antigen gegen das Virus der infektiösen Bursitis (IBDV), das Virus der infektiöse Laryngotracheitis (ILTV), das Virus der Newcastle-Krankheit (NDV), das aviäre Influenzavirus (AIV) oder das aviäre infektiöse Bronchitis Virus (IBV) schützt.

4. Der rekombinante Gallid-Herpesvirus-3 Vektor nach einem der Ansprüche 1 bis 3, wobei das mindestens eine Antigen vorzugsweise ausgewählt ist aus der Gruppe bestehend aus
(a) VP2, VP3, VP4 und VPX von IBDV;
(b) Glykoprotein B, Glykoprotein I, Glykoprotein D, Glykoprotein E und Glykoprotein C von ILTV;
(c) Newcastle-Krankheit Virus-Fusionsprotein (NDV-F) und virale Hämagglutinin-Neuraminidase (NDV-NH) von NDV
(d) Hämagglutinin (HA) und Neuraminidase (NA) der aviären Influenza, und
(e) S1- und S2-Protein von IBV.

5. Vakzine umfassed den rekombinanten Gallid-Herpesvirus-3 Vektor nach einem der vorhergehenden Ansprüche, der gegebenenfalls einen pharmazeutisch annehmbaren Hilfsstoff, Träger oder ein Adjuvans enthält.

6. Die Vakzine nach Anspruch 5, umfassend einen weiteren Marek-Krankheit Virus (MDV) Vektor, der ausgewählt ist aus der Gruppe bestehend aus Gallid-Herpesvirus-3 Vektor, natürlich abgeschwächtem MDV-1-Stamm Rispens (CVI-988) Vektor und Truthahn-Herpesvirus (HTV)-Stamm Fc126 Vektor.

7. Die Vakzine nach Anspruch 6, wobei der weitere MDV Vektor ein rekombinanter MDV Vektor ist und der rekombinante Marek-Krankheit Virus (MDV) Vektor ein oder mehrere heterologe(s) Polynukleotid(e) umfasst, das/die für mindestens ein Antigen eines Vogelpathogens kodieren und dies exprimieren.

8. Die Vakzine nach Anspruch 7, wobei der rekombinante Marek Virus Vektor ein zweiter rekombinanter Gallid-Herpesvirus-3 Vektor, vorzugsweise ein rekombinanter Gallid-Herpesvirus-3 Stamm-SB-1 Vektor, zusätzlich zu dem ersten rekombinanten Gallid-Herpesvirus-3 Vektor nach einem der Ansprüche 1 bis 4 ist, und wobei
(a) der zweite rekombinante Gallid-Herpesvirus-3 Vektor das/die eine oder mehreren heterologe(n) Polynukleotid(e), eingefügt in die intergenen Loci UL3/UL4 oder UL3/UL4 und UL21/UL22 des zweiten Gallid-Herpesvirus-3 Vektors umfasst; und
(b) der zweite rekombinante Gallid-Herpesvirus-3 Vektor mindestens ein heterologes Polynukleotid umfasst, das für ein anderes Antigen eines Vogelpathogens kodiert und dieses exprimiert als das/die eine oder mehreren heterologe(n) Polynukleotid(e) des ersten rekombinanten Gallid-Herpesvirus-3 Vektors.

9. Bakterielles künstliches Chromosom (BAC), umfassend ein Polynukleotid, das für den rekombinanten Gallid-Herpesvirus-3 Vektor nach einem der Ansprüche 1 bis 4 kodiert.

10. Die Vakzine nach einem der Ansprüche 5 bis 8 zur Verwendung in der Vakzinierung einer Vogelart gegen die Marek-Krankheit und eine oder mehrere Krankheit(en), die von einem oder mehreren Vogelpathogen(en) verursacht wird/werden.

11. Die Vakzine nach einem der Ansprüche 5 bis 8 zur Verwendung beim Schutz einer Vogelart gegen klinische Symptome verursacht durch das Virus der Marek-Krankheit, und gegen klinische Symptome verursacht durch ein oder mehrere Vogelpathogen(e).

12. Die Vakzine zur Anwendung nach Anspruch 10 oder 11, wobei
(a) die eine oder mehreren Krankheit(en) oder klinischen Symptome verursacht werden durch ein oder mehrere vom Virus der infektiösen Bursitis (IBDV), Virus der infektiöse Laryngotracheitis (ILTV), Virus der Newcastle-Krankheit (NDV), aviäres Influenzavirus (AIV) oder aviäres infektiöses Bronchitis Virus (IBV), und/oder
(b) die Vogelart Geflügel ist, vorzugsweise Huhn, Ente, Gans, Truthahn, Wachtel, Perlhuhn oder Taube, besonders bevorzugt Truthahn oder Huhn.

13. Der rekombinanter Gallid-Herpesvirus-3 (GaHV3; MDV-2) Vektor, umfassend einen oder mehrere Marker eingefügt in die intergenen Loci UL3/UL4 oder UL3/UL4 und UL21/UL22 des Gallid-Herpesvirus-3 Vektors, wobei der/die Marker vorzugsweise ein Selektionsmarker-Gen, ein Reporter-Gen oder ein DNA-Strichcode ist/sind.

14. Ein *in vitro* Verfahren zur Herstellung eines rekombinanten Gallid-Herpesvirus-3 Vektors, umfassend
(a) Bereitstellen eines Gallid-Herpesvirus-3 Vektors,
(b) Einfügen eines oder mehrerer heterologer Polynukleotide kodierend und exprimierend für mindestens ein Antigen eines Vogelpathogens in die intergenen Loci UL3/UL4 oder UL3/UL4 und UL21/UL22 des Gallid-Herpesvirus 3 Vektors, und gegebenenfalls
(c) Amplifizieren des Gallid-Herpesvirus-3 Vektors umfassend ein oder mehrere heterologe Polynukleotide kodierend für mindestens ein Antigen eines Vogelpathogens aus Schritt (b).

15. Ein *in-vitro* Verfahren zur Herstellung eines rekombinanten Gallid-Herpesvirus-3 Vektors, umfassend
(a) Bereitstellen eines Gallid-Herpesvirus-3 Vektors, der einen oder mehrere Marker in den intergenen Loci UL3/UL4 oder UL3/UL4 und UL21/UL22 des Gallid-Herpesvirus-3 Vektors umfasst,
(b) Ersetzen der einen oder der mehreren Marker durch eine Expressionskassette, die ein oder mehrere heterologe(s) Polynukleotid(e) umfasst kodierend für mindestens ein Antigen eines Vogelpathogens, und
(c) Amplifizieren des Gallid-Herpesvirus-3 Vektors umfassend ein oder mehrere heterologe Polynukleotide) kodierend für mindestens ein Antigen eines Vogelpathogens aus Schritt (b).

16. Das *in-vitro* Verfahren nach Anspruch 14 oder 15, wobei Schritt (a) das Bereitstellen eines bakteriellen künstlichen Chromosoms umfasst, das ein Polynukleotid umfasst, das für den rekombinanten Gallid-Herpesvirus-3 Vektor kodiert, und wobei das Verfahren gegebenenfalls zusätzlich die Schritte umfasst
(d) Isolieren des bakteriellen künstlichen Chromosoms umfassend ein Polynukleotid kodierend für den rekombinanten Gallid-Herpesvirus-3 Vektor umfassend ein oder mehrere heterologe Polynukleotide kodierend für mindestens ein Antigen eines Vogelpathogens aus Schritt (b),
(e) Transfektion embryonale Hühnerfibroblasten mit dem bakteriellen künstlichen Chromosom aus Schritt (d).

## Revendications

1. Vecteur recombiné de l'herpèsvirus aviaire de type 3 (GaHV3 ; MDV-2) comprenant un ou plusieurs polynucléotides hétérologues codant pour et exprimant au moins un antigène d'un agent pathogène aviaire, insérés dans les loci intergéniques UL3/UL4 ou UL3/UL4 et UL21/UL22 du vecteur de l'herpèsvirus aviaire de type 3.

2. Vecteur recombiné de l'herpèsvirus aviaire de type 3 selon la revendication 1, dans lequel le vecteur recombiné de l'herpèsvirus aviaire de type 3 (GaHV3 ; MDV-2) est un vecteur de la souche SB-1 de l'herpèsvirus aviaire de type 3 (GaHV3 ; MDV-2).

3. Vecteur recombiné de l'herpèsvirus aviaire de type 3 selon l'une quelconque des revendications 1 ou 2, dans lequel l'au moins un antigène est protecteur contre le virus de la bursite infectieuse (IBDV), le virus de la laryngotrachéite infectieuse (ILTV), le virus de la maladie de Newcastle (NDV), le virus de la grippe aviaire (AIV) ou le virus de la bronchite infectieuse aviaire (IBV).

4. Vecteur recombiné de l'herpèsvirus aviaire de type 3 selon l'une quelconque des revendications 1 à 3, dans lequel l'au moins un antigène est choisi préférentiellement parmi le groupe constitué de
(a) VP2, VP3, VP4 et VPX de l'IBDV ;
(b) glycoprotéine B, glycoprotéine I, glycoprotéine D, glycoprotéine E et glycoprotéine C de l'ILTV ;
(c) protéine de fusion du virus de la maladie de Newcastle (NDV-F) et hémagglutinine-neuraminidase du NDV (NDV-NH)
(d) hémagglutinine (HA) et neuraminidase (NA) de la grippe aviaire, et
(e) protéine S1 et S2 de l'IBV.

5. Vaccin comprenant le vecteur recombiné de l'herpèsvirus aviaire de type 3 selon l'une quelconque des revendications précédentes, comprenant éventuellement un excipient, véhicule ou adjuvant pharmaceutiquement acceptable.

6. Vaccin selon la revendication 5 comprenant un autre vecteur de virus de la maladie de Marek (MDV) choisi parmi le groupe constitué du vecteur de l'herpèsvirus aviaire de type 3, du vecteur de la souche Rispens (CVI-988) du MDV-1 naturellement atténuée et du vecteur de la souche Fc126 d'un herpèsvirus de la dinde (HTV).

7. Vaccin selon la revendication 6 dans lequel l'autre vecteur de MDV est un vecteur recombiné de MDV et le vecteur recombiné de virus de la maladie de Marek (MDV) comprend un ou plusieurs polynucléotides hétérologues codant pour et exprimant au moins un antigène d'un agent pathogène aviaire.

8. Vaccin selon la revendication 7, dans lequel le vecteur recombiné de virus de la maladie de Marek est un deuxième vecteur recombiné de l'herpèsvirus aviaire de type 3, préférentiellement un vecteur recombiné de la souche SB-1 de l'herpèsvirus aviaire de type 3, en plus du premier vecteur recombiné de l'herpèsvirus aviaire de type 3 selon l'une quelconque des revendications 1 à 4, et dans lequel
(a) le deuxième vecteur recombiné de l'herpèsvirus aviaire de type 3 comprend les un ou plusieurs polynucléotides hétérologues insérés dans les loci intergéniques UL3/UL4 ou UL3/UL4 et UL21/UL22 dudit deuxième vecteur recombiné de l'herpèsvirus aviaire de type 3 ; et
(b) le deuxième vecteur recombiné de l'herpèsvirus aviaire de type 3 comprend au moins un polynucléotide hétérologue codant pour et exprimant un antigène d'un agent pathogène aviaire différent de ceux des un ou plusieurs polynucléotides hétérologues du premier vecteur recombiné de l'herpèsvirus aviaire de type 3.

9. Chromosome bactérien artificiel (BAC) comprenant un polynucléotide codant pour le vecteur recombiné de l'herpèsvirus aviaire de type 3 selon l'une quelconque des revendications 1 à 4.

10. Vaccin selon l'une quelconque des revendications 5 à 8 pour son utilisation dans la vaccination d'une espèce aviaire contre la maladie de Marek et contre une ou plusieurs maladies provoquées par un ou plusieurs agents pathogènes aviaires.

11. Vaccin selon l'une quelconque des revendications 5 à 8 pour son utilisation dans la protection d'une espèce aviaire contre les symptômes cliniques provoqués par le virus de la maladie de Marek et contre les symptômes cliniques provoqués par un ou plusieurs agents pathogènes aviaires.

12. Vaccin pour son utilisation selon la revendication 10 ou 11, dans lequel
(a) les une ou plusieurs maladies ou les un ou plusieurs symptômes cliniques sont provoqués par un ou plusieurs virus parmi le virus de la bursite infectieuse (IBDV), le virus de la laryngotrachéite infectieuse (ILTV), le virus de la maladie de Newcastle (NDV), le virus de la grippe aviaire (AIV) ou le virus de la bronchite infectieuse aviaire (IBV), et/ou
(b) l'espèce aviaire est de la volaille, préférentiellement poulet, canard, oie, dinde, caille, pintade ou pigeon, plus préférentiellement dinde ou poulet.

13. Vecteur recombiné de l'herpèsvirus aviaire de type 3 (GaHV3 ; MDV-2) comprenant un ou plusieurs marqueurs insérés dans les loci intergéniques UL3/UL4 ou UL3/UL4 et UL21/UL22 du vecteur de l'herpèsvirus aviaire de type 3, dans lequel le marqueur est préférentiellement un gène marqueur de sélection, un gène rapporteur ou un code- barres génétique.

14. Procédé *in vitro* pour la production d'un vecteur recombiné de l'herpèsvirus aviaire de type 3 comprenant
(a) la fourniture d'un vecteur de l'herpèsvirus aviaire de type 3,
(b) l'insertion d'un ou plusieurs polynucléotides hétérologues codant pour et exprimant au moins un antigène d'un agent pathogène aviaire dans les loci intergéniques UL3/UL4 ou UL3/UL4 et UL21/UL22 du vecteur de l'herpèsvirus aviaire de type 3, et éventuellement
(c) l'amplification du vecteur de l'herpèsvirus aviaire de type 3 comprenant un ou plusieurs polynucléotides hétérologues codant pour au moins un antigène d'un agent pathogène aviaire de l'étape (b).

15. Procédé *in vitro* pour la production d'un vecteur recombiné de l'herpèsvirus aviaire de type 3 comprenant
(a) la fourniture d'un vecteur de l'herpèsvirus aviaire de type 3 comprenant un ou plusieurs marqueurs dans les loci intergéniques UL3/UL4 ou UL3/UL4 et UL21/UL22 du vecteur de l'herpèsvirus aviaire de type 3,
(b) le remplacement des un ou plusieurs marqueurs avec une cassette d'expression comprenant un ou plusieurs polynucléotides hétérologues codant pour au moins un antigène d'un agent pathogène aviaire, et
(c) l'amplification du vecteur de l'herpèsvirus aviaire de type 3 comprenant un ou plusieurs polynucléotides hétérologues codant pour au moins un antigène d'un agent pathogène aviaire de l'étape (b).

16. Procédé in vitro selon la revendication 14 ou 15, dans lequel l'étape (a) comprend la fourniture d'un chromosome bactérien artificiel comprenant un polynucléotide codant pour le vecteur recombiné de l'herpèsvirus aviaire de type 3 et dans lequel le procédé comprend en outre éventuellement les étapes suivantes
(d) l'isolement du chromosome bactérien artificiel comprenant un polynucléotide codant pour le vecteur recombiné de l'herpèsvirus aviaire de type 3 comprenant un ou plusieurs polynucléotides hétérologues codant pour au moins un antigène d'un agent pathogène aviaire de l'étape (b)
(e) la transfection de fibroblastes embryonnaires de poulet avec le chromosome bactérien artificiel de l'étape (d).


### SEQUENCE LISTING

<110> Pirbright Institute
<120> Recombinant Gallid herpesvirus 3 vaccines encoding heterologous avian
   pathogen antigens
<130> 113566P1144EP
<160> 40
<170> BiSSAP 1.3.6
<210> 1
   <211> 96
   <212> DNA
   <213> Gallid herpesvirus 3
<220>
   <223> intergenic locus UL3/4
<400> 1
   caagaagcat ctaaaacgcg cttgattgtc gagtggctga ataaaatctt tattgatcga 60
   ctcgctttcc tatttctgat ttaataacca tagatg 96
<210> 2
   <211> 100
   <212> DNA
   <213> Gallid herpesvirus 3
<220>
   <223> intergenic locus UL21/22
<400> 2
   gccgggcata tacagaacgt aagccaagct ggagtttgtg taagtatgtg ctctacagcg 60
   tgcgggaagg gcggttcgcg aataaacaca acagtacagg 100
<210> 3
   <211> 100
   <212> DNA
   <213> Gallid herpesvirus 3
<220>
   <223> intergenic locus UL10/11
<400> 3
   agtgaatggg atgaatagga acgcccgaaa cataataaaa cgctaaatct acaagtgatt 60
   gtcgcgcgac ttatttatca ctataacgta tcgttacatt 100
<210> 4
   <211> 100
   <212> DNA
   <213> Gallid herpesvirus 3
<220>
   <223> intergenic locus UL40/41
<400> 4
   gtatcgaacg atctttaatt agcctgtgtg caactgtact ttctacccct acccacaaga     60
   attaataaat gaattcaaat tatcgctttt cgcaccgtgt                          100
<210> 5
   <211> 101
   <212> DNA
   <213> Gallid herpesvirus 3
<220>
   <223> intergenic locus UL50/51
<400> 5
   aaacggcagc attcgatacg gaatcgggca ggagcgagag agagtgtgcg tatgtaatcg     60
   tgcgcaacta tacattattg cccgctcgac ccgaagccgt c                        101
<210> 6
   <211> 100
   <212> DNA
   <213> Gallid herpesvirus 3
<220>
   <223> intergenic locus UL26/27
<400> 6
   ggatccgcta tgtcgacgta taagtttata cattttgcga ccgcaatagc aaataaaagt     60
   aaaataatcg tatgcgcacg tgagaattta tttatcgaga                          100
<210> 7
   <211> 100
   <212> DNA
   <213> Gallid herpesvirus 3
<220>
   <223> intergenic locus UL45/46
<400> 7
   gagagaccga gcattagagt agcacttatt tattctatcg cagagaaaca gcgcgcgttc     60
   aaaaaaaaca caggcggggt acgataaatt tacgcggccg                          100
<210> 8
   <211> 1236
   <212> DNA
   <213> Artificial Sequence
<220>
   <223> galK expression cassette
<400> 8
   gaattcctgt tgacaattaa tcatcggcat agtatatcgg catagtataa tacgacaagg      60
   tgaggaacta aacccaggag gcagatcatg agtctgaaag aaaaaacaca atctctgttt     120
   gccaacgcat ttggctaccc tgccactcac accattcagg cgcctggccg cgtgaatttg     180
   attggtgaac acaccgacta caacgacggt ttcgttctgc cctgcgcgat tgattatcaa     240
   accgtgatca gttgtgcacc acgcgatgac cgtaaagttc gcgtgatggc agccgattat     300
   gaaaatcagc tcgacgagtt ttccctcgat gcgcccattg tcgcacatga aaactatcaa     360
   tgggctaact acgttcgtgg cgtggtgaaa catctgcaac tgcgtaacaa cagcttcggc     420
   ggcgtggaca tggtgatcag cggcaatgtg ccgcagggtg ccgggttaag ttcttccgct     480
   tcactggaag tcgcggtcgg aaccgtattg cagcagcttt atcatctgcc gctggacggc     540
   gcacaaatcg cgcttaacgg tcaggaagca gaaaaccagt ttgtaggctg taactgcggg     600
   atcatggatc agctaatttc cgcgctcggc aagaaagatc atgccttgct gatcgattgc     660
   cgctcactgg ggaccaaagc agtttccatg cccaaaggtg tggctgtcgt catcatcaac     720
   agtaacttca aacgtaccct ggttggcagc gaatacaaca cccgtcgtga acagtgcgaa     780
   accggtgcgc gtttcttcca gcagccagcc ctgcgtgatg tcaccattga agagttcaac     840
   gctgttgcgc atgaactgga cccgatcgtg gcaaaacgcg tgcgtcatat actgactgaa     900
   aacgcccgca ccgttgaagc tgccagcgcg ctggagcaag gcgacctgaa acgtatgggc     960
   gagttgatgg cggagtctca tgcctctatg cgcgatgatt tcgaaatcac cgtgccgcaa    1020
   attgacactc tggtagaaat cgtcaaagct gtgattggcg acaaaggtgg cgtacgcatg    1080
   accggcggcg gatttggcgg ctgtatcgtc gcgctgatcc cggaagagct ggtgcctgcc    1140
   gtacagcaag ctgtcgctga acaatatgaa gcaaaaacag gtattaaaga gactttttac    1200
   gtttgtaaac catcacaagg agcaggacag tgctga                               1236
<210> 9
   <211> 3031
   <212> DNA
   <213> Artificial Sequence
<220>
   <223> IBDV VP2 expression cassette
<400> 9
   ggatccccca actccgcccg ttttatgact agaaccaata gtttttaatg ccaaatgcac 60
   tgaaatcccc taatttgcaa agccaaacgc cccctatgtg agtaatacgg ggacttttta 120
   cccaatttcc caagcggaaa gccccctaat acactcatat ggcatatgaa tcagcacggt 180
   catgcactct aatggcggcc catagggact ttccacatag ggggcgttca ccatttccca 240
   gcataggggt ggtgactcaa tggcctttac ccaagtacat tgggtcaatg ggaggtaagc      300
   caatgggttt ttcccattac tggcaagcac actgagtcaa atgggacttt ccactgggtt      360
   ttgcccaagt acattgggtc aatgggaggt gagccaatgg gaaaaaccca ttgctgccaa      420
   gtacactgac tcaataggga ctttccaatg ggtttttcca ttgttggcaa gcatataagg      480
   tcaatgtggg tgagtcaata gggactttcc attgtattct gcccagtaca taaggtcaat      540
   agggggtgaa tcaacaggaa agtcccattg gagccaagta cactgcgtca atagggactt      600
   tccattgggt tttgcccagt acataaggtc aataggggat gagtcaatgg gaaaaaccca      660
   ttggagccaa gtacactgac tcaataggga ctttccattg ggttttgccc agtacataag      720
   gtcaataggg ggtgagtcaa caggaaagtt ccattggagc caagtacatt gagtcaatag      780
   ggactttcca atgggttttg cccagtacat aaggtcaatg ggaggtaagc caatgggttt      840
   ttcccattac tggcacgtat actgagtcat tagggacttt ccaatgggtt ttgcccagta      900
   cataaggtca ataggggtga atcaacagga aagtcccatt ggagccaagt acactgagtc      960
   aatagggact ttccattggg ttttgcccag tacaaaaggt caataggggg tgagtcaatg     1020
   ggtttttccc attattggca cgtacataag gtcaataggg gtgagtcatt gggtttttcc     1080
   agccaattta attaaaacgc catgtacttt cccaccattg acgtcaatgg gctattgaaa     1140
   ctaatgcaac gtgaccttta aacggtactt tcccatagct gattaatggg aaagtaccgt     1200
   tctcgagcca atacacgtca atgggaagtg aaagggcagc caaaacgtaa caccgccccg     1260
   gttttcccct ggaaattcca tattggcacg cattctattg gctgagctgc gttctacgtg     1320
   ggtataagag gcgcgaccag cgtcggtacc gtcgcagtct tcggtctgac caccgtagaa     1380
   cgcagagctc ctcgctgcag gcggccgctc tagaactcgt cgatcgcagc gatgataaac     1440
   ctgcaagatc aaacccaaca gattgttccg ttcatacgga gccttctgat gccaacaacc     1500
   ggaccggcgt ccattccgga cgacaccctg gagaagcaca ctctcaggtc agagacctcg     1560
   acctacaatt tgactgtggg ggacacaggg tcagggctaa ttgtcttttt ccctggattc     1620
   cctggctcaa ttgtgggtgc tcactacaca ctgcagagca atgggaacta caagttcgat     1680
   cagatgctcc tgactgccca gaacctaccg gccagctaca actactgcag actagtgagt     1740
   cggagtctca cagtgaggtc aagcacactc cctggtggcg tttatgcact aaacggcacc     1800
   ataaacgccg tgaccttcca aggaagcctg agtgaactga cagatgttag ctacaatggg     1860
   ttgatgtctg caacagccaa catcaacgac aaaattggga atgtcctggt aggggaaggg     1920
   gtcactgtcc tcagcctacc cacatcatat gatcttgggt atgtgaggct tggtgacccc     1980
   attcccgcta tagggcttga cccaaaaatg gtagctacat gcgacagcag tgacaggccc     2040
   agagtctaca ccataactgc agccgatgat taccaattct catcacagta ccaaccaggt     2100
   ggggtaacaa tcacactgtt ctcagccaac attgatgcta tcacaagcct cagcattggg     2160
   ggagagctcg tgtttcaaac aagcgtccaa ggccttgtac tgggcgccac catctacctt 2220
   ataggctttg atgggactgc ggtaatcacc agagctgtag ccgcagataa tgggctgacg 2280
   gccggcaccg acaatcttat gccattcaat cttgtcattc caaccaatga gataacccag 2340
   ccaatcacat ccatcaaact ggagatagtg acctccaaaa gtggtggtca ggcaggggat 2400
   cagatgtcat ggtcggcaag tgggagccta gcagtgacga tccatggtgg caactatcca 2460
   ggggccctcc gtcccgtcac actagtagcc tacgaaagag tggcaacagg atccgtcgtt 2520
   acggtcgctg gggtgagtaa cttcgagctg attccaaatc ctgaactagc aaagaacctg 2580
   gttacagaat acggccgatt tgacccagga gccatgaact acacaaaatt gatactgagt 2640
   gagagggacc gtcttggcat caagaccgtc tggccaacaa gggagtacac tgattttcgt 2700
   gagtacttca tggaggtggc cgacctcaac tctcccctga agattgcagg agcatttggc 2760
   ttcaaagaca taatccgggc tataaggagg taagcttgat ctagagcggc cgcggggatc 2820
   cagacatgat aagatacatt gatgagtttg gacaaaccac aactagaatg cagtgaaaaa 2880
   aatgctttat ttgtgaaatt tgtgatgcta ttgctttatt tgtaaccatt ataagctgca 2940
   ataaacaagt taacaacaac aattgcattc attttatgtt tcaggttcag ggggaggtgt 3000
   gggaggtttt ttcggatcct ctagagtcga c 3031
<210> 10
   <211> 1362
   <212> DNA
   <213> Infectious bursal disease virus 52/70
<220>
   <223> IBDV VP2 nucleotide sequence
<400> 10
   atgataaacc tgcaagatca aacccaacag attgttccgt tcatacggag ccttctgatg      60
   ccaacaaccg gaccggcgtc cattccggac gacaccctgg agaagcacac tctcaggtca     120
   gagacctcga cctacaattt gactgtgggg gacacagggt cagggctaat tgtctttttc     180
   cctggattcc ctggctcaat tgtgggtgct cactacacac tgcagagcaa tgggaactac     240
   aagttcgatc agatgctcct gactgcccag aacctaccgg ccagctacaa ctactgcaga     300
   ctagtgagtc ggagtctcac agtgaggtca agcacactcc ctggtggcgt ttatgcacta     360
   aacggcacca taaacgccgt gaccttccaa ggaagcctga gtgaactgac agatgttagc     420
   tacaatgggt tgatgtctgc aacagccaac atcaacgaca aaattgggaa tgtcctggta     480
   ggggaagggg tcactgtcct cagcctaccc acatcatatg atcttgggta tgtgaggctt     540
   ggtgacccca ttcccgctat agggcttgac ccaaaaatgg tagctacatg cgacagcagt     600
   gacaggccca gagtctacac cataactgca gccgatgatt accaattctc atcacagtac     660
   caaccaggtg gggtaacaat cacactgttc tcagccaaca ttgatgctat cacaagcctc      720
   agcattgggg gagagctcgt gtttcaaaca agcgtccaag gccttgtact gggcgccacc     780
   atctacctta taggctttga tgggactgcg gtaatcacca gagctgtagc cgcagataat     840
   gggctgacgg ccggcaccga caatcttatg ccattcaatc ttgtcattcc aaccaatgag     900
   ataacccagc caatcacatc catcaaactg gagatagtga cctccaaaag tggtggtcag     960
   gcaggggatc agatgtcatg gtcggcaagt gggagcctag cagtgacgat ccatggtggc    1020
   aactatccag gggccctccg tcccgtcaca ctagtagcct acgaaagagt ggcaacagga    1080
   tccgtcgtta cggtcgctgg ggtgagtaac ttcgagctga ttccaaatcc tgaactagca    1140
   aagaacctgg ttacagaata cggccgattt gacccaggag ccatgaacta cacaaaattg    1200
   atactgagtg agagggaccg tcttggcatc aagaccgtct ggccaacaag ggagtacact    1260
   gattttcgtg agtacttcat ggaggtggcc gacctcaact ctcccctgaa gattgcagga    1320
   gcatttggct tcaaagacat aatccgggct ataaggaggt aa                       1362
<210> 11
   <211> 1206
   <212> DNA
   <213> Artificial Sequence
<220>
   <223> murine CMV IE promoter and enhancer sequence
<400> 11
   gttcaccatt tcccagcata ggggtggtga ctcaatggcc tttacccaag tacattgggt      60
   caatgggagg taagccaatg ggtttttccc attactggca agcacactga gtcaaatggg     120
   actttccact gggttttgcc caagtacatt gggtcaatgg gaggtgagcc aatgggaaaa     180
   acccattgct gccaagtaca ctgactcaat agggactttc caatgggttt ttccattgtt     240
   ggcaagcata taaggtcaat gtgggtgagt caatagggac tttccattgt attctgccca     300
   gtacataagg tcaatagggg gtgaatcaac aggaaagtcc cattggagcc aagtacactg     360
   cgtcaatagg gactttccat tgggttttgc ccagtacata aggtcaatag gggatgagtc     420
   aatgggaaaa acccattgga gccaagtaca ctgactcaat agggactttc cattgggttt     480
   tgcccagtac ataaggtcaa tagggggtga gtcaacagga aagttccatt ggagccaagt      540
   acattgagtc aatagggact ttccaatggg ttttgcccag tacataaggt caatgggagg     600
   taagccaatg ggtttttccc attactggca cgtatactga gtcattaggg actttccaat     660
   gggttttgcc cagtacataa ggtcaatagg ggtgaatcaa caggaaagtc ccattggagc     720
   caagtacact gagtcaatag ggactttcca ttgggttttg cccagtacaa aaggtcaata     780
   gggggtgagt caatgggttt ttcccattat tggcacgtac ataaggtcaa taggggtgag     840
   tcattgggtt tttccagcca atttaattaa aacgccatgt actttcccac cattgacgtc     900
   aatgggctat tgaaactaat gcaacgtgac ctttaaacgg tactttccca tagctgatta     960
   atgggaaagt accgttctcg agccaataca cgtcaatggg aagtgaaagg gcagccaaaa    1020
   cgtaacaccg ccccggtttt cccctggaaa ttccatattg gcacgcattc tattggctga    1080
   gctgcgttct acgtgggtat aagaggcgcg accagcgtcg gtaccgtcgc agtcttcggt    1140
   ctgaccaccg tagaacgcag agctcctcgc tgcaggcggc cgctctagaa ctcgtcgatc    1200
   gcagcg                                                               1206
<210> 12
   <211> 453
   <212> PRT
   <213> Infectious bursal disease virus 52/70
<220>
   <223> IBVD VP protein sequence
<400> 12
   Met Ile Asn Leu Gln Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Ser Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Ile Gly Gly Glu Leu Val Phe Gln Thr Ser Val Gln Gly Leu Val
                   245                 250                 255
   Leu Gly Ala Thr Ile Tyr Leu Ile Gly Phe Asp Gly Thr Ala Val Ile
               260                 265                 270
   Thr Arg Ala Val Ala Ala Asp Asn Gly Leu Thr Ala Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Ser Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg
       450
<210> 13
   <211> 74
   <212> DNA
   <213> Artificial Sequence
<220>
   <223> forward primer UL21/22F-GalKF primer
<400> 13
   gccgggcata tacagaacgt aagccaagct ggagtttgtg taagtatgtg cctgttgaca     60
   attaatcatc ggca                                                        74
<210> 14
   <211> 71
   <212> DNA
   <213> Artificial Sequence
<220>
   <223> reverse primer UL21/22R-GalKR
<400> 14
   cctgtactgt tgtgtttatt cgcgaaccgc ccttcccgca cgctgtagag tcagcactgt     60
   cctgctcctt g                                                           71
<210> 15
   <211> 74
   <212> DNA
   <213> Artificial Sequence
<220>
   <223> forward primer UL26/27F-GalKF
<400> 15
   ggatccgcta tgtcgacgta taagtttata cattttgcga ccgcaatagc cctgttgaca     60
   attaatcatc ggca                                                        74
<210> 16
   <211> 71
   <212> DNA
   <213> Artificial Sequence
<220>
   <223> reverse primer UL26/27R-GalKR
<400> 16
   tctcgataaa taaattctca cgtgcgcata cgattatttt acttttattt tcagcactgt 60
   cctgctcctt g 71
<210> 17
   <211> 74
   <212> DNA
   <213> Artificial Sequence
<220>
   <223> forward primer UL3/4F-GalKF
<400> 17
   taaaacgcgc ttgattgtcg agtggctgaa taaaatcttt attgatcgac cctgttgaca 60
   attaatcatc ggca 74
<210> 18
   <211> 70
   <212> DNA
   <213> Artificial Sequence
<220>
   <223> reverse primer UL3/4R-GalKR
<400> 18
   atagattccc cgccccatct atggttatta aatagaaata ggaaagcgat cagcactgtc 60
   ctgctccttg 70
<210> 19
   <211> 74
   <212> DNA
   <213> Artificial Sequence
<220>
   <223> forward primer UL10/11F-GalKF
<400> 19
   agtgaatggg atgaatagga acgcccgaaa cataataaaa cgctaaatct cctgttgaca 60
   attaatcatc ggca 74
<210> 20
   <211> 71
   <212> DNA
   <213> Artificial Sequence
<220>
   <223> reverse primer UL10/11R-GalKR
<400> 20
   aatgtaacga tacgttatag tgataaataa gtcgcgcgac aatcacttgt tcagcactgt 60
   cctgctcctt g 71
<210> 21
   <211> 74
   <212> DNA
   <213> Artificial Sequence
<220>
   <223> forward primer UL40/41F-GalKF
<400> 21
   gtatcgaacg atctttaatt agcctgtgtg caactgtact ttctacccct cctgttgaca 60
   attaatcatc ggca 74
<210> 22
   <211> 71
   <212> DNA
   <213> Artificial Sequence
<220>
   <223> reverse primer UL40/41R-GalKR
<400> 22
   acacggtgcg aaaagcgata atttgaattc atttattaat tcttgtgggt tcagcactgt 60
   cctgctcctt g 71
<210> 23
   <211> 74
   <212> DNA
   <213> Artificial Sequence
<220>
   <223> forward primer UL50/51F-GalKF
<400> 23
   gaaacggcag cattcgatac ggaatcgggc aggagcgaga gagagtgtgc cctgttgaca 60
   attaatcatc ggca 74
<210> 24
   <211> 71
   <212> DNA
   <213> Artificial Sequence
<220>
   <223> reverse primer UL50/51R-GalKR
<400> 24
   gtatgtaatc gtgcgcaact atacattatt gcccgctcga cccgaagccg tcagcactgt     60
   cctgctcctt g                                                          71
<210> 25
   <211> 20
   <212> DNA
   <213> Artificial Sequence
<220>
   <223> MDV-2 Forward primer
<400> 25
   agcatgcggg aagaaaagag 20
<210> 26
   <211> 21
   <212> DNA
   <213> Artificial Sequence
<220>
   <223> MDV-2 Reverse primer
<400> 26
   gaaaggtttt ccgctcccat a 21
<210> 27
   <211> 22
   <212> DNA
   <213> Artificial Sequence
<220>
   <223> MDV-2 PROBE
<400> 27
   cgcccgtaat gcacccgtga ct 22
<210> 28
   <211> 19
   <212> DNA
   <213> Artificial Sequence
<220>
   <223> Ovo forward primer
<400> 28
   cactgccact gggctctgt 19
<210> 29
   <211> 21
   <212> DNA
   <213> Artificial Sequence
<220>
   <223> Ovo reverse primer
<400> 29
   gcaatggcaa taaacctcca a 21
<210> 30
   <211> 30
   <212> DNA
   <213> Artificial Sequence
<220>
   <223> Ovo probe
<400> 30
   agtctggaga agtctgtgca gcagcctcca 30
<210> 31
   <211> 499
   <212> PRT
   <213> Gallid herpesvirus 1
<220>
   <223> glycoprotein E
<400> 31
   Met Asn Met Leu Val Ile Val Leu Ala Ser Cys Leu Ala Arg Leu Thr
   1               5                   10                  15
   Phe Ala Thr Arg His Val Leu Phe Leu Glu Gly Thr Gln Ala Val Leu
               20                  25                  30
   Gly Glu Asp Asp Pro Arg Asn Val Pro Glu Gly Thr Val Ile Lys Trp
           35                  40                  45
   Thr Lys Val Leu Arg Asn Ala Cys Lys Met Lys Ala Ala Asp Val Cys
       50                  55                  60
   Ser Ser Pro Asn Tyr Cys Phe His Asp Leu Ile Tyr Asp Gly Gly Lys
   65                  70                  75                  80
   Lys Asp Cys Pro Pro Ala Gly Pro Leu Ser Ala Asn Leu Val Ile Leu
                   85                  90                  95
   Leu Lys Arg Gly Glu Ser Phe Val Val Leu Gly Ser Gly Leu His Asn
               100                 105                 110
   Ser Asn Ile Thr Asn Ile Met Trp Thr Glu Tyr Gly Gly Leu Leu Phe
           115                 120                 125
   Asp Pro Val Thr Arg Ser Asp Glu Gly Ile Tyr Phe Arg Arg Ile Ser
       130                 135                 140
   Gln Pro Asp Leu Ala Met Glu Thr Thr Ser Tyr Asn Val Ser Val Leu
   145                 150                 155                 160
   Ser His Val Asp Glu Lys Ala Pro Ala Pro His Glu Val Glu Ile Asp
                   165                 170                 175
   Thr Ile Lys Pro Ser Glu Ala His Ala His Val Glu Leu Gln Met Leu
               180                 185                 190
   Pro Phe His Glu Leu Asn Asp Asn Ser Pro Thr Tyr Val Thr Pro Val
           195                 200                 205
   Leu Arg Val Phe Pro Pro Thr Glu His Val Lys Phe Asn Val Thr Tyr
       210                 215                 220
   Ser Trp Tyr Gly Phe Asp Val Lys Glu Glu Cys Glu Glu Val Lys Leu
   225                 230                 235                 240
   Phe Glu Pro Cys Val Tyr His Pro Thr Asp Gly Lys Cys Gln Phe Pro
                   245                 250                 255
   Ala Thr Asn Gln Arg Cys Leu Ile Gly Ser Val Leu Met Ala Glu Phe
               260                 265                 270
   Leu Gly Ala Ala Ser Leu Leu Asp Cys Ser Arg Asp Thr Leu Glu Asp
           275                 280                 285
   Cys His Glu Asn Arg Val Pro Asn Leu Arg Phe Asp Ser Arg Leu Ser
       290                 295                 300
   Glu Ser Arg Ala Gly Leu Val Ile Ser Pro Leu Ile Ala Ile Pro Lys
   305                 310                 315                 320
   Val Leu Ile Ile Val Val Ser Asp Gly Asp Ile Leu Gly Trp Ser Tyr
                   325                 330                 335
   Thr Val Leu Gly Lys Arg Asn Ser Pro Arg Val Val Val Glu Thr His
               340                 345                 350
   Met Pro Ser Lys Val Pro Met Asn Lys Val Val Ile Gly Ser Pro Gly
           355                 360                 365
   Pro Met Asp Glu Thr Gly Asn Tyr Lys Met Tyr Phe Val Val Ala Gly
       370                 375                 380
   Val Thr Ala Thr Cys Val Ile Leu Thr Cys Ala Leu Leu Val Gly Lys
   385                 390                 395                 400
   Lys Lys Cys Pro Ala His Gln Met Gly Thr Phe Ser Lys Thr Glu Pro
                   405                 410                 415
   Leu Tyr Ala Pro Leu Pro Lys Asn Glu Phe Glu Ala Gly Gly Leu Thr
               420                 425                 430
   Asp Asp Glu Glu Val Ile Tyr Asp Glu Val Tyr Glu Pro Leu Phe Arg
           435                 440                 445
   Gly Tyr Cys Lys Gln Glu Phe Arg Glu Asp Val Asn Thr Phe Phe Gly
       450                 455                 460
   Ala Val Val Glu Gly Glu Arg Ala Leu Asn Phe Lys Ser Ala Ile Ala
   465                 470                 475                 480
   Ser Met Ala Asp Arg Ile Leu Ala Asn Lys Ser Gly Arg Arg Asn Met
                   485                 490                 495
   Asp Ser Tyr
<210> 32
   <211> 362
   <212> PRT
   <213> Gallid herpesvirus 1
<220>
   <223> glycoprotein I
<400> 32
   Met Ala Ser Leu Leu Gly Thr Leu Ala Leu Leu Ala Ala Thr Leu Ala
   1               5                   10                  15
   Pro Phe Gly Ala Met Gly Ile Val Ile Thr Gly Asn His Val Ser Ala
               20                  25                  30
   Arg Ile Asp Asp Asp His Ile Val Ile Val Ala Pro Arg Pro Glu Ala
           35                  40                  45
   Thr Ile Gln Leu Gln Leu Phe Phe Met Pro Gly Gln Arg Pro His Lys
       50                  55                  60
   Pro Tyr Ser Gly Thr Val Arg Val Ala Phe Arg Ser Asp Ile Thr Asn
   65                  70                  75                  80
   Gln Cys Tyr Gln Glu Leu Ser Glu Glu Arg Phe Glu Asn Cys Thr His
                   85                  90                  95
   Arg Ser Ser Ser Val Phe Val Gly Cys Lys Val Thr Glu Tyr Thr Phe
               100                 105                 110
   Ser Ala Ser Asn Arg Leu Thr Gly Pro Pro His Pro Phe Lys Leu Thr
           115                 120                 125
   Ile Arg Asn Pro Arg Pro Asn Asp Ser Gly Met Phe Tyr Val Ile Val
       130                 135                 140
   Arg Leu Asp Asp Thr Lys Glu Pro Ile Asp Val Phe Ala Ile Gln Leu
   145                 150                 155                 160
   Ser Val Tyr Gln Phe Ala Asn Thr Ala Ala Thr Arg Gly Leu Tyr Ser
                   165                 170                 175
   Lys Ala Ser Cys Arg Thr Phe Gly Leu Pro Thr Val Gln Leu Glu Ala
               180                 185                 190
   Tyr Leu Arg Thr Glu Glu Ser Trp Arg Asn Trp Gln Ala Tyr Val Ala
           195                 200                 205
   Thr Glu Ala Thr Thr Thr Ser Ala Glu Ala Thr Thr Pro Thr Pro Val
       210                 215                 220
   Thr Ala Thr Ser Ala Ser Glu Leu Glu Ala Glu His Phe Thr Phe Pro
   225                 230                 235                 240
   Trp Leu Glu Asn Gly Val Asp His Tyr Glu Pro Thr Pro Ala Asn Glu
                   245                 250                 255
   Asn Ser Asn Val Thr Val Arg Leu Gly Thr Met Ser Pro Thr Leu Ile
               260                 265                 270
   Gly Val Thr Val Ala Ala Val Val Ser Ala Thr Ile Gly Leu Val Ile
           275                 280                 285
   Val Ile Ser Ile Val Thr Arg Asn Met Cys Thr Pro His Arg Lys Leu
       290                 295                 300
   Asp Thr Val Ser Gln Asp Asp Glu Glu Arg Ser Gln Thr Arg Arg Glu
   305                 310                 315                 320
   Ser Arg Lys Phe Gly Pro Met Val Ala Cys Glu Ile Asn Lys Gly Ala
                   325                 330                 335
   Asp Gln Asp Ser Glu Leu Val Glu Leu Val Ala Ile Val Asn Pro Ser
               340                 345                 350
   Ala Leu Ser Ser Pro Asp Ser Ile Lys Met
           355                 360
<210> 33
   <211> 883
   <212> PRT
   <213> Gallid herpesvirus 1
<220>
   <223> glycoprotein B
<400> 33
   Met Gln Ser Tyr Ile Ala Val Asn Ile Asp Met Ala Ser Leu Lys Met
   1               5                   10                  15
   Leu Ile Cys Val Cys Val Ala Ile Leu Ile Pro Ser Thr Leu Ser Gln
               20                  25                  30
   Asp Ser His Gly Ile Ala Gly Ile Ile Asp Pro Arg Asp Thr Ala Ser
           35                  40                  45
   Met Asp Val Gly Lys Ile Ser Phe Ser Glu Ala Ile Gly Ser Gly Ala
       50                  55                  60
   Pro Lys Glu Pro Gln Ile Arg Asn Arg Ile Phe Ala Cys Ser Ser Pro
   65                  70                  75                  80
   Thr Gly Ala Ser Val Ala Arg Leu Ala Gln Pro Arg His Cys His Arg
                   85                  90                  95
   His Ala Asp Ser Thr Asn Met Thr Glu Gly Ile Ala Val Val Phe Lys
               100                 105                 110
   Gln Asn Ile Ala Pro Tyr Val Phe Asn Val Thr Leu Tyr Tyr Lys His
           115                 120                 125
   Ile Thr Thr Val Thr Thr Trp Ala Leu Phe Ser Arg Pro Gln Ile Thr
       130                 135                 140
   Asn Glu Tyr Val Thr Arg Val Pro Ile Asp Tyr His Glu Ile Val Arg
   145                 150                 155                 160
   Ile Asp Arg Ser Gly Glu Cys Ser Ser Lys Ala Thr Tyr His Lys Asn
                   165                 170                 175
   Phe Met Phe Phe Glu Ala Tyr Asp Asn Asp Glu Ala Glu Lys Lys Leu
               180                 185                 190
   Pro Leu Val Pro Ser Leu Leu Arg Ser Thr Val Ser Lys Ala Phe His
           195                 200                 205
   Thr Thr Asn Phe Thr Lys Arg His Gln Thr Leu Gly Tyr Arg Thr Ser
       210                 215                 220
   Thr Ser Val Asp Cys Val Val Glu Tyr Leu Gln Ala Arg Ser Val Tyr
   225                 230                 235                 240
   Pro Tyr Asp Tyr Phe Gly Met Ala Thr Gly Asp Thr Val Glu Ile Ser
                   245                 250                 255
   Pro Phe Tyr Thr Lys Asn Thr Thr Gly Pro Arg Arg His Ser Val Tyr
               260                 265                 270
   Arg Asp Tyr Arg Phe Leu Glu Ile Ala Asn Tyr Gln Val Arg Asp Leu
           275                 280                 285
   Glu Thr Gly Gln Ile Arg Pro Pro Lys Lys Arg Asn Phe Leu Thr Asp
       290                 295                 300
   Glu Gln Phe Thr Ile Gly Trp Asp Ala Met Glu Glu Lys Glu Ser Val
   305                 310                 315                 320
   Cys Thr Leu Ser Lys Trp Ile Glu Val Pro Glu Ala Val Arg Val Ser
                   325                 330                 335
   Tyr Lys Asn Ser Tyr His Phe Ser Leu Lys Asp Met Thr Met Thr Phe
               340                 345                 350
   Ser Ser Gly Lys Gln Pro Phe Asn Ile Ser Arg Leu His Leu Ala Glu
           355                 360                 365
   Cys Val Pro Thr Ile Ala Thr Glu Ala Ile Asp Gly Ile Phe Ala Arg
       370                 375                 380
   Lys Tyr Ser Ser Thr His Val Arg Ser Gly Asp Ile Glu Tyr Tyr Leu
   385                 390                 395                 400
   Gly Ser Gly Gly Phe Leu Ile Ala Phe Gln Lys Leu Met Ser His Gly
                   405                 410                 415
   Leu Ala Glu Met Tyr Leu Glu Glu Ala Gln Arg Gln Asn His Leu Pro
               420                 425                 430
   Arg Gly Arg Glu Arg Arg Gln Ala Ala Gly Arg Arg Thr Ala Ser Leu
           435                 440                 445
   Gln Ser Gly Pro Gln Gly Asp Arg Ile Thr Thr His Ser Ser Ala Thr
       450                 455                 460
   Phe Ala Met Leu Gln Phe Ala Tyr Asp Lys Ile Gln Ala His Val Asn
   465                 470                 475                 480
   Glu Leu Ile Gly Asn Leu Leu Glu Ala Trp Cys Glu Leu Gln Asn Arg
                   485                 490                 495
   Gln Leu Ile Val Trp His Glu Met Lys Lys Leu Asn Pro Asn Ser Leu
               500                 505                 510
   Met Thr Ser Leu Phe Gly Gln Pro Val Ser Ala Arg Leu Leu Gly Asp
           515                 520                 525
   Ile Val Ala Val Ser Lys Cys Ile Glu Ile Pro Ile Glu Asn Ile Arg
       530                 535                 540
   Met Gln Asp Ser Met Arg Met Pro Gly Asp Pro Thr Met Cys Tyr Thr
   545                 550                 555                 560
   Arg Pro Val Leu Ile Phe Arg Tyr Ser Ser Ser Pro Glu Ser Gln Phe
                   565                 570                 575
   Ser Ala Asn Ser Thr Glu Asn His Asn Leu Asp Ile Leu Gly Gln Leu
               580                 585                 590
   Gly Glu His Asn Glu Ile Leu Gln Gly Arg Asn Leu Ile Glu Pro Cys
           595                 600                 605
   Met Ile Asn His Arg Arg Tyr Phe Leu Leu Gly Glu Asn Tyr Leu Leu
       610                 615                 620
   Tyr Glu Asp Tyr Thr Phe Val Arg Gln Val Asn Ala Ser Glu Ile Glu
   625                 630                 635                 640
   Glu Val Ser Ile Phe Ile Asn Leu Asn Ala Thr Ile Leu Glu Asp Leu
                   645                 650                 655
   Asp Phe Val Pro Val Glu Val Tyr Thr Arg Glu Glu Leu Arg Asp Thr
               660                 665                 670
   Gly Thr Leu Asn Tyr Asp Asp Val Val Arg Tyr Gln Asn Ile Tyr Asn
           675                 680                 685
   Lys Arg Phe Arg Asp Ile Asp Thr Val Ile Arg Gly Asp Arg Gly Asp
       690                 695                 700
   Ala Ile Phe Arg Ala Ile Ala Asp Phe Phe Gly Asn Thr Leu Gly Glu
   705                 710                 715                 720
   Val Gly Lys Ala Leu Gly Thr Val Val Met Thr Ala Ala Ala Ala Val
                   725                 730                 735
   Ile Ser Thr Val Ser Gly Ile Ala Ser Phe Leu Ser Asn Pro Phe Ala
               740                 745                 750
   Ala Leu Gly Ile Gly Ile Ala Val Val Val Ser Ile Ile Leu Gly Leu
           755                 760                 765
   Leu Ala Phe Lys Tyr Val Met Asn Leu Lys Ser Asn Pro Val Gln Val
       770                 775                 780
   Leu Phe Pro Gly Ala Val Pro Pro Ala Gly Thr Pro Pro Arg Pro Ser
   785                 790                 795                 800
   Arg Arg Tyr Tyr Lys Asp Glu Glu Glu Val Glu Glu Asp Ser Asp Glu
                   805                 810                 815
   Asp Asp Arg Ile Leu Ala Thr Arg Val Leu Lys Gly Leu Glu Leu Leu
               820                 825                 830
   His Lys Asp Glu Gln Lys Ala Arg Arg Gln Lys Ala Arg Phe Ser Ala
           835                 840                 845
   Phe Ala Lys Asn Met Arg Asn Leu Phe Arg Arg Lys Pro Arg Thr Lys
       850                 855                 860
   Glu Asp Asp Tyr Pro Leu Leu Glu Tyr Pro Ser Trp Ala Glu Glu Ser
   865                 870                 875                 880
   Glu Asp Glu
<210> 34
   <211> 553
   <212> PRT
   <213> Newcastle disease virus
<220>
   <223> NDV-F
<400> 34
   Met Gly Ser Arg Pro Phe Thr Lys Asn Pro Ala Pro Met Met Leu Thr
   1               5                   10                  15
   Ile Arg Val Ala Leu Val Leu Ser Cys Ile Cys Pro Ala Asn Ser Ile
               20                  25                  30
   Asp Gly Arg Pro Phe Ala Ala Ala Gly Ile Val Val Thr Gly Asp Lys
           35                  40                  45
   Ala Val Asn Ile Tyr Thr Ser Ser Gln Thr Gly Ser Ile Ile Val Lys
       50                  55                  60
   Leu Leu Pro Asn Leu Pro Lys Asp Lys Glu Ala Cys Ala Lys Ala Pro
   65                  70                  75                  80
   Leu Asp Ala Tyr Asn Arg Thr Leu Thr Thr Leu Leu Thr Pro Leu Gly
                   85                  90                  95
   Asp Ser Ile Arg Arg Ile Gln Glu Ser Val Thr Thr Ser Gly Gly Gly
               100                 105                 110
   Arg Gln Gly Arg Leu Ile Gly Ala Ile Ile Gly Gly Val Ala Leu Gly
           115                 120                 125
   Val Ala Thr Ala Ala Gln Ile Thr Ala Ala Ala Ala Leu Ile Gln Ala
       130                 135                 140
   Lys Gln Asn Ala Ala Asn Ile Leu Arg Leu Lys Glu Ser Ile Ala Ala
   145                 150                 155                 160
   Thr Asn Glu Ala Val His Glu Val Thr Asp Gly Leu Ser Gln Leu Ala
                   165                 170                 175
   Val Ala Val Gly Lys Met Gln Gln Phe Val Asn Asp Gln Phe Asn Lys
                   180                 185                 190
   Thr Ala Gln Glu Leu Asp Cys Ile Lys Ile Ala Gln Gln Val Gly Val
               195                 200                 205
   Glu Leu Asn Leu Tyr Leu Thr Glu Leu Thr Thr Val Phe Gly Pro Gln
       210                 215                 220
   Ile Thr Ser Pro Ala Leu Asn Lys Leu Thr Ile Gln Ala Leu Tyr Asn
   225                 230                 235                 240
   Leu Ala Gly Gly Asn Met Asp Tyr Leu Leu Thr Lys Leu Gly Ile Gly
                   245                 250                 255
   Asn Asn Gln Leu Ser Ser Leu Ile Gly Ser Gly Leu Ile Thr Gly Asn
                   260                 265                 270
   Pro Ile Leu Tyr Asp Ser Gln Thr Gln Leu Leu Gly Ile Gln Val Thr
               275                 280                 285
   Leu Pro Ser Val Gly Asn Leu Asn Asn Met Arg Ala Thr Tyr Leu Glu
       290                 295                 300
   Thr Leu Ser Val Ser Thr Thr Arg Gly Phe Ala Ser Ala Leu Val Pro
   305                 310                 315                 320
   Lys Val Val Thr Gln Val Gly Ser Val Ile Glu Glu Leu Asp Thr Ser
                   325                 330                 335
   Tyr Cys Ile Glu Thr Asp Leu Asp Leu Tyr Cys Thr Arg Ile Val Thr
                   340                 345                 350
   Phe Pro Met Ser Pro Gly Ile Tyr Ser Cys Leu Ser Gly Asn Thr Ser
               355                 360                 365
   Ala Cys Met Tyr Ser Lys Thr Glu Gly Ala Leu Thr Thr Pro Tyr Met
       370                 375                 380
   Thr Ile Lys Gly Ser Val Ile Ala Asn Cys Lys Met Thr Thr Cys Arg
   385                 390                 395                 400
   Cys Val Asn Pro Pro Gly Ile Ile Ser Gln Asn Tyr Gly Glu Ala Val
                   405                 410                 415
   Ser Leu Ile Asp Lys Gln Ser Cys Asn Val Leu Ser Leu Gly Gly Ile
                   420                 425                 430
   Thr Leu Arg Leu Ser Gly Glu Phe Asp Val Thr Tyr Gln Lys Asn Ile
               435                 440                 445
   Ser Ile Gln Asp Ser Gln Val Ile Ile Thr Gly Asn Leu Asp Ile Ser
       450                 455                 460
   Thr Glu Leu Gly Asn Val Asn Asn Ser Ile Ser Asn Ala Leu Asn Lys
   465                 470                 475                 480
   Leu Glu Glu Ser Asn Arg Lys Leu Asp Lys Val Asn Val Lys Leu Thr
                   485                 490                 495
   Ser Thr Ser Ala Leu Ile Thr Tyr Ile Val Leu Thr Ile Ile Ser Leu
                   500                 505                 510
   Val Phe Gly Ile Leu Ser Leu Ile Leu Ala Cys Tyr Leu Met Tyr Lys
               515                 520                 525
   Gln Lys Ala Gln Gln Lys Thr Leu Leu Trp Leu Gly Asn Asn Thr Leu
       530                 535                 540
   Asp Gln Met Arg Ala Thr Thr Lys Met
   545                 550
<210> 35
   <211> 577
   <212> PRT
   <213> Newcastle disease virus
<220>
   <223> NDV-NH
<400> 35
   Met Asp Arg Ala Val Ser Gln Val Ala Leu Glu Asn Asp Glu Arg Glu
   1               5                   10                  15
   Ala Lys Asn Thr Trp Arg Leu Ile Phe Arg Ile Ala Ile Leu Phe Leu
               20                  25                  30
   Thr Val Val Thr Leu Ala Ile Ser Val Ala Ser Leu Leu Tyr Ser Met
           35                  40                  45
   Gly Ala Ser Thr Pro Ser Asp Leu Val Gly Ile Pro Thr Arg Ile Ser
       50                  55                  60
   Arg Ala Glu Glu Lys Ile Thr Ser Thr Leu Gly Ser Asn Gln Asp Val
   65                  70                  75                  80
   Val Asp Arg Ile Tyr Lys Gln Val Ala Leu Glu Ser Pro Leu Ala Leu
                   85                  90                  95
   Leu Asn Thr Glu Thr Thr Ile Met Asn Ala Ile Thr Ser Leu Ser Tyr
               100                 105                 110
   Gln Ile Asn Gly Ala Ala Asn Asn Ser Gly Trp Gly Ala Pro Ile His
           115                 120                 125
   Asp Pro Asp Tyr Ile Gly Gly Ile Gly Lys Glu Leu Ile Val Asp Asp
       130                 135                 140
   Ala Ser Asp Val Thr Ser Phe Tyr Pro Ser Ala Phe Gln Glu His Leu
   145                 150                 155                 160
   Asn Phe Ile Pro Ala Pro Thr Thr Gly Ser Gly Cys Thr Arg Ile Pro
                   165                 170                 175
   Ser Phe Asp Met Ser Ala Thr His Tyr Cys Tyr Thr His Asn Val Ile
               180                 185                 190
   Leu Ser Gly Cys Arg Asp His Ser His Ser Tyr Gln Tyr Leu Ala Leu
           195                 200                 205
   Gly Val Leu Arg Thr Ser Ala Thr Gly Arg Val Phe Phe Ser Thr Leu
       210                 215                 220
   Arg Ser Ile Asn Leu Asp Asp Thr Gln Asn Arg Lys Ser Cys Ser Val
   225                 230                 235                 240
   Ser Ala Thr Pro Leu Gly Cys Asp Met Leu Cys Ser Lys Ala Thr Glu
                   245                 250                 255
   Thr Glu Glu Glu Asp Tyr Asn Ser Ala Val Pro Thr Arg Met Val His
               260                 265                 270
   Gly Arg Leu Gly Phe Asp Gly Gln Tyr His Glu Lys Asp Leu Asp Val
           275                 280                 285
   Thr Thr Leu Phe Gly Asp Trp Val Ala Asn Tyr Pro Gly Val Gly Gly
       290                 295                 300
   Gly Ser Phe Ile Asp Ser Arg Val Trp Phe Ser Val Tyr Gly Gly Leu
   305                 310                 315                 320
   Lys Pro Asn Ser Pro Ser Asp Thr Val Gln Glu Gly Lys Tyr Val Ile
                   325                 330                 335
   Tyr Lys Arg Tyr Asn Asp Thr Cys Pro Asp Glu Gln Asp Tyr Gln Ile
               340                 345                 350
   Arg Met Ala Lys Ser Ser Tyr Lys Pro Gly Arg Phe Gly Gly Lys Arg
           355                 360                 365
   Ile Gln Gln Ala Ile Leu Ser Ile Lys Val Ser Thr Ser Leu Gly Glu
       370                 375                 380
   Asp Pro Val Leu Thr Val Pro Pro Asn Thr Val Thr Leu Met Gly Ala
   385                 390                 395                 400
   Glu Gly Arg Ile Leu Thr Val Gly Thr Ser His Phe Leu Tyr Gln Arg
                   405                 410                 415
   Gly Ser Ser Tyr Phe Ser Pro Ala Leu Leu Tyr Pro Met Thr Val Ser
               420                 425                 430
   Asn Lys Thr Ala Thr Leu His Ser Pro Tyr Thr Phe Asn Ala Phe Thr
           435                 440                 445
   Arg Pro Gly Ser Ile Pro Cys Gln Ala Ser Ala Arg Cys Pro Asn Ser
       450                 455                 460
   Cys Val Thr Gly Val Tyr Thr Asp Pro Tyr Pro Leu Ile Phe Tyr Arg
   465                 470                 475                 480
   Asn His Thr Leu Arg Gly Val Phe Gly Thr Met Leu Asp Gly Glu Gln
                   485                 490                 495
   Ala Arg Leu Asn Pro Ala Ser Ala Val Phe Asp Ser Thr Ser Arg Ser
               500                 505                 510
   Arg Ile Thr Arg Val Ser Ser Ser Ser Ile Lys Ala Ala Tyr Thr Thr
           515                 520                 525
   Ser Thr Cys Phe Lys Val Val Lys Thr Asn Lys Thr Tyr Cys Leu Ser
       530                 535                 540
   Ile Ala Glu Ile Ser Asn Thr Leu Phe Gly Glu Phe Arg Ile Val Pro
   545                 550                 555                 560
   Leu Leu Val Glu Ile Leu Lys Asp Asp Gly Val Arg Glu Ala Arg Ser
                   565                 570                 575
   Gly
<210> 36
   <211> 568
   <212> PRT
   <213> Influenza A virus
<220>
   <223> H5 HA
<400> 36
   Met Glu Lys Ile Val Leu Leu Phe Ala Ile Val Ser Leu Val Lys Ser
   1               5                   10                  15
   Asp His Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asn Gly Val Lys
       50                  55                  60
   Pro Leu Ile Leu Lys Asp Cys Ser Val Ala Gly Trp Leu Leu Gly Asn
   65                  70                  75                  80
   Pro Lys Cys Asp Glu Phe Ile Asp Val Pro Glu Trp Ser Tyr Ile Val
                   85                  90                  95
   Glu Lys Ala Asn Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Arg Ile Ile Pro Lys Asp Ser Trp Gln Asp His Glu Ala Ser
       130                 135                 140
   Leu Gly Val Ser Ala Ala Cys Ser His Gln Gly Asn Ser Ser Phe Phe
   145                 150                 155                 160
   Arg Asn Val Val Trp Leu Leu Lys Lys Asp Asp Ala Tyr Pro Ile Ile
                   165                 170                 175
   Lys Lys Ser Tyr Asn Asn Thr Asn Lys Glu Asp Leu Leu Val Leu Trp
               180                 185                 190
   Gly Ile His His Pro Asn Asp Gly Ala Glu Gln Ile Lys Leu Tyr Gln
           195                 200                 205
   Asn Pro Thr Thr Tyr Val Ser Ile Gly Thr Ser Thr Leu Asn Gln Arg
       210                 215                 220
   Leu Val Pro Arg Ile Ala Thr Arg Ser Lys Val Asn Gly Gln Ser Gly
   225                 230                 235                 240
   Arg Ile Asp Phe Phe Trp Thr Ile Leu Lys Pro Asn Asp Ala Ile Asn
                   245                 250                 255
   Phe Glu Ser Asn Gly Asn Phe Ile Ala Pro Glu Tyr Ala Tyr Lys Ile
               260                 265                 270
   Val Lys Lys Gly Asp Ser Thr Ile Met Arg Ser Glu Val Glu Tyr Gly
           275                 280                 285
   Asn Cys Ser Thr Arg Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Lys Lys Leu Val Leu Ala Thr Gly Leu Arg Asn Ser
                   325                 330                 335
   Pro Gln Ile Glu Arg Arg Arg Arg Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Arg Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Lys Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Val
       530                 535                 540
   Leu Ala Ile Met Val Ala Gly Leu Ser Leu Trp Met Cys Ser Asn Gly
   545                 550                 555                 560
   Ser Leu Gln Cys Arg Ile Cys Ile
                   565
<210> 37
   <211> 560
   <212> PRT
   <213> Influenza A virus
<220>
   <223> H7 HA
<400> 37
   Met Asn Thr Gln Ile Leu Val Phe Ile Ala Cys Val Leu Ile Lys Ala
   1               5                   10                  15
   Lys Gly Asp Lys Ile Cys Leu Gly His His Ala Val Ala Asn Gly Thr
               20                  25                  30
   Lys Val Asn Thr Leu Thr Glu Arg Gly Ile Glu Val Val Asn Ala Thr
           35                  40                  45
   Glu Thr Val Glu Thr Ala Asn Ile Glu Lys Ile Cys Thr Lys Gly Lys
       50                  55                  60
   Arg Pro Thr Asp Leu Gly Gln Cys Gly Leu Leu Gly Thr Leu Ile Gly
   65                  70                  75                  80
   Pro Pro Gln Cys Asp Gln Phe Leu Glu Phe Glu Ser Asp Leu Ile Ile
                   85                  90                  95
   Glu Arg Arg Glu Gly Asn Asp Val Cys Tyr Pro Gly Lys Phe Thr Asn
               100                 105                 110
   Glu Glu Ser Leu Arg Gln Ile Leu Arg Gly Ser Gly Gly Val Asp Lys
           115                 120                 125
   Glu Ser Met Gly Phe Thr Tyr Ser Gly Ile Arg Thr Asn Gly Ala Thr
       130                 135                 140
   Ser Ala Cys Arg Arg Ser Gly Ser Ser Phe Tyr Ala Glu Met Lys Trp
   145                 150                 155                 160
   Leu Leu Ser Asn Ser Asp Asn Ala Ala Phe Pro Gln Met Thr Lys Ser
                   165                 170                 175
   Tyr Arg Asn Pro Arg Asn Lys Pro Ala Leu Ile Val Trp Gly Ile His
               180                 185                 190
   His Ser Gly Ser Thr Thr Glu Gln Thr Lys Leu Tyr Gly Ser Gly Asn
           195                 200                 205
   Lys Leu Ile Thr Val Gly Ser Ser Lys Tyr Gln Gln Ser Phe Thr Pro
       210                 215                 220
   Ser Pro Gly Ala Arg Pro Gln Val Asn Gly Gln Ser Gly Arg Ile Asp
   225                 230                 235                 240
   Phe His Trp Leu Leu Leu Asp Pro Asn Asp Thr Val Thr Phe Thr Phe
                   245                 250                 255
   Asn Gly Ala Phe Ile Ala Pro Asp Arg Ala Ser Phe Phe Arg Gly Glu
               260                 265                 270
   Ser Leu Gly Val Gln Ser Asp Val Pro Leu Asp Ser Ser Cys Gly Gly
           275                 280                 285
   Asp Cys Phe His Ser Gly Gly Thr Ile Val Ser Ser Leu Pro Phe Gln
       290                 295                 300
   Asn Ile Asn Ser Arg Thr Val Gly Lys Cys Pro Arg Tyr Val Lys Gln
   305                 310                 315                 320
   Ser Ser Leu Leu Leu Ala Thr Gly Met Arg Asn Val Pro Glu Asn Pro
                   325                 330                 335
   Lys Thr Arg Gly Leu Phe Gly Ala Ile Ala Gly Phe Ile Glu Asn Gly
               340                 345                 350
   Trp Glu Gly Leu Ile Asp Gly Trp Tyr Gly Phe Arg His Gln Asn Ala
           355                 360                 365
   Gln Gly Glu Gly Ile Ala Ala Asp Tyr Lys Ser Thr Gln Ser Ala Ile
       370                 375                 380
   Asp Gln Ile Thr Gly Lys Leu Asn Arg Leu Ile Asp Lys Thr Asn Gln
   385                 390                 395                 400
   Gln Phe Glu Leu Ile Asp Asn Glu Phe Asn Glu Ile Glu Gln Gln Ile
                   405                 410                 415
   Gly Asn Val Ile Asn Trp Thr Arg Asp Ser Met Thr Glu Val Trp Ser
               420                 425                 430
   Tyr Asn Ala Glu Leu Leu Val Ala Met Glu Asn Gln His Thr Ile Asp
           435                 440                 445
   Leu Ala Asp Ser Glu Met Asn Lys Leu Tyr Glu Arg Val Arg Lys Gln
       450                 455                 460
   Leu Arg Glu Asn Ala Glu Glu Asp Gly Thr Gly Cys Phe Glu Ile Phe
   465                 470                 475                 480
   His Lys Cys Asp Asp Gln Cys Met Glu Ser Ile Arg Asn Asn Thr Tyr
                   485                 490                 495
   Asp His Thr Gln Tyr Arg Ala Glu Ser Leu Gln Asn Arg Ile Gln Ile
               500                 505                 510
   Asp Pro Val Lys Leu Ser Ser Gly Tyr Lys Asp Ile Ile Leu Trp Phe
           515                 520                 525
   Ser Phe Gly Ala Ser Cys Phe Leu Leu Leu Ala Ile Ala Met Gly Leu
       530                 535                 540
   Val Phe Ile Cys Ile Lys Asn Gly Asn Met Arg Cys Thr Ile Cys Ile
   545                 550                 555                 560
<210> 38
   <211> 560
   <212> PRT
   <213> Influenza A virus
<220>
   <223> H9 HA
<400> 38
   Met Glu Thr Val Ser Leu Ile Thr Ile Leu Leu Val Ala Thr Val Ser
   1               5                   10                  15
   Asn Ala Asp Lys Ile Cys Ile Gly Tyr Gln Ser Thr Asn Ser Thr Glu
               20                  25                  30
   Thr Val Asp Thr Leu Thr Glu Asn Asn Val Pro Val Thr His Ala Lys
           35                  40                  45
   Glu Leu Leu His Thr Glu His Asn Gly Met Leu Cys Ala Thr Ser Leu
       50                  55                  60
   Gly His Pro Leu Ile Leu Asp Thr Cys Thr Ile Glu Gly Leu Ile Tyr
   65                  70                  75                  80
   Gly Asn Pro Ser Cys Asp Leu Leu Leu Gly Gly Arg Glu Trp Ser Tyr
                   85                  90                  95
   Ile Val Glu Arg Pro Ser Ala Val Asn Gly Leu Cys Tyr Pro Gly Asn
               100                 105                 110
   Val Glu Asn Leu Glu Glu Leu Arg Ser Leu Phe Ser Ser Ala Arg Ser
           115                 120                 125
   Tyr Gln Arg Ile Gln Ile Phe Pro Asp Thr Ile Trp Asn Val Ser Tyr
       130                 135                 140
   Ser Gly Thr Ser Lys Ala Cys Ser Asp Ser Phe Tyr Arg Ser Met Arg
   145                 150                 155                 160
   Trp Leu Thr Gln Lys Asn Asn Ala Tyr Pro Ile Gln Asp Ala Gln Tyr
                   165                 170                 175
   Thr Asn Asn Gln Glu Lys Asn Ile Leu Phe Met Trp Gly Ile Asn His
               180                 185                 190
   Pro Pro Thr Asp Thr Ala Gln Thr Asn Leu Tyr Thr Arg Thr Asp Thr
           195                 200                 205
   Thr Thr Ser Val Ala Thr Glu Glu Ile Asn Arg Ile Phe Lys Pro Leu
       210                 215                 220
   Ile Gly Pro Arg Pro Leu Val Asn Gly Leu Met Gly Arg Ile Asp Tyr
   225                 230                 235                 240
   Tyr Trp Ser Val Leu Lys Pro Gly Gln Thr Leu Arg Ile Arg Ser Asn
                   245                 250                 255
   Gly Asn Leu Ile Ala Pro Trp Tyr Gly His Ile Leu Ser Gly Glu Ser
               260                 265                 270
   His Gly Arg Ile Leu Lys Thr Asp Leu Lys Arg Gly Ser Cys Thr Val
           275                 280                 285
   Gln Cys Gln Thr Glu Lys Gly Gly Leu Asn Thr Thr Leu Pro Phe Gln
       290                 295                 300
   Asn Val Ser Lys Tyr Ala Phe Gly Asn Cys Ser Lys Tyr Ile Gly Ile
   305                 310                 315                 320
   Lys Ser Leu Lys Leu Ala Val Gly Leu Arg Asn Val Pro Ser Arg Ser
                   325                 330                 335
   Ser Arg Gly Leu Phe Gly Ala Ile Ala Gly Phe Ile Glu Gly Gly Trp
               340                 345                 350
   Ser Gly Leu Val Ala Gly Trp Tyr Gly Phe Gln His Ser Asn Asp Gln
           355                 360                 365
   Gly Val Gly Met Ala Ala Asp Arg Asp Ser Thr Gln Arg Ala Ile Asp
       370                 375                 380
   Lys Ile Thr Ser Lys Val Asn Asn Ile Val Asp Lys Met Asn Lys Gln
   385                 390                 395                 400
   Tyr Glu Ile Ile Asp His Glu Phe Ser Glu Val Glu Thr Arg Leu Asn
                   405                 410                 415
   Met Ile Asn Asn Lys Ile Asp Asp Gln Ile Gln Asp Ile Trp Ala Tyr
               420                 425                 430
   Asn Ala Glu Leu Leu Val Leu Leu Glu Asn Gln Lys Thr Leu Asp Glu
           435                 440                 445
   His Asp Ala Asn Val Asn Asn Leu Tyr Asn Lys Val Lys Arg Ala Leu
       450                 455                 460
   Gly Ser Asn Ala Val Glu Asp Gly Lys Gly Cys Phe Glu Leu Tyr His
   465                 470                 475                 480
   Lys Cys Asp Asp Gln Cys Met Glu Thr Ile Arg Asn Gly Thr Tyr Asn
                   485                 490                 495
   Arg Arg Lys Tyr Gln Glu Glu Ser Lys Leu Glu Arg Gln Lys Ile Glu
               500                 505                 510
   Gly Val Lys Leu Glu Ser Glu Gly Thr Tyr Lys Ile Leu Thr Ile Tyr
           515                 520                 525
   Ser Thr Val Ala Ser Ser Leu Val Ile Ala Met Gly Phe Ala Ala Phe
       530                 535                 540
   Leu Phe Trp Ala Met Ser Asn Gly Ser Cys Arg Cys Asn Ile Cys Ile
   545 550 555 560
<210> 39
   <211> 449
   <212> PRT
   <213> Influenza A virus
<220>
   <223> H5N1 neuraminidase
<400> 39
   Met Asn Pro Asn Arg Lys Ile Ile Thr Ile Gly Ser Ile Cys Met Val
   1 5 10 15
   Ile Gly Ile Val Ser Leu Met Leu Gln Ile Gly Asn Ile Ile Ser Ile
   20 25 30
   Trp Val Ser His Ser Ile Gln Thr Glu Asn Gln His Gln Ser Glu Pro
   35 40 45
   Ile Ser Asn Thr Asn Phe Leu Thr Glu Lys Ala Val Ala Ser Val Thr
   50 55 60
   Leu Ala Gly Asn Ser Ser Leu Cys Pro Ile Ser Gly Trp Ala Val His
   65 70 75 80
   Ser Lys Asp Asn Gly Ile Arg Ile Gly Ser Lys Gly Asp Val Phe Val
   85 90 95
   Ile Arg Glu Pro Phe Ile Ser Cys Ser His Leu Glu Cys Arg Thr Phe
   100 105 110
   Phe Leu Thr Gln Gly Ala Leu Leu Asn Asp Lys His Ser Asn Gly Thr
   115 120 125
   Val Lys Asp Arg Ser Pro His Arg Thr Leu Met Ser Cys Pro Val Gly
   130 135 140
   Glu Ala Pro Ser Pro Tyr Asn Ser Arg Phe Glu Ser Val Ala Trp Ser
   145 150 155 160
   Ala Ser Ala Cys His Asp Gly Thr Ser Trp Leu Thr Ile Gly Ile Ser
   165 170 175
   Gly Pro Asp Asn Gly Ala Val Ala Val Leu Lys Tyr Asn Gly Ile Ile
   180 185 190
   Thr Asp Thr Ile Lys Ser Trp Arg Asn Asn Ile Leu Arg Thr Gln Glu
   195 200 205
   Ser Glu Cys Ala Cys Val Asn Gly Ser Cys Phe Thr Val Met Thr Asp
   210 215 220
   Gly Pro Ser Asn Gly Gln Ala Ser Tyr Lys Ile Phe Lys Met Glu Lys
   225 230 235 240
   Gly Lys Val Val Lys Ser Val Glu Leu Asp Ala Pro Asn Tyr His Tyr
   245 250 255
   Glu Glu Cys Ser Cys Tyr Pro Asp Ala Gly Glu Ile Thr Cys Val Cys
   260 265 270
   Arg Asp Asn Trp His Gly Ser Asn Arg Pro Trp Val Ser Phe Asn Gln
   275 280 285
   Asn Leu Glu Tyr Gln Ile Gly Tyr Ile Cys Ser Gly Val Phe Gly Asp
   290 295 300
   Asn Pro Arg Pro Asn Asp Gly Thr Gly Ser Cys Gly Pro Met Ser Leu
   305 310 315 320
   Asn Gly Ala Tyr Gly Val Lys Gly Phe Ser Phe Lys Tyr Gly Asn Gly
   325 330 335
   Val Trp Ile Gly Arg Thr Lys Ser Thr Asn Ser Arg Ser Gly Phe Glu
   340 345 350
   Met Ile Trp Asp Pro Asn Gly Trp Thr Gly Thr Asp Ser Ser Phe Ser
   355 360 365
   Val Lys Gln Asp Ile Val Ala Ile Thr Asp Trp Ser Gly Tyr Ser Gly
   370 375 380
   Ser Phe Val Gln His Pro Glu Leu Thr Gly Leu Asp Cys Ile Arg Pro
   385                 390                 395                 400
   Cys Phe Trp Ile Glu Leu Ile Arg Gly Arg Pro Lys Glu Ser Thr Ile
                   405                 410                 415
   Trp Thr Ser Gly Ser Ser Ile Ser Phe Cys Gly Val Asn Ser Asp Thr
               420                 425                 430
   Val Gly Trp Ser Trp Pro Asp Gly Ala Glu Leu Pro Phe Thr Ile Asp
           435                 440                 445
   Lys
<210> 40
   <211> 1158
   <212> PRT
   <213> Infectious bronchitis virus
<220>
   <223> spike protein
<400> 40
   Met Leu Gly Lys Ser Leu Leu Ile Val Thr Val Leu Phe Ala Leu Cys
   1               5                   10                  15
   Ser Ala Thr Leu Tyr Thr His Asp Tyr Val Tyr Tyr Tyr Gln Ser Ala
               20                  25                  30
   Tyr Arg Pro Pro Asn Gly Trp His Leu Gln Gly Gly Ala Tyr Ala Val
           35                  40                  45
   Val Asn Ser Thr Asn Lys Thr Asn Asn Ala Gly Ala Ala Ser Glu Cys
       50                  55                  60
   Ser Val Gly Val Leu Phe Asn Tyr Thr Asn Gly Asn Asp Val Gly Tyr
   65                  70                  75                  80
   Asn Asn Ser Ala Ser Ser Val Ala Met Thr Ala Pro Leu Pro Gly Met
                   85                  90                  95
   Ser Trp Ser Lys Thr Gln Phe Cys Thr Ala His Cys Asn Phe Ser Asp
               100                 105                 110
   Phe Thr Val Phe Val Thr His Cys Phe Ala Asn Ser Cys Pro Leu Thr
           115                 120                 125
   Gly Arg Ile Glu Lys Asn His Ile Arg Ile Ser Ala Met Arg Asn Gly
       130                 135                 140
   Ser Leu Phe Tyr Asn Leu Thr Val Ser Val Ser Lys Tyr Pro Lys Phe
   145                 150                 155                 160
   Lys Ser Leu Gln Cys Val Asn Asn Phe Thr Ser Val Tyr Leu Asn Gly
                   165                 170                 175
   Asp Leu Val Phe Thr Ser Asn Lys Thr Thr Asp Val Ile Gly Ala Gly
               180                 185                 190
   Val Tyr Phe Lys Ala Gly Gly Pro Ile Thr Tyr Lys Ile Met Lys Glu
           195                 200                 205
   Phe Lys Val Leu Ala Tyr Phe Val Asn Gly Thr Val Gln Asp Val Ile
       210                 215                 220
   Leu Cys Asp Asp Thr Pro Arg Gly Leu Leu Ala Cys Gln Tyr Asn Thr
   225                 230                 235                 240
   Gly Asn Phe Ser Asp Gly Phe Tyr Pro Phe Thr Asn Ser Ser Leu Val
                   245                 250                 255
   Lys Lys Lys Phe Ile Val Tyr Arg Glu Ser Ser Val Asn Thr Thr Leu
               260                 265                 270
   Ile Leu Thr Asn Tyr Thr Phe Tyr Asn Val Thr Asn Ala Pro Pro Asn
           275                 280                 285
   Gln Gly Gly Val Gln Ser Ile Leu Thr Tyr Gln Thr Gln Thr Ala Gln
       290                 295                 300
   Ser Gly Tyr Tyr Asn Phe Asn Leu Ser Phe Leu Ser Ser Phe Val Tyr
   305                 310                 315                 320
   Lys Gln Ser Asp Tyr Met Tyr Gly Ser Tyr His Pro Ala Cys Asn Phe
                   325                 330                 335
   Arg Leu Glu Thr Ile Asn Asn Gly Leu Trp Phe Asn Ser Leu Ser Val
               340                 345                 350
   Ser Leu Ala Tyr Gly Pro Leu Gln Gly Gly Cys Lys Gln Ser Val Phe
           355                 360                 365
   Ser Ser Arg Ala Thr Cys Cys Tyr Ala Tyr Ser Tyr Asn Gly Pro Arg
       370                 375                 380
   Ala Cys Lys Gly Val Tyr Ala Gly Glu Leu Arg Gln Asn Phe Glu Cys
   385                 390                 395                 400
   Gly Leu Leu Val Tyr Val Thr Lys Ser Asp Gly Ser Arg Ile Gln Thr
                   405                 410                 415
   Ala Thr Glu Pro Pro Val Ile Thr Gln His Asn Tyr Asn Asn Ile Thr
               420                 425                 430
   Leu Asn Thr Cys Val Asp Tyr Asn Ile Tyr Gly Arg Val Gly Arg Gly
           435                 440                 445
   Phe Ile Thr Asn Val Thr Asp Leu Ser Ser Ser Tyr Asn Tyr Leu Ala
       450                 455                 460
   Asp Gly Gly Leu Ala Ile Leu Asp Thr Ser Gly Ala Ile Asp Ile Phe
   465                 470                 475                 480
   Val Val Gln Gly Glu His Gly Phe Asn Tyr Tyr Lys Val Asn Pro Cys
                   485                 490                 495
   Glu Asp Val Asn Gln Gln Phe Val Val Ser Gly Gly Lys Leu Val Gly
               500                 505                 510
   Ile Leu Thr Ser Arg Asn Ala Ser Gly Ser Gln Pro Leu Glu Asn Gln
           515                 520                 525
   Phe Tyr Ile Lys Leu Thr Lys Glu Thr Arg Arg Phe Arg Arg Ser Thr
       530                 535                 540
   Ser Glu Asn Val Thr Ser Cys His Tyr Val Ser Tyr Gly Arg Phe Cys
   545                 550                 555                 560
   Ile Gln Pro Asp Gly Ser Ile Lys Gln Ile Val Pro Gln Glu Leu Gln
                   565                 570                 575
   Asn Phe Val Ala Pro Leu Leu Asn Val Thr Glu Asn Val Leu Ile Pro
               580                 585                 590
   Asn Ser Phe Asn Leu Thr Val Thr Asp Glu Tyr Ile Gln Thr Arg Met
           595                 600                 605
   Asp Lys Val Gln Ile Asn Cys Leu Gln Tyr Val Cys Gly Asn Ser Leu
       610                 615                 620
   Asp Cys Arg Lys Leu Phe Gln Gln Tyr Gly Pro Val Cys Asp Asn Ile
   625                 630                 635                 640
   Leu Ser Ile Val Asn Ser Val Gly Gln Lys Glu Asp Met Glu Leu Leu
                   645                 650                 655
   Ser Phe Tyr Ser Ser Thr Lys Pro Ala Gly Tyr Asn Ala Pro Val Phe
               660                 665                 670
   Ser Asn Ile Ser Thr Gly Asp Phe Asn Ile Ser Leu Leu Leu Thr Pro
           675                 680                 685
   Ser Ser Ser Pro Arg Gly Arg Ser Phe Ile Glu Asp Leu Leu Phe Thr
       690                 695                 700
   Ser Val Glu Thr Val Gly Leu Pro Thr Asp Ala Glu Tyr Lys Lys Cys
   705                 710                 715                 720
   Thr Ala Gly Pro Leu Gly Thr Leu Lys Asp Leu Val Cys Ala Arg Glu
                   725                 730                 735
   Tyr Asn Gly Leu Leu Val Leu Pro Pro Ile Ile Thr Ala Asp Met Gln
               740                 745                 750
   Thr Met Tyr Thr Ala Ser Leu Val Ala Ser Met Ala Phe Gly Gly Ile
           755                 760                 765
   Thr Ala Ala Gly Ala Ile Pro Phe Ala Thr Gln Ile Gln Ala Arg Ile
       770                 775                 780
   Asn His Leu Gly Ile Thr Gln Ser Leu Leu Leu Lys Asn Gln Glu Arg
   785                 790                 795                 800
   Ile Ala Ala Ser Phe Asn Lys Ala Ile Gly His Met Gln Glu Gly Phe
                   805                 810                 815
   Arg Ser Thr Ser Leu Ala Leu Gln Gln Val Gln Asp Val Val Asn Lys
               820                 825                 830
   Gln Ser Ala Ile Leu Thr Glu Thr Met Asn Ser Leu Asn Lys Asn Phe
           835                 840                 845
   Gly Ala Ile Ser Ser Val Ile Gln Asp Ile Tyr Ala Gln Leu Asp Ala
       850                 855                 860
   Ile Gln Ala Asp Ala Gln Val Asp Arg Leu Ile Thr Gly Arg Leu Ser
   865                 870                 875                 880
   Ser Leu Ser Val Leu Ala Ser Ala Lys Gln Ser Glu Tyr Ile Arg Val
                   885                 890                 895
   Ser Gln Gln Arg Glu Leu Ala Thr Gln Lys Ile Asn Glu Cys Val Lys
               900                 905                 910
   Ser Gln Ser Asn Arg Tyr Gly Phe Cys Gly Ser Gly Arg His Val Leu
           915                 920                 925
   Ser Ile Pro Gln Asn Ala Pro Asn Gly Ile Val Phe Ile His Phe Thr
       930                 935                 940
   Tyr Thr Pro Glu Ser Phe Val Asn Val Thr Ala Ile Val Gly Phe Cys
   945                 950                 955                 960
   Val Gln Pro Ala Asn Ala Ser Gln Tyr Ala Ile Val Pro Val Asn Gly
                   965                 970                 975
   Arg Gly Ile Phe Ile Gln Val Asn Gly Ser Tyr Tyr Ile Thr Ala Arg
               980                 985                 990
   Asp Met Tyr Met Pro Arg Asp Ile Thr Ala Gly Asp Ile Val Thr Leu
           995                 1000                1005
   Thr Ser Cys Gln Ala Asn Tyr Val Asn Val Asn Lys Thr Val Ile Thr
       1010                1015                1020
   Thr Phe Val Glu Asp Asp Asp Phe Asp Phe Asp Asp Glu Leu Ser Lys
   1025                1030                1035                1040
   Trp Trp Asn Asp Thr Lys His Glu Leu Pro Asp Phe Asp Asp Phe Asn
                   1045                1050                1055
   Tyr Thr Val Pro Ile Leu Asn Ile Ser Gly Glu Ile Asp Arg Ile Gln
               1060                1065                1070
   Gly Val Ile Gln Gly Leu Asn Asp Ser Ile Ile Asp Leu Glu Glu Leu
           1075                1080                1085
   Ser Ile Ile Lys Thr Tyr Ile Lys Trp Pro Trp Tyr Val Trp Leu Ala
       1090                1095                1100
   Ile Gly Phe Ala Ile Ile Ile Phe Ile Leu Ile Leu Gly Trp Val Phe
   1105                1110                1115                1120
   Phe Met Thr Gly Cys Cys Gly Cys Cys Cys Gly Cys Phe Gly Ile Ile
                   1125                1130                1135
   Pro Leu Met Ser Lys Cys Gly Lys Lys Ser Ser Tyr Tyr Thr Thr Phe
               1140                1145                1150
   Asp Asn Asp Val Val Thr
           1155